(19) Europäisches Patentamt
European Patent Office
Office européen des brevets

(11) Publication number: 0 293 785
A2

(12) **EUROPEAN PATENT APPLICATION**

(21) Application number: 88108528.6

(22) Date of filing: 27.05.88

(51) Int. Cl.4: C12N 15/00 , C12P 21/02 , C12N 5/00 , C07K 13/00 , A61K 37/02

The microorganism(s) has (have) been deposited with American Type Culture Collection under number(s) CRL 9434.

(30) Priority: 29.05.87 US 55662
25.01.88 US 147842

(43) Date of publication of application:
07.12.88 Bulletin 88/49

(84) Designated Contracting States:
AT BE CH DE ES FR GB GR IT LI LU NL SE

(71) Applicant: ONCOGEN
3005 First Avenue
Seattle, WA 98121(US)

(72) Inventor: Purchio, Antony F.
801 33rd Avenue
Seattle Washington 98112(US)
Inventor: Gentry, Larry
645 Bennington Dr.
Maumee Ohio 43537(US)
Inventor: Twardzik, Daniel
9600 Timberland Pl.
Bainbridge Island Washington 98110(US)

(74) Representative: Kinzebach, Werner, Dr. et al
Patentanwälte Reitstötter, Kinzebach und Partner Sternwartstrasse 4 Postfach 86 06 49
D-8000 München 86(DE)

(54) **Cloning and expression of simian transforming growth factor-SS1.**

(57) Recombinant transforming growth factor-β1 (TGF-β1) is expressed to high levels in Chinese hamster ovary (CHO) cells using dihydrofolate reductase (dhfr) gene amplification. The expression plasmid was derived from the pSV2 vectors and contained, in tandem, the simian TGF-β1 and mouse dhfr cDNAs. Transcription of both cDNAs was controlled by the SV40 early promoter. Stepwise selection of transfected CHO cells in increasing concentrations of methotrexate yielded cell lines expressing amplified TGF-β1 nucleic acid sequences. The expression plasmid DNA was amplified greater than 35-fold in one of the methotrexate selected transfectants. The major proteins secreted by these cells consisted of latent TGF-β1 and TGF-β1 precursor polypeptides as judged by immunoblots using site-specific anti-peptide antibodies derived from various regions of the TGF-β1 precursor. Levels of recombinant TGF-β1 protein secreted by these cells approached 30 ug/24 hour/$10^7$ cells.

EP 0 293 785 A2

# CLONING AND EXPRESSION OF SIMIAN TRANSFORMING GROWTH FACTOR-β1

## 1. INTRODUCTION

The present invention relates to the cloning and expression of simian transforming growth factor-β1 (rTGF-β1). The product of the invention has a bioactivity equivalent to that of authentic mature human TGF-β1.

## 2. BACKGROUND OF THE INVENTION

The transforming growth factor (TGF) family of growth modulating peptides consists of two structurally and functionally dissimilar molecules, TGF-alpha and TGF-β. TGF-alpha, synthesized and released by retroviral transformed rodent cell lines (DeLarco et al., 1978, Proc. Natl. Acad. Sci. USA 75:4001-4005; Twardzik et al., 1982, Science 216:894-897) and some human tumor cell lines (Todaro et al., 1980, Proc. Natl. Acad. Sci. USA 77:5258-5262), competes with epidermal growth factor (EGF) for binding to the EGF receptor (Todaro et al., 1976, Nature 264, 26-29) and stimulates tyrosine specific phosphorylation of the EGF receptor (Reynolds et al., 1981, Nature 292:259-262). A potent mitogen for cells of mesenchymal origin, the mature form of TGF-alpha comprises 50 amino acid residues, shares sequence homology with both rodent and human EGF, (Marquardt et al., 1983, Proc. Natl. Acad. Sci. USA 80:4684-4688) and is cleaved from a 159 amino acid precursor (Derynck et al., 1984, Cell 38:287-297; Lee et al., 1985, Nature 313:489-491).

Very recently a protein isolated from bovine demineralized bone has been identified as being related to TGF-β (Seyedin et al., 1987, J. Biol. Chem. 262:1946-1949). The protein has also been isolated from porcine platelets (Cheifetz et al., 1987, Cell 48:409-415), a human prostatic adenocarcinoma cell line PC-3 (Ikeda et al., 1987, Biochemistry 26:2406-2410), and a human glioblastoma cell line (Wrann et al., 1987, EMBO 6:1633-1636). Partial amino acid sequence of this protein indicated that it was homologous to TGF-β and has been termed TGF-β2. The human (Derynck et al., 1985, Nature 316:701-705), mouse (Derynck et al., 1986, J. Biol. Chem. 261:4377-4379) and simian (Sharples et al., 1987, DNA 6:239-244) TGF-β1 described previously has been and is hereinafter termed TGF-β1.

TGF-β1, a disulfide linked homodimer (9 cysteine residues per chain) contains two identical subunits (112 amino acid residues per subunit) and utilizes a receptor distinct from either TGF-alpha or EGF (Frolik et al., 1984, J. Biol. Chem. 260:10995-11000; Tucker et al., 1984, Proc. Natl. Acad. Sci. USA 81:6757-6761). This potent modulator of cell behavior is synthesized by a variety of normal and transformed cells in culture (Roberts et al., 1981, Proc. Natl. Acad. Sci. USA 78:5339-5343) and has been purified from various sources including placenta (Frolik et al., 1983, Proc. Natl. Acad. Sci. USA 80:3676-3680), kidney (Roberts et al., 1983, Biochemistry 22:5692-5698), urine (Twardzik et al., 1985, J. Cell. Biochem. 28:289-297) and blood platelets (Childs et al., 1982, Proc. Natl. Acad. Sci. USA 79:5312-5316). TGF-β1 requires either TGF-alpha or EGF to promote the anchorage independent growth of normal rat kidney (NRK) fibroblasts; however the requirement for TGF-alpha or EGF is less stringent to promote anchorage independent growth of AKR-2, a murine indicator cell (Tucker et al., 1983, Cancer Res. 43:1581-1586). In contrast to stimulating cell growth, TGF-β1 from human platelets and a functionally related poltypeptide with nearly identical biochemical properties isolated from African Green monkey cells (BSC-1) has also been shown to exhibit growth inhibitory effects on some cells in culture (Tucker et al., 1984, Science 226:705-707). Both the bifunctionality of TGF-β1 (inhibition/stimulation) and its apparent ubiquitous distribution suggests it may play a key role in regulating the growth and behavior of mammalian cells.

The amino acid sequence deduced from cDNAs encoding the TGF-β1 precursor of human (Derynck et al., 1985, Nature 316:701-705) and mouse (Derynck et al., 1986, J. Biol. Chem. 261:4377-4379) origin indicate a high degree of homology not only in the area of the mature TGF-β1 sequence but also in the amino terminal precursor region.

Although the gene for human TGF-β1 has been identified and sequenced, the cloning and expression of large quantities of active TGF-β1 has not heretofore been reported. A number of factors may be responsible for the difficulty in expressing an active TGF-β1, one of which may involve the complexity of the tertiary structure of the molecule. Mature TGF-β1 has a number of interchain and intrachain disulfide bonds, the formation of which requires proper processing during expression of the gene product. Moreover, the mature form of TGF-β1 derived from a glycosylated larger precursor molecule. Correct glycosylation patterns of the precursor may be required for correct processing, secretion and cleavage of the mature form of TGF-β1. Thus, the cloning and expression of a gene having the correct coding sequence in an inappropriate expression vector/host cell system may result in the expression of a product having the proper primary structure (i.e., amino acid sequence) but incorrect secondary and tertiary structures (i.e., folding and conformation) resulting in an inactive molecule. Thus, the production of large quantities of TGF-β1 has been hampered.

## 3. SUMMARY OF THE INVENTION

The present invention relates to the production of large quantities of simian TGF-β1 by eucaryotic host cells transfected with recombinant DNA vectors containing the simian TGF-β1 coding sequence controlled by expression regulatory elements. A cDNA clone coding for simian TGF-β1 precursor was obtained from a cDNA library made from an African Green Monkey cell line, BSC-40. The deduced amino acid sequence of the mature simian TGF-β1 shows 100% homology with that of the mature human TGF-β1. Strong sequence homology was found between the precursor regions of the human and simian proteins with only five amino acid changes out of 278 residues. The simian (and murine) precursor sequence was found to code for one less amino acid residue than the human.

Expression vectors were constructed which contain the entire coding sequence for the simian TGF-β1 placed under the control of SV40 expression elements. They were used to transfect Chinese Hamster Ovary cells (CHO cells). The resulting CHO transfectants produce and secrete both mature rTGF-β1 which has a biological activity comparable to authentic TGF-β1 as well as the precursor form of rTGF-β1 which also has a biological activity.

## 4. DESCRIPTION OF THE FIGURES

FIG. 1 Nucleotide sequence of simian TGF-β1 cDNA and deduced amino acid sequence. The 1600 bp insert of pTGF-β1-2 was subcloned into the M13mp18 and M13mp18 cloning vectors (Yanisch-Perron et al., 1985 Gene 33:103-119) and both strands were sequenced using the dideoxy chain-termination method (Sanger et al., 1977 Proc. Natl. Acad. Sci. USA 74:5463-5467). The deduced amino acid sequence of simian TGF-β1 is presented directly above the cDNA sequence. The human TGF-β1 nucleotide sequence is aligned with and presented directly below the simian cDNA sequence; dots indicate homologous nucleotide residues within the sequences. Amino acid differences between the human and simian proteins are indicated in the top line. The mature TGF-β1 sequence is boxed and the signal peptide is overlined.

FIG. 2. Northern blot analysis of RNA from a human (MCF-7) and simian (BSC-40) cell line using a simian TGF-β1 cDNA probe. Polyadenylated RNA was isolated from MCF-7 cells and BSC-40 cells as described (Purchio et al., 1979, J. Virol. 29:763-769), fractionated on a 1% agarose-formaldehyde gel (12), transferred to a nylon membrane (Hybond, Amersham) and hybridized to [$^{32}$P]- labeled pTGF-β1-2 probe. Lane 1, human MCF-7 RNA (5 ug); lane 2, simian BSC-40 RNA (5 ug). 28S and 18S indicate the position of migration of 28S and 18S ribosomal RNAs.

Fig. 3. Construction of the amplifiable expression plasmid pSV2 (TGF-β1-dhfr). The details of the construction are outlined in Section 7 infra. The final recombinant plasmid contained the TGF-β1 cDNA encoding the entire precursor form of TGF-β1 and the mouse dhfr cDNA in tandem. Initiation of transcription of TGF-β1 and dhfr mRNAs are driven by the SV40 early promoter. Polyadeny-lation signals and other sequences responsible for correct RNA processing are supplied by 3′ SV40 sequences. Restriction sites in parenthesis are lost during construction of the amplifiable expression plasmid.

FIG. 4. Detection of TGF-β1 nucleic acid sequences in the CHO cells at various stages of MTX selection. Panel A: Northern blot analysis for detection of TGF-β1 mRNA sequences. Poly (A)+ containing mRNA (5 ug) was fractionated on a 1% agarose-formaldehyde gel, blotted onto Hybond N membranes (Amersham) and probed with the radiolabeled TGF-β1 DNA as described in Materials and Methods. The lane containing the nontransfected CHO mRNA was exposed for 48 hours to reveal endogenous levels of the 2.5 Kb TGF-β1 message. Lanes containing mRNA from TGF-β1-3 cells were exposed 5 minutes. Ribosomal markers are shown to the right. Panel B: Southern blot analysis of genomic DNA from the TGF-β1-3 CHO cell transfectants at various stages of MTX selection. High-molecular weight DNA from the TGF-β1-3 transfectants was digested with BamHI or EcoRI and 20 ug was fractionated onto a 1% agarose gel. The DNA was transferred to Hybond N (Amersham) membranes and probed with the nick-translated TGF-β1 DNA. Exposure time was 30 hours for TGF-β1-3-0 cells and 10 hours for TGF-β1-3-200 and 2000 cells. DNA size markers are indicated at the left of the figure. Arrows denote the sizes of DNA predicted to hybridize to the TGF-β1 probe.

FIG. 5. Growth inhibition assay of CCL-64 mink lung epithelial cells with recombinant or natural β1-TGF. Panel A: Growth inhibition assay employing natural TGF-β1 purified from bovine spleen ; 50% inhibition is typically attained using 8-12 picograms of TGF-β1. Panel B: Serum free supernatants were collected from confluent monolayers of the TGF-β1-3 transfectant at various stages of amplification. The media was dialyzed extensively against 0.2 M acetic acid and assayed for growth inhibition as described in Section 7.1.6 infra. The results displayed are normalized for 1 x $10^7$ cells per 5 ml collection. ——— , unamplified TGF-β1-3 cells; o————o TGF-β1-3 cells adapted to 2 µM methotrexate; o————o TGF-β1-3 cells adapted to 20 µM methotrexate.

FIG. 6. Acid activation of recombinant TGF-β1. A 24 hour collection of serum free supernatants from TGF-β1-3/2000 cells was made. Equal portions were then dialyzed against 0.2M acetic acid or 50mM $NH_4HCO_3$ (pH 7.0). As a control, supernatant first dialyzed versus 50mM $NH_4HCO_3$ was then dialyzed against 0.2M acetic acid. A dose response bioactivity curve of the differentially processed material is shown. ——— , supernatant dialyzed against $NH_4HCO_3$. o————o , supernatant dialyzed against 0.2M acetic acid. o————o , $NH_4HCO_3$ treated material further dialyzed versus 0.2 acetic acid.

FIG. 7. Line diagram illustrating the structural features of the rTGF-β1 protein and indicating the peptide regions used for the generation of site specific anti-peptide antisera. The one-letter code for amino acids is used: A (alanine), C (cysteine), D (aspartic acid), E (glutamic acid), F (Phenylalanine), G (Glycine), H (histidine), I (Isoleucine), K (lysine), L (leucine), M (methionine), N (asparagine), P (proline), Q (glutamine), R (arginine), S (serine), T (threonine), V (valine), W (tryptophan), Y (tyrosine). Functionally important domains of TGF-β1 are identified: leader sequence, precursor sequences, and mature TGF-β1.

FIG. 8. Identification of recombinant TGF-β1 in the conditioned media of TGF-β1-3 cells by immunoblotting. Serum free supernatants were collected from confluent cultures of cells and dialyzed extensively against 0.2 M acetic acid. After lyophilization, the material was solubilized in SDS-sample buffer, the equivalent of 2 x $10^5$ cells fractionated on SDS-polyacrylamide gels, and immunoreactive TGF-β1 proteins were detected by immunoblotting. Anti-TGF-$\beta 1_{-369-381}$ was utilized for the immunoblots. For the peptide blocking experiment, 50 ug/ml of $peptide_{369-381}$ was added to the antibody prior to the immunoblot. Panel A: Immunoblot of material collected in supernatants from TGF-β1-3 adapted to 0, 2, or 20 µM methotrexate and fractionated on SDS polyacrylamide gels under reducing conditions. Natural bovine spleen TGF-β1 (100 ng) was included for comparison. Panel B: Supernatants were fractionated under non-reducing reducing conditions. Bovine spleen TGF-β1 (250 ng) was included.

FIG. 9. Immunoblot of secreted recombinant TGF-β1 produced by TGF-β1-3 cells probed with anti-TGF-$\beta 1_{81-94}$ and anti-TGF-$\beta 1_{225-236}$. Supernatants were collected, processed as described in FIG. 8 and fractionated on gradient SDS-polyacrylamide gels. (A) Immunoblot of supernatants fractionated on reducing SDS-polyacrylamide gels. An immunoblot performed with a mixture of precursor-specific and TGF-β1-specific antibodies is shown in the last panel to show the three distinct forms of recombinant material. (B) Immunoblot of supernatants fractionated on SDS-polyacrylamide gels under non-reducing conditions. A mixture of precursor-specific and TGF-β1 specific antibodies is shown in the last panel.

FIG. 10. Detection of mature and precursor forms of rTGF-β1 in total secreted proteins of TGF-β1-3/0 and TGF-β1-3/2000 cells. Cells grown to confluency on 60 mm round tissue culture dishes were labeled in 3ml of DMEM lacking methionine, cysteine and fetal bovine serum and containing 100 uCi/ml of $^{35}S$-methionine and $^{35}S$-cysteine. After 18 hours, the serum-free labeled supernatant was collected, clarified, and fractionated on reducing 7.5-17.5% SDS-polyacrylamide gels. Following electrophoresis, the gel was processed with $En^3$Hance and autoradiographed. The results show an 8 hour exposure using 3 ul of the labeled supernatant.

FIG. 11. Detection of TGF-β1 related proteins secreted by TGFβ3-2000 cells. A) Line diagram of TGF-β1 precursor. See text for discussion. B) TGFβ3-2000 cells were grown to confluency in 100 mm dishes as described previously (Gentry et al., 1987, Mol. Cell. Biol. 7:3418-3427). Serum-free supernatants (5 mL) were collected, dialyzed against 0.2 M acetic acid and 0.5 mL samples were lyophylized and immunoblotted as described (Gentry et al., 1987, Mol. Cell. Biol. 7:3418-3427) using a pool of antipeptide antibodies against amino acid residues 369-381 (Anti-$TGF\beta_{369-381}$) and 81-94 (anti-TGBβ81;94): lane 1, non-reduced sample; lane 2, reduced sample. The numbers on the left (lane 1) and right (lane 2) indicate the position of molecular weight standards in kilodaltons. C) TGFβ3-2000 cells were grown to confluency in 60 mm tissue culture dishes and pulsed in serum-free medium (3 mL) lacking methionine and cysteine and containing 100-200 µ Ci/mL of [$^{35}$S]-cysteine and [$^{35}$S]-methionine for 15 min. Cells were then chased for 4 hours in serum-free medium containing methionine and cysteine and a 10 µl sample was analyzed on a 7.5%-15% polyacrylamide-SDS gradient gel as described (Laemmli, 1970, Nature 227:680-685) under non-reducing conditions (lane 1). A second dish of cells was labeled for 24 hours in serum-free medium containing 200 µ Ci/mL [$^{3}$H]-glucosamine and the cell free supernatant was dialyzed for 48 hours against 0.2 M acetic acid. Two hundred microliters of this material was lyophylized and analyzed on a 7.5%-15% polyacrylamide-SDS gradient gel under non-reducing conditions. The gel was fluorographed and exposed for autoradiography using Cronx-4 X-ray film (DuPont). D) Same as C) except samples were run under reducing conditions. E) TGFβ3-2000 cells were labeled with 1 m Ci/mL of [$^{32}$P]-orthophosphate in serum and phosphate free medium. Cell free supernatants were treated as described above, fractionated on a 15% polyacrylamide-SDS gell under reducing conditions and the gel was autoradiographed. F) [$^{32}$P]-labeled precursor was purified by two cycles of polyacrylamide-SDS gel electrophoresis and hydrolyzed for 1 hour at 95° C in 6M HCl (Cooper et al., 1983, Meth. Enzymol. 99:387-402). The digestion products were separated by electrophoresis at ph 1.9 and at ph 3.5, and detected by autoradiography. Internal standards (P-ser, P-thr, p-tyr) were detected with ninhydrin.

FIG. 12. Digestion of serum-free supernatants from TGFβ3-2000 cells with various glycolytic enzymes. A) TGFβ3-2000 cells were grown to confluence and incubated for 24 hours in serum-free medium. The medium was dialyzed against 0.2 M acetic acid, lyophylized, and samples were treated with neuraminidase (.25 units/mL, lane 3), N-glycanase (20 units/mL, lane 2) or endoglycosidase H (0.2 units/mL, lane 4). The digests were fractionated by SDS-polyacrylamide gel electrophoresis under reducing conditions and analyzed by immunoblotting as described in the legend to Figure 11. Lane 1 contains an untreated sample. B) Serum-free supernatants from TGFβ3-2000 cells were labeled with [$^{35}$S]-methionine and [$^{35}$S]-cysteine as described in the legend to Figure 1 and digested as above with endoglycosidase H (lane 2), neuraminidase (lane 3) and N-glycanase (lane 4). Lane 1 contains an untreated sample. Digests were fractionated by SDS-polyacrylamide gel electrophoresis under reducing conditions and the gels were autoradiographed. N-glycanase was purchased from Genzyme (Boston, Mass.) and endoglycosidase H and neuraminidase were from Calbiochem (La Jolla, CA): buffer conditions were as recommended by the manufacturer.

FIG. 13. Cell-free translation of TGF-β1 specific transcripts. A) A 1350 base pair Pst I-Eco R fragment containing the entire coding region of TGF-β1 (3) was subcloned into pSP64 (Pharmacia) and 5 µg of linearized plasmid was transcribed with SP6 polymerase (Kreig and Melton, 1984, Nucleic Acids Res. 18:7057-7070) purchased from Bethesda Research Labs (Baltimore, MD) using ionic conditions described previously (Pelham and Jackson, 1976, Eur. J. Biochem. 67:247-251). The reaction was digested with DNase, extracted twice with phenol: chloroform: isoamylalcohol (24:24:1) and ethanol precipitated. The RNA was dissolved in 50 µl of $H_2O$ and 10 µl (lane 2) was fractionated in a 1% agarose-urea gel as described (Purchio et al., 1980, J. Virol. 35:629-639). Lane 1 contains reticulocyte ribosomal RNA markers. The gel was stained with ethidium bromide, illuminated with UV light and photographed. B) The RNA described above was treated for 10 minutes at 22° C for 10 mM methyl mercury, adjusted to 20 mM 2-mercaptoethanol and 1 µg was translated in a message-dependent reticulocyte cell-free translation system (Purchio et al., 1983, J. Virol. 48:320-324) in a total volume of 50 µl using [$^{35}$S]-methionine and ionic conditions described previously (Sharples et al., 1987, DNA 6:239-244). The reactions were fractionated on a 10% polyacrylamide-SDS gel (Laemmli, 1970, Nature 227:680-685); the gel was fluorographed and exposed to Cronex-4 X-ray film. Lane 1, no added RNA; lane 2, 1 µg TGF-β1 RNA.

FIG. 14. Gel permeation chromatography on a Bio-Sil TSK-250 column (21.5 x 600 mm) of 20 mg protein after ammonium sulfate precipitation of 500 mL serum-free supernatant from TGF-β-3-2000 cells. The column was equilibrated with 0.1% TFA in water containing 40% (v/v) acetonitrile at 2 mL/min, at 22° C; 4 mL fractions were collected. Aliquots of the indicated fractions were assayed for growth-inhibitory activity on mink lung epithelial cells (-o-). The solid line gives the protein absorbance at 254 nm. The following proteins were used as markers: α-chymotrypsinogen (Mr 25,700), bovine pancreatic ribonuclease A (Mr 13,700), and insulin (Mr 5,700).

FIG. 15. Purification of rTGF-β1 by reversed-phase HPLC. Elution pattern of 0.65 mg protein from gel permeation chromatography purified TGF-β1 (Figure 2, pool B) on a µBondpak $C_{18}$ column (10-µm particle size, 3.9 x 300 mm). Elution was achieved with a linear 10-min gradient of 0.05% TFA in water to 30% acetonitrile in 0.045% TFA, and a 10-min gradient of 36-60% acetonitrile in 0.045% TFA. The column was operated at a flow rate of 0.2 mL/min, at 22° C. Aliquots of the indicated fractions were assayed for growth-inhibitory activity on mink lung epithelial cells (-o-). The horizontal bar indicates pooled rTGF-β1. UV-absorbing material was continuously monitored at 214 nm ( ——— ); the dashed line (----) denotes the concentration of acetonitrile.

FIG. 16. SDS-polyacrylamide gel analysis of purified rTGF-β1 proteins. Precursor and mature forms of TGF-β1 were fractionated by SDS-PAGE under non-reducing (A) or reducing (B) conditions and stained with Coomassie blue R-250. nTGF-β1 was isolated from bovine spleen and used for comparison. Marker proteins in KDal are indicated at the left and right of the figure.

FIG. 17. Coomassie blue staining pattern of proteins from the conditioned medium of amplified CHO cells exressing TGF-β1. Conditioned medium was dialyzed, fractionated on 15% SDS-polyacrylamide gels under reducing conditions, and stained with Coomassie blue R-250. For reference, the letters a, b, and c are noted at the left of the figure to indicate rTGF-β1 molecules. Lane 1, 0.25 ml of conditioned medium; Lane 2, shows marker proteins with the indicated molecular weight in KDal.

FIG. 18. Gel permeation chromatography on a Bio-Sil TSK-250 column (7.5 x 600 mm) of CNBr peptides of rTGF-β1-precursor. Elution pattern of 800 pmol of rTGF-β1-precursor cleaved with CNBr. The column was equilibrated with 0.1% TFA in water containing 40% acetonitrile at 0.25 mL/min, at 22°C. UV-absorbing material was monitored at 214 nm. Peaks designated M refer to CNBr peptides subjected to Edman degradation; numbers refer to the position of that particular fragment or fragments connected by disulfide bonds in the complete sequence (Sharples et al., 1987, DNA 6:239-244).

FIG. 19. Growth inhibition curves of purified TGF-β1 proteins using mink lung indicator cells. Growth inhibition was assessed as described in the text. Concentration of TGF-β1 polypeptides was determined by amino acid analysis. -Δ-, recombinant precursor protein; -●-, nTGF-β1 from bovine spleen; -o-, rTGF-β1.

FIG. 20. (A) Proposed structure of TGF-β1 precursor highlighting its disulfide cross-linked nature. (B) Summary of processing events of pre-pro-TGF-β1 in transfected CHO cells. Proteolytic processing sites have been highlighted. Asterisks denote glycosylation sites. Darkened, signal peptide; open, pro region; hatched, mature TGF-β1.

FIG. 21. Effect of TGF-β1 and TGF-β2 on the growth of A549 human lung tumors in nude mice. Male nude mice (Balb/c-nu+/nu+) at 12 weeks of age, were injected in the dorsal neck region subcutaneously with 1.3 X $10^6$ human lung carcinoma cells (A549) in a volume of 0.2 mL of phosphate buffered saline. Palpable tumors (< $10mm^3$ - 3x3x1 mm) developed in 20 days in approximately 80% of the animals. Tumor bearing animals were randomly assigned to different cages. Day 1 of treatment corresponds to the first day animals were treated after measurable tumors developed. Each group of 5 animals were injected as indicated subcutaneously adjacent to the tumor (peritumorally) in a carrier volume of 0.10 mL. Control group(s) were injected with either high pressure liquid chromatography purified bovine serum albumin (2 µg) or a synthetic peptide (200 ng) corresponding to a loop region of epidermal growth factor (residues 11-21). Tumor size was measured with calipers in three dimensions before subsequent injections on the days indicated on the abscissa. Values for each point represent average tumor volume. TGF-β1 and TGF-β2 were purified to homogeneity from bovine bone as previously described (Massaque et al., 1986, Proc. Nat. Acad. Sci., 83:8206-8210; Sporn et al., 1983, Science 219:1329) and stabilized with a ten-fold excess of bovine serum albumin. Total amounts of each factor administered for the duration of this particular experiment were 1.4 µg per animal.

FIG. 22. Dose response of TGF-β1 inhibition of A549 human carcinoma tumors in nude mice. Protocol is identical to that described in description of FIG. 21 except that initial tumor size at day 1 of treatment (day 25 post tumor cell inoculation) was larger (20 $mm^3$). Control animal groups were injected with BSA; treated animal groups received 5 injections every third day of either 12.5, 50 or 200 ng of TGF-β1 peritumorally administered over a 15-day period. Values represent the average tumor volume from each group of animals at day 15.

FIG. 23. Photograph of non-treated and TGF-β1 treated A549 lung carcinoma bearing nude mice. Top of photo is control animal bearing large subcutaneous tumor at day 20 (actual photo of mice from experiment described in FIG. 21); bottom of photo is animal representative of TGF-β1-treated group (same experiment). Inset (upper right) shows excised tumors from control (left) and treated (right) animals. (Size differences in photo between tumors in animals and after excision reflects difference in camera range.)

FIG. 24. Histological examintion of tumors excised from mock and TGF-β1-treated animals. Freshly excised tumors (day 20 of treatment, FIG. 21) were fixed in buffered formalin imbedded in paraffin, sectioned and stained with Gamori's trichrome. Section of tumor derived from mock-treated animals, Panel A (20X), Panel C (100X). Section of tumor derived from TGF-β1 treated animals. Panel B (20X), Panel D (100X). Panel D inset; vessel wall from section of tumor derived from TGF-β1 treated-animals (100X).

FIG. 25. Staining pattern of fixed human lung tumor sections from mock-treated and TGF-β1-treated animals. Sections of tumors derived from mock-treated animals stained with PAS (Panel A), 100X; with Alcian Blue (Panel C) 100X; section of tumor derived from TGF-β1-treated animals stained with PAS, (Panel B) 100X; Alcian Blue (Panel D) 100X.

FIG. 26. SDS-Polyacrylamide gel electrophoresis of TGF-β1 precursor proteins released by clone 17 cells. (A) Line diagram of TGF-β1 protein; , indicates glycosylation sites which are phosphorylated; , indicates non-phosphorylated glycosylation sites. Cell free supernatants from clone 17 cells were labeled with [$^{35}$S]-methionine and [$^{35}$S]-cysteine (B), [$^{3}$H]-glucosamine (C), [$^{3}$H]-mannose (D) and [$^{32}$P]-phosphate (E) in serum-free media; samples were processed and analyzed on a 7.5-15% gradient SDS-polyacrylamide gel (B and C), or on a 15% SDS-polyacrylamide gel (D and E).

FIG. 27. N-glycanase digestion of TGF-β1 precursor proteins produced by clone 17 cells. (A) [$^{35}$S]-labeled cell free supernatants were processed, treated with N-glycanase and analyzed on a 7.5-15% gradient SDS-polyacrylamide gel: lane 1, no enzyme; lane 2, plus N-glycanase. (B) Same as in A, except the label was [$^{32}$P]-phosphate.

FIG. 28. Amino acid sequences of TGF-β1 precursor glycopeptides. Peptides obtained by cleavage with S. aureus V8 protease (E), and trypsin (T) are indicated. , indicates glycosylation sites which are phosphorylated; , indicates non-phosphorylated glycosylation sites. The numbering refers to the position of the particular glycopeptide in the complete sequence of the TGF-β1 precursor. x, unidentified residue.

FIG. 29. Gel permeation chromatography of CNBr peptides of [$^{32}$P]-labeled TGF-β1 precursor. Chromatography on a Bio-sil TSK-250 column (7.5 x 600 mm). Elution pattern of 650 pmol of S-pyridylethylated TGF-β1 precursor and of 165,000 cpm of S-pyridylethylated [$^{32}$P]-TGF-β1 precursor cleaved with CNBr. The indicated fractions were measured for [$^{32}$P]- radioactivity (-o-). The solid line gives the protein absorbance at 214 nm. The following proteins were used as markers: α-lactoglobulin (Mr 18,400), cytochrome C (Mr 12,300), and insulin (Mr 5,700). Peaks designated by an M refer to CNBr peptides; numbers refer to the position of that particular fragment in the complete sequence of the TGF-β1 precursor (Sharples et al., 1987, DNA 6:239-244).

FIG. 30. Reversed-phase high performance liquid chromatography of S. aureus V8 protease peptides of CNBr peptide M(39-113). Chromotography on an RP-300 column (2.1 x 30 mm). Elution pattern of 370 pmol of M(39-113) containing 12,500 cpm of [$^{32}$P]-M(39-113) digested with S. aureus V8 protease. The elution of peptides was achieved with a 2-hour linear gradient of 0.1% trifluoroacetic acid in water to 60% acetonitrile containing 0.08% trifluoroacetic acid at a flow rate of 100 μl/min, at 35°C. UV-absorbing material was monitored at 215 nm (---). [$^{32}$P] radioactivity (-o-) was determined in an LS 6800 liquid scintillation counter (Beckman). Peaks designated by an E refer to V8 protease peptides subjected to Edman degradation.

FIG. 31. Reversed-phase high performance liquid chromatography of S. aureus V8 protease peptides and trypsin peptides of CNBr peptide M(134-253). Chromatography on an RP-300 column (2.1 x 30 mm). (A) Elution pattern of 400 pmol of M(134-253) containing 44,000 cpm of [$^{32}$P]-M(134-253) digested with S. aureus V8 protease. (B) Elution pattern of trypsin peptides derived from pool A (FIG. 31A) containing 3,100 cpm of [$^{32}$P]-E(170-194). The chromatography conditions are described in the legend to FIG. 30. Peaks designated by an E refer to V8 protease peptides, and peaks designated with a T refer to trypsin peptides subjected to Edman degradation.

FIG. 32. Identification of mannose-6-phosphate. (A) [$^{32}$P]-labeled TGF-β1 precursor proteins were hydrolyzed with acid for 2 hours. Hydrolysis products were mixed with non-radioactive phosphoaminoacids and mannose-6-phosphate and separated by electrophoresis at pH 1.9, and orthogonally, at pH 3.5. An autoradiogram is shown. The sample is spotted at lower right (arrowhead). Mannose-6-phosphate (M6P), phosphoserine (PS), phosphothreonine (PT), phosphotyrosine (PY) and inorganic phosphate (Pi), are marked. [$^{32}$P]- was detected comigraging with mannose-6-phosphate and Pi, and in positions expected for phospho-oligosaccharides (small arrowheads). (B) Similar anlyses of products of 1 hour hydrolysis of (B) E-(76-91) and (C) E(134-139). (D) [$^{32}$P]-labeled TGF-β1 precursor proteins were hydrolyzed and separated by electrophoresis at pH 8.9 followed by chromatography.

## 5. DESCRIPTION OF THE INVENTION

The present invention relates to the production of a biologically active, mature form of rTGF-β1 from the simian TGF-β1 precursor gene coding sequence and its product. The mature biologically active TGF-β1 may be produced by the cloning and expression of the full-length nucleotide coding sequence of simian

TGF-β1 in a host cell which processes the precursor correctly so that a mature rTGF-β1 is produced, which has a biological activity that is virutally indistinguishable from that of authentic natural TGF-β1.

The present invention also relates to a biologically active rTGF-β1 precursor protein which can be produced using the simian TGF-β1 precursor gene. Biologically active rTGF-β1 precursors may be produced by the cloning and expression of the full-length nucleotide coding sequence of simian TGF-β1 in an appropriate host cell capable of secreting the TGF-β1 precursors.

The method of the invention may be divided into the following stages solely for the purposes of description: (a) isolation or generation of the coding sequence for the precursor form of simian TGF-β1; (b) construction of an expression vector which will direct the expression of the simian TGF-β1 coding sequence; (c) transfection of appropriate host cells which are capable of replicating and expressing the gene and processing the gene product to produce the mature biologically active form of TGF-β1 and/or TGF-β1 precursors; and (d) identification and purification of the TGF-β1 precursors and the mature, biologically active TGF-β1.

Once a transfectant is identified that expresses high levels of TGF-β1 precursors and/or bioactive, mature TGF-β1, the practice of the invention involves the expansion of that clone and isolation of the gene product expressed.

The method of the invention is demonstrated herein, by way of examples in which the cDNA of the simian TGF-β1 precursor coding region was prepared, cloned, and sequenced. The coding region was then placed under the control of SV40 expression control elements and used to transfect CHO cells. The CHO transfectants produced a mature rTGF-β1 with biological activity that was indistinguishable from that of natural TGF-β1 as well as larger biologically active precursor forms.

The various aspects of the method of the invention are described in more detail in the subsections below and in the examples that follow.

## 5.1. ISOLATION OR GENERATION OF THE SIMIAN TGF-β1 CODING REGION

The nucleotide coding sequence for simian TGF-β1 is depicted in FIG. 1. In the practice of the method of the invention, this nucleotide sequence, or its functional equivalent can be used to generate the recombinant molecules which will direct the expression of the TGF-β1 product. Due to the degeneracy of the nucleotide coding sequences, other DNA sequences which encode substantially the same amino acid sequence as depicted in FIG. 1 may be used in the practice of the present invention for the cloning of TGF-β1. Such alterations of the nucleotide sequence of FIG. 1 include deletions, additions or substitutions of different nucleotide residues resulting in a sequence that encodes the same or a functionally equivalent gene product. The gene product may contain deletions, additions or substitutions of amino acid residues within the sequence, which result in a silent change thus producing a bioactive product. Such amino acid substitions may be made on the basis of similarity in polarity, charge, solubility, hydrophobicity, hydrophilicity and/or the amphipathic nature of the residues involved. For example, negatively charged amino acids include aspartic acid and glutamic acid; positively charged amino acids include lysine and arginine; amino acids with uncharged polar head groups or nonpolar head groups having similar hydrophilicity values include the following: leucine, isoleucine, valine; glycine, alanine; asparagine, glutamine; serine, threonine; phenylalanine, tyrosine.

The nucleotide coding sequence for TGF-β1 may be obtained from simian cell sources that produce high levels of TGF-β1-like activity. The coding sequence may be obtained by cDNA cloning of RNA isolated and purified from such cellular sources or by genomic cloning. Either cDNA or genomic libraries of clones may be prepared from the DNA fragments generated using techniques well known in the art including but not limited to the use of restriction enzymes. The fragments which encode TGF-β1 may be identified by screening such libraries with a nucleotide probe that is substantially complementary to any portion of the sequence depicted in FIG. 1. Full length clones, i.e., those containing the entire coding region for the TGF-β1 precursor may be selected for expression.

In an alternate embodiment of the invention, the coding sequence of FIG. 1 could be synthesized in whole or part, using chemical methods well known in the art.

In the examples described herein, the simian TGF-β1 coding sequence was obtained by cDNA cloning of the TGF-β1 precursor coding sequence derived from polyadenylated RNA isolated from the African green monkey cell line, BSC-40, which had been shown to produce high levels of a growth inhibitor functionally related to TGF-β1. The entire coding region was sequenced and compared to the published sequences of human and murine TGF-β1 (see FIG. 1). The deduced amino acid sequence of the mature simian TGF-β1 demonstrates 100% homology with that of mature human TGF-β1. The precursor sequences also demonstrate strong sequence homology with only five amino acid differences between the human and simian precursor sequences. Interestingly, the simian (and murine) precursor sequence encode one less amino acid residue than the sequence reported for human TGF-β1 (see FIG. 1, amino acid residue numbers 158-159).

The remarkable conservation in the sequence of TGF-β1 between simian, rodent and human species suggests that strong evolutionary pressure was required, by necessity, to conserve important functionality. This applies not only to the mature form of TGF-β1, which has an important bifunctional role as a growth regulator, but also to the polypeptide region of the precursor upstream of the mature TGF-β1 sequence which may also exhibit coordinate growth modulatory activities.

The major TGF-β1 mRNA species in BSC-40 cells is 2.5kb, in agreement with results obtained for murine and human TGF-β1 specific messages (FIG. 2). The minor bands seen at 4kb and 1.45kb may represent either aberrantly processed transcripts or may code for a TGF-β1 like molecule (CIF-B) recently described by Seyedin (1987, J. Biol. Chem. 262: 1946-1949) which contains extensive homology to TGF-β1.

The deduced polypeptide sequence of the simian, human, and mouse clones contain a nearly identical contiguous stretch of 14 hydrophobic residues in positions 8-21 which may constitute an amino terminal signal peptide. In addition, an Arg-Arg dipeptide immediately preceeds the amino terminus of mature TGF-β1 suggesting similar proteolytic cleavage sites. The simian TGF-β1 precursor also contains three potential N-glycosylation sites not found in the mature molecule. Thus, not only has the primary structure of the mature and precursor polypeptide of TGF-β1 been conserved among species, but distinct post translational modification sites as well.

BSC-40 cells sythesize and release high levels of TGF-β1 relative to other cell lines we have tested. Media conditioned by BSC cells contain an activity which in the presence of nanogram levels of EGF and TGF-alpha stimulates the anchorage independent growth of NRK cells and exhibits biochemical characteristics identical to TGF-β1 purified from human platelets (Frolik et al., 1983, Proc. Natl. Acad. Sci. USA 80:3676-3680; Roberts et al., 1983, Biochemistry 22:5692-5698). In addition, the isolation of a growth inhibitor from BSC-1 cells functionally similar to TGF-β1 which competes with platelet derived TGF-β1 for binding to TGF-β1 membrane receptors has been described (Tucker et al., 1984, Science 226:705-707). The large amount of TGF-β1 specific mRNA synthesized by BSC-40 cells, a cell line derived from BSC-1 cells, suggests that the bifunctional growth modulator identified by Tucker et al. in BSC-1 conditioned media is indeed TGF-β1. This is also supported by recent data which confirms Tucker et al.'s observations that TGF-β1 from other sources also inhibits the growth of some tumor cells in culture (Roberts et al., 1985, Proc. Natl. Acad. Sci. USA 82:119-123). However, formal proof that the BSC-1 derived inhibitor is indeed the product of the gene described herein will require sequence analysis of the TGF-β1 like activity released by BSC-1 cells in culture.

## 5.2. CONSTRUCTION OF EXPRESSION VECTORS CONTAINING THE TGF-β1 CODING SEQUENCE

In order to express a biologically active, mature form of TGF-β1, an expression vector/host system should be chosen which provides not only for high levels of transcription and translation but for the correct processing of the gene product. This is especially important when employing the entire coding sequence of the simian TGF-β1 precursor in the expression constructs because the mature form of TGF-β1 appears to be derived from the precursor product via cellular processing events. The proposed processing scheme is presented in FIG. 20. In addition an expression/host cell system which provides for secretion of the product may be selected.

In particular, it appears that the mature TGF-β1, a disulfide linked homodimer of 112 amino acids per subunit is formed by cellular processing involving proteolytic cleavage of the full length precursor at the Arg-Arg amino acid (residue numbers 277 and 278 in FIG. 1). In addition, the simian TGF-β1 precursor contains three potential N-glycosylation sites not found in the mature form and analysis of [$^3$H] glucosamine labeled serum free supernatants from a line of chinese hamster ovary cells which secrete high levels of recombinant TGF-β1 indicate that the TGF-β1 precursor, but not the mature form, is glycosylated. Thus, the proper glycosylation of the precursor may be important to the cellular synthesis and release or secretion of the mature molecule. The TGF-β1 precursor, but not the mature form, is also phosphorylated, further suggesting the functional importance of the precursor. Moreover, the mature form of TGF-β1 comprises a disulfide linked dimer involving nine cysteine residues per subunit. Some of these are involved in interchain and others in intrachain disulfide bonds which affect the tertiary structure and configuration of the mature molecule, and, as a result, its biological activity. Thus, the ability of a host cell used in the expression system to correctly express and process the simian TGF-β1 gene product is important to the production of a biologically active, mature TGF-β1.

In the examples described herein, the mature, bioactive form of TGF-β1 was successfully produced using simian virus 40 (SV40) expression control elements in a Chinese Hamster Ovary (CHO) host cell system. However, a variety of other animal host/expression vector systems (i.e., vectors which contain the necessary elements for directing the replication, transcription and translation of the TGF-β1 coding sequence in an appropriate host cell) may be utilized equally well by the skilled artisan. These include, but are not limited to, virus expression vector/mammalian host cell systems (e.g., cytomegalovirus, vaccinia virus, adenovirus, and the like); insect virus expression vector/insect cell systems (e.g., baculovirus); or nonviral promoter expression systems derived from the genomes of mammalian cells (e.g., the mouse metallothionine promoter).

The expression elements of these vectors vary in their strength of specificities. Depending on the host/vector system utilized, any one of a number of suitable transcription and translation elements may be used. For instance, when cloning in mammalian cell systems, promoters isolated from the genome of mammalian cells, (e.g. mouse metallothionien promoter) or from viruses that grow in these cells, (e.g. vaccinia virus 7.5K promoter) may be used. Promoters produced by recombinant DNA or synthetic techniques may also be used to provide for transcription of the inserted sequences.

Specific initiation signals are also required for suficient translation of inserted protein coding sequences. These signals include the ATG initiation codon and adjacent sequences. In cases where the entire TGF-β1 gene including its own initiation codon and adjacent sequences are inserted into the appropriate expression vectors, no additional translational control signal may be needed. However, in cases where only a portion of the coding sequence is inserted, exogenous translational control signals, including the ATG initiation codon must be provided. Furthermore, the initiation codon must be in phase with the reading frame of the TGF-β1 coding sequences to ensure translation of the entire insert. These exogenous translational control signals and initiation codons can be of a variety of origins, both natural and synthetic. The efficiency of expression may be enhanced by the inclusion of transcription attenuation sequences, enhancer elements, etc.

Any of the methods previously described for the insertion of DNA fragments into a vector may be used to construct expression vectors containing the TGF-β1 gene and appropriate transcriptional/translational control signals. These methods may include in vitro recombinant DNA techniques, synthetic techniques and in vivo recombinations (genetic recombination).

In cases where an adenovirus is used as an expression vector, the TGF-β1 coding sequence may be ligated to an adenovirus transcription/translation control complex, e.g., the late promoter and tripartite leader sequence. This chimeric gene may then be inserted in the adenovirus genome by in vitro or in vivo recombination. Insertion in a non-essential region of the viral genome (e.g., region E1 or E3) will result in a recombinant virus that is viable and capable of expressing TGF-β1 in infected hosts. Similarly, the vaccinia 7.5K promoter may be used.

An alternative expression system which could be used to express TGF-β1 is an insect system. In one such system, Autographa californica nuclear polyhedrosis virus (AcNPV) is used as a vector to express foreign genes. The virus grows in Spodoptera frugiperda cells. The TGF-β1 coding sequence may be cloned into non-essential regions (for example the polyhedrin gene) of the virus and placed under control of an AcNPV promoter (for example the polyhedrin promoter). Successful insertion of the TGF-β1 coding sequence will result in inactivation of the polyhedrin gene and production of non-occluded recombinant virus (i.e., virus lacking the proteinaceous coat coded for by the polyhedrin gene). These recombinant viruses are then used to infect Spodoptera fugiperda cells in which the inserted gene is expressed.

In addition, a host cell strain may be chosen which modulates the expression of the inserted sequences, or modifies and processes the gene product in the specific fashion desired. Expression from certain promoters can be elevated in the presence of certain inducers, (e.g. zinc and cadmium ions for metallothionein promoters). Therefore, expression of the genetically engineered TGF-β1 may be controlled. This is important if the protein product of the cloned foreign gene is lethal to host cells. Furthermore, modifications (e.g. glycosylation) and processing (e.g., cleavage) of protein products are important for the function of the protein. Different host cells have characteristic and specific mechanisms for the post-translational processing and modificatin of proteins. Appropriate cell lines or host systems can be chosen to ensure the correct modification and processing of the foreign protein expressed.

## 5.3 IDENTIFICATION OF TRANSFECTANTS OR TRANSFORMANTS EXPRESSING THE TGF-β1 GENE PRODUCT

The host cells which contain the simian TGF-β1 coding sequence and which express the biologically active, mature product may be identified by at least four general approaches: (a) DNA-DNA hybridization; (b) the presence or absence of "marker" gene functions; (c) assessing the level of transcription as mesured by the expression of TGF-β1 mRNA transcripts in the host cell; and (d) detection of the mature gene product as measured by immunoassay and, ultimately, by its biological activity.

In the first approach, the presence of the simian TGF-β1 coding sequence inserted in the expression vector can be detected by DNA-DNA hybridization using probes comprising nucleotide sequences that are homologous to the simian TGF-β1 coding sequence substantially as shown in FIG. 1, or portions or derivatives thereof

In the second approach, the recombinant expression vector/host system can be identified and selected based upon the presence or absence of certain "marker" gene functions (e.g., thymidine kinase activity, resistance to antibiotics, resistance to methotrexate, transformation phenotype, occlusion body formation in baculovirus, etc.). For example if the TGF-β1 coding sequence is inserted within a marker gene sequence of the vector, recombinants containing the TGF-β1 coding sequence can be identified by the absence of the marker gene function. Alternatively, a marker gene can be placed in tandem with the TGF-β1 sequence under the control of the same or different promoter used to control the expression of the TGF-β1 coding sequence. Expression of the marker in response to induction or selection indicates expression of the TGF-β1 coding sequence.

In the third approach, transcriptional activity for the TGF-β1 coding region can be assessed by hybridization assays. For example, polyadenylated RNA can be isolated and analyzed by Northern blot using a probe homologous to the TGF-β1 coding sequence or particular portions thereof. Alternatively, total nucleic acids of the host cell may be extracted and assayed for hybridization to such probes.

In the fourth approach, the expression of the mature protein product can be assessed immunologically, for example by Western blots, immunoassays such as radioimmuno-precipitation, enzyme-lined immunoassays and the like. The ultimate test of the success of the expression system, however, involves the detection of the biologically active TGF-β1 gene product. Where the host cell secretes the gene product the cell free media obtained from the cultured transfectant host cell may be assayed for TGF-β1 activity. Where the gene product is not secreted, cell lysates may be assayed for such activity. In either case biological assays such as the growth inhibition assay described herein or the stimulation of anchorage independent growth in target cells, also described herein or the like may be used.

Once a clone that produces high levels of biologically active, mature TGF-β1 is identified, the clone may be expanded and the TGF-β1 may be purified using techniques well known in the art. Such methods include immunoaffinity purification, chromatographic methods including high performance liquid chromatography, and the like.

Despite the fact that the amino acid sequence of the simian TGF-β1 precursor differs somewhat from that of the predicted human precursor, the amino acid sequence of the biologically active mature form of the simian TGF-β1 produced in accordance with the invention is identical to that of the human mature form. In addition, the simian TGF-β1 of the invention has a biological activity that is indistinguishable from that of natural TGF-β1. This indicates that the expression/host cell systems of the invention are capable of processing the expression product so that a molecule having biological activity identical to that of authentic natural TGF-β1 is produced. As a result, the simian TGF-β1 produced in accordance with the invention may be used for all applications in which TGF-β1 may be used.

## 5.4. INITIAL CHARACTERIZATION OF THE TGF-β1 GENE PRODUCT

The amino portion of the precursor region of TGF-β1 from human, rodent and simian sources show a high degree of homology (Derynck et al., 1985, Nature 316:701-705; Derynck et al., 1986, J. Biol. Chem. 261:4377-4379; Sharples et al., 1987, DNA 6:239-244), suggesting an important biological function may be associated with this part of the molecule. The data presented in Section 8, infra, demonstrating that this portion of the TGF-β1 precursor is glycosylated and phosphorylated support this contention since one might assume that a cell would not go through the expense of performing these secondary modifications were it not for a specific function. These modifications may be important for dimerization of the precursor or for directing its movement out of the cell: perhaps this phospho-glycoprotein is free to perform other intra- or extracellular functions. There is evidence which suggests that glycosylation of the precursor is involved in the transport of mature TGF-β1 out of the cell.

## 5.5. THE TGF-β1 PRECURSOR: CELLULAR PROCESSING, STRUCTURAL NATURE AND POSSIBLE FUNCTION

The cDNA cloning of TGF-β1 described in section 6, infra, suggests that the molecule undergoes a variety of post translational processing events before secretory exit (Derynck et al., 1985, Nature 316:701-705; Derynck et al., 1986, J. Biol. Chem. 261:4377-4379; Sharples et al., 1987, DNA 6:239-244). Protein purification and amino terminal sequencing techniques were used to characterize the recombinant TGF-β1 proteins produced and released by CHO-TGF-β1-3-2000 cells (Section 8, infra).

The amino-terminals sequence analysis of isolated precursor and mature TGF-β1 polypeptides, described in section 8.3 and 8.4, infra provides information about these proteolytic processing events. Precursor polypeptides isolated from reduced polyacrylamide gels generated a major protein sequence which, according to that predicted from the simian cDNA (Sharples et al., 1987, DNA 6:239-244), begins at Leu-30 of the TGF-β1 precursor. No heterogeneity in the amino-terminal sequence was observed indicating a specificity in proteolytic processing. This result implicates the Gly-29/Leu-30 peptide bond as the signal peptidase cleavage site and is consistent with that predicted by the signal peptide prediction method of von Heijne (von Heijne, 1986, Nucleic Acids. Res. 14:4683-4690). In addition to signal peptide cleavage, purification and examination of the mature TGF-β1 (Sections 8.2 and 8.4, infra) revealed that rTGF-β1 is proteolytically processed at the predicted dibasic protease site (Sharples et al., 1987, DNA 6:239-244) resulting in a mature polypeptide. Furthermore, protein sequence analysis of the carboxy-terminal CNBr fragment of mature TGF-β1 suggests an intact molecule. Thus, CHO cells possess the appropriate proteases necessary for correctly processing pre-pro-TGF-β1.

The major biological activity secreted by transfected CHO cells is the mature dimeric growth factor. The results presented in Section 8.2, infra indicate that greater than 95% of the activity present in CHO conditioned medium copurifies with mature rTGF-β1, whereas less that 5% copurifies with the larger rTGF-β1 precursor. In addition, recombinant TGF-β1 behaved identically to nTGF-β1 and possessed an identical specific biological activity (Section 8.5, infra). The rTGF-β1 precursor, in contrast, was 50-fold less active than the mature growth factor. A comparison of mink lung inhibition profiles show a slightly altered dose-response curve suggesting a different receptor affinity for the precursor as compared to the mature TGF-β1.

Although the results presented in Section 8.5, infra and discussed above suggest that the rTGF-β1 precursor is biologically active, an indepth structural analysis has revealed some intriguing anomalies complicating definitive interpretations. Protein sequence analysis and SDS-PAGE reveal that the isolated precursor consists of pro-TGF-β1, mature TGF-β1, and the pro region of the precursor interlinked by disulfide bonds. Chemical cleavage of this disulfide linked mixture with CNBr and separation of CNBr peptides clearly show that the Cys-33 of the precursor forms a disulfide bond with one cysteine residue of mature TGF-β1 (FIG. 20A). The existence of the monomeric chain of TGF-β1 interconnected with precursor sequences limits any definite conclusions which can be made concerning the biological activity of the intact precursor. The formation of this disulfide-linked complex in CHO cells raised a question about its significance in tissues and cells which secrete TGF-β1 naturally. The very high level secretion of rTGF-β1 polypeptides by CHO cells may lead to an unnatural crosslinking due to improperly folded rTGF-β1 resulting in an expression artifact. Alternatively, disulfide-linked precursor complex may represent an important intermediate in TGF-β1 processing. It is interesting to note that disulfide-linked precursor complexes have been observed in latent isolated forms of TGF-β1 (Miyazono et al., 1988, J. Cell Biochem. Suppl. 12A:200; Wakefield et al., 1987, J. Biol. Chem. Suppl. 11A:46).

Based on results presented in Section 8.2-8.5, infra, the processing of pre-pro-TGF-β1 in transfected CHO cells is proposed (FIG. 20B). While the proposed processing scheme is not complete, it emphasizes several of the steps which have been at least partially defined. Although the order of the various processing steps has not been characterized completely, they are described as occuring in succession to facilitate comprehension. The first step involves signal peptide cleavage at the Gly-29/Leu-30 peptide bond. This cleavage event most likely occurs co-translationally during transit of the precursor through the rough endoplasmic reticulum membrane (Blobel and Dobberstein, 1975, J. Cell. Biol. 67:835-851; Walter et al.,

1984, Cell 38:5-8). Following cleavage of the signal peptide, core glycosylation units (Rothman et al., 1978, Cell 15:1447-1454) are added to pro-TGF-β1 at each of the predicted N-glycosylation sites located at Asn-82, Asn-136 and Asn-176 (Sections 8.1 and 9). The core glycosylated pro-TGF-β1 is then sequentially processed during transit through the golgi to yield a phosporylated glycoprotein, containing complex, sialated oligosaccharides. At some stage during synthesis or transit, proteolytic cleavage at the dibasic residue and disulfide isomerization occurs, releasing mature TGF-β1.

The results described in section 9, infra, show the presence of mannose-6-phosphate in the TGF-β1 precursor and raise the possibility that the precursor possesses an independent function.

Mannose-6-phosphate, a phosphorylated sugar analog, appears to play a fundamental role in the targeted transport and intercellular exchange of lysosomal enzymes (von Figura, 1986, Ann. Rev. Biochem. 55: 167-193). Specific receptors which recognize the mannose-6-phosphate residues of lysosomal enzymes have been identified and are essential components of the transport system. Secreted lysosomal proteins containing mannose-6-phosphate have been identified in the conditioned medium of tissue culture cells (Gal and Gottesman, 1986, J. Biol. Chem. 261:1760-1765; Capony et al., 1981, J. Cell. Biol. 104:253-262; Baumbach et al., 1984, Proc. Natl. Acad. Sci. USA 81:2985-2989; Sahagian and Gottesman, 1982, J. Biol. Chem. 257:11145-11150). All of these proteins, however, exhibit acid hydrolase activity. The mannose-6-phosphate residues of the TGF-β1 precursor may direct pro-TGF-β1 to lysosomes for proteolytic processing to yield mature TGF-β1. Alterntively, the mannose-6-phosphate residues may function to target the cleaved TGF-β1 precursor to lysosomes for degradation.

It has recently been reported that the cation-independent mannose-6-phosphate receptor is identical to the insulin-like growth factor II (IGF-11) receptor (Morgan et al., 1987, Nature 329:301-307; Roth et al., 1987, Biochem. Biophys. Res. Comm. 149:600-606; MacDonald, 1988, Science 239:1134-1137). This receptor appears to be bifunctional, containing separate binding sites for IGF-II and mannose-6-phosphate. Although the biological significance of a single receptor whcih binds IGF-II and proteins containing mannose-6-phosphate is unclear, this bifunctional receptor may play important roles for signal transduction and/or for targeted sorting of receptor bound proteins. Proliferin, a prolactin-related glycoprotein, thought to be an autocrine growth regulator (Lee and Nathens, 1987, Endocrinology 120:208-213), has been shown to contain mannose-6-phosphate and to bind tightly to IGF-II/mannose-6-phosphate receptors (Lee and Nathens, 1988, J. Biol. Chem. 263:3521-3527). It is possible that the TGF-β1 precursor interacts specifically with this bifunctional or other mannose-6-phosphate cell surface receptor.

## 5.6. BIOLOGICAL ACTIVITY OF TGF-β1

The mature TGF-β1 product of the invention is a potent inhibitor of tumor cell growth in vitro and in vivo. Experiments measuring the anti-proliferative effect of TGF-β1 on mink lung epithelial cells demonstrated that the recombinant TGF-β1 of the invention (rTGF-β1) and natural TGF-β1(nTGF-β1) have identical specific activities (Section 8.5, infra). In addition, the dose response curves of rTGF-β1 and nTGF-β1 are virtually indistinguishable.

The results in Section 8.6., infra demonstrate that both natural TGF-β1 and the rTGF-β1 of the invention inhibit tumor growth in vivo. The tumorstasis observed in TGF-β treated human lung tumors is also accompanied by induction of a more differntiated-like phenotype. Mucous secreting cells, a minor population in the mock-treated, poorly differentiated tumors, dominated TGF-β1 treated tumors. Consistent with the secretory role of the goblet cell in normal tissues (Robbins and Angell, 1971, Basic Pathology, W.B. Sander G., Philidelphia, PA) and thus a more differentiated state, is the enhanced expression of mucin-like and hyaluronic acid products found in TGF-β1 treated tumors. Innumerable other histological indices also suggest the TGF-β1 mediated induction of the differentiated phenotype, including the pronounced display of columnar epithelial cell organization surrounding the vessel wall and increased deposition of extracellular matrix, i.e., collagen.

The powerful inhibitory effect of rTGF-β1 on the growth and differentiation of human lung adenocarcinomas in athymic nude mice provide additional evidence that the rTGF-β1 of the invention may find use in the development of novel, perhaps less cytotoxic, cancer therapy regimens.

## 6. EXAMPLE: cDNA CLONING OF TGF-β1 PRECURSOR

The examples that follow describe the cDNA cloning of the TGF-β1 precursor coding sequence from an African Green monkey cell line (BSC-40, a subline of BSC-1 cells) previously shown to produce high levels of a growth inhibitor functionally related to TGF-β1. Exceedingly strong sequence homology between the simian precursor and the gene product predicted for both human and mouse TGF-β1 were found.

### 6.1. MATERIALS AND METHODS

The following procedures were used to clone the cDNA encoding the TGF-β1 precursor.

#### 6.1.1. GROWTH OF CELLS AND RNA EXTRACTION

BSC-40 cells were grown in Dulbecco's modified Eagle medium containing 10% fetal calf serum. MCF-7 cells were grown in the same medium containing 6 units/ml insulin. Polyadenylated RNA was isolated from these cells by oligo[dT]-cellulose chromatography as described (Purchio et al., 1979, J. Virol. 29:763-769).

#### 6.1.2. cDNA LIBRARY CONSTRUCTION AND SCREENING

Double stranded cDNA was synthesized from BSC-40 polyadenylated RNA as described (Maniatis et al., 1982, Molecular Cloning: A Laboratory Manual, Cold Spring Harbor Laboratory, Cold Spring Harbor, NY, 371-372) and after treatment with EcoRI methylase was ligated to oligonucleotide linkers containing an EcoRI restriction enzyme recognition site (EcoRI linkers). The cDNA was digested with EcoRI and fractionated by chromatography on Sephacryl S-1000. cDNA fractions greater than 750 base pairs (bp) were pooled and ligated into lambda gt10 which had been cut with EcoRI (Davis et al., 1980, A Manual for Genetic Engineering: Advanced Bacterial Genetics; Cold Spring Harbor Laboratory, Cold Spring Harbor, NY), packaged (Grosveld et al., 1981, Gene 13:227-237) and plated on E. coli $C_{600}$ $rK^-mK^+hfl$. The library was screened by plaque hybridization (Bentonet al., 1977, Science 196:180-182) to a [$^{32}P$]-labelled oligonucleotide probe [5′-CACGCAGCAGTTCTTCTCCGTGGAGCTGAAGCAATA-3′] complementary to codons 6 through 17 of the mature TGF-β1 molecule (Derynck et al., 1985, Nature 316:701-705). Several cDNA clones were isolated after tertiary screening and subcloned into pBR322. One clone (pTGF-β1-2) containing a 1600 bp insert was subcloned into the M13mp18 and M13mp19 cloning vectors (Yanisch-Perron et al., 1985, Gene 33:103-119) and both strands were sequenced using the dideoxy chain-termination method (Sanger et al., 1977, Proc. Natl. Acad. Sci. USA 74:5463-5467).

#### 6.1.3. NORTHERN BLOT ANALYSIS

Polyadenylated RNA was isolated from MCF-7 cells and BSC-40 cells as described (Purchio et al., 1979, J. Virol. 29:763-769), fractionated on a 1% agarose-formaldehyde gel (Lehrach et al., 1977, Biochemistry 16:4743-4751), transferred to a nylon membrane (Hybond, Amersham) and hybridized to [$^{32}P$]-labeled pTGF-β1-2 probe. Hybridization was carried out at 42°C in 50% formamide containing 0.9M NaCl, 50mM sodium phosphate, 5mM EDTA, 0.1% SDS, 4XDenharts solution (Maniatis et al., 1982, Molecular Cloning: A Laboratory Manual, Cold Spring Harbor Laboratory, Cold Spring Harbor, NY, 371-372), 0.4 mg/ml yeast tRNA and 0.25 mg/ml denatured calf thymus DNA. Filters were washed at 65°C in 0.25XSSC (Maniatis et al., 1982), 0.1%SDS, dried and exposed to Cronex-4 X-ray film (DuPont) with the aid of lightening plus intensifier screens (DuPont).

## 6.2. RESULTS

The cDNA library constructed in lambda gt10 using polyadenylated RNA from BSC-40 cells as described above was screened with a 36-mer deoxyoligonucleotide complementary to codons 6 through 17 of the mature TGF-β1 molecule (Derynck et al., 1985, Nature 316:701-705). Due to the close similarity between bovine TGF-β1 (Roberts et al., 1983, Biochemistry 22:5692-5698) and human TGF-β1 (Derynck et al., 1985, Nature 316:701-705) we selected a probe derived from human sequences to screen the simian TGF-β1 cDNA clones.

A total of 13 positive clones were identified after three rounds of plaque purification. A 1600 bp clone, pTGF-β1-2, which appeared by restriction enzyme analysis to contain the entire TGF-β1 precursor coding region, was chosen for sequencing. The DNA sequence, along with the deduced amino acid sequence, is shown in FIG. 1. A single open reading frame was found coding for a 390 amino acid polypeptide. The mature TGF-β1 polypeptide consists of the carboxy terminal 112 residues. This arrangement is the same as that described for human TGF-β1 (Derynck et al., 1985, Nature 316:701-705) and mouse TGF-β1 (Derynck et al., 1986, J. Biol. Chem. 261:4377-4379). The simian cDNA clone is 98% homologous to the human cDNA clone throughout the coding region with only 27 mismatches (FIG. 1) and one gap of three bases.

Within the 5′-noncoding region, the DNA sequences of the human and simian TGF-β1 are 91% homologous with 3 gaps while the 3′-noncoding regions are 94% homologous with no gaps. Just upstream of the 5′ initiating codon is a potential stem structure which is also present in the human cDNA clone but not in the mouse clone. An apparent insertion within this region in the mouse clone would inhibit formation of this structure. Like the human and mouse clones, the simian cDNA clone was not full length as no 3′-polyadenylated track was identified. The G-C rich nature of the 3′-end of the TGF-β1 precursor mRNA may have resulted in a secondary structure which prevented DNA synthesis through this region. The 3′-noncoding region exhibits a remarkable repetition of the purine sequence, CCCC, following the termination codon. This sequence occurs nine times in the human sequence with an additional repeat containing one base difference. Both the simian and murine clones contain 8 repetitions with an additional 2 containing a single base difference.

Northern blot analysis using the TGF-β1-2 probe showed hybridization of a major 2.5Kb polyadenylated RNA species from BSC-40 cells and from the human mammary carcinoma cell line MCF-7 (FIG. 2). This is the same size as the major transcript found in human cell lines (Derynck et al., 1985, Nature 316:701-705) and mouse cell lines (Derynck et al., 1986, J. Biol. Chem. 261:4377-4379). Minor bands at 4kb and 1.45kb can also be seen in lane 2 of FIG. 2.

The amino acid homology between the human and simian TGF-β1 precursor proteins is also shown in FIG. 1. Only five amino acid changes occur, all of which are found within the amino terminal precursor region: there is complete homology between the simian and human mature TGF-β1 proteins at the amino acid level. By contrast, only 84% homology (at the amino acid level) was found to exist between the simian and mouse precursor TGF-β1 molecules; the differences include the following: one amino acid change, a serine (mouse) to an alanine (simian) was found in the mature TGF-β1 coding region. The human gene codes for an extra amino acid (arginine, position 158) in the precursor which is neither present in the simian nor murine clone. Presumably this change is due to an insertion within the human gene which was sequenced (Derynck et al., 1985, Nature 316:701-705). The close identity between the precursor region of the monkey, mouse and human TGF-β1 protein suggests that this part of the molecule may also have an important biological function.

The carboxy terminus of the precursor contains the 112 amino acid mature form of simian TGF-β1 and is identified by its homology to the corresponding human gene product. This cysteine rich (9 residues) region of the precursor represents the secreted form of TGF-β1 and does not contain any putative glycosylation sites, whereas three potential N-glycosylation sites (Asn at positions 82, 136 and 177) can be identified in other regions of the precursor. It is known that the TGF-β1 homodimer, both of human and rodent origin, requires intact disulfide bonds to maintain activity (Messague, 1984 J. Biol. Chem. 259:9756-9761). The amino-terminus of mature human TGF-β1 as established by primary sequence analysis (Derynck et al., 1985, Nature 316:701-705) is preceded by an Arg-Arg dipeptide. This similar cleavage site is also present in an identical position in the simian homolog. Cleavage either during or after the translational process, concomitant with either or both intrachain and interchain disulfide bond formation, would result in the genesis of the active homodimer (dimerization). In this regard, the TGF-β1 precursor contains a leucine rich region of 14 hydrophobic amino acids (positions 8-21, FIG. 1) which could function as a signal peptide and play a role in the secretion/processing cascade.

## 7. EXAMPLE: EXPRESSION OF TGF-β1

The examples that follow describe the expression of active TGF-β1 in Chinese Hamster Ovary (CHO) cells transfected with a recombinant plasmid containing the coding sequence for TGF-β1 under the control of simian virus 40 (SV40) expression elements. The experiments described herein demonstrate that a number of CHO transfectants produced and secreted high levels of TGF-β1. The TGF-β1 released by the transfected cells demonstrated biological activity comparable to that of authentic natural TGF-β1 as determined by growth inhibition assays using target cells that are sensitive to TGF-β1.

### 7.1. MATERIALS AND METHODS

#### 7.1.1. CELL CULTURE

Dihydrofolate reductase (dhfr)-deficient Chinese hamster ovary (CHO) cells (Urlaub and Chasin, 1980 Proc. Natl. Acad. Sci. U.S.A. 77: 4216) were propagated in Ham's F-12 medium (Gibco Laboratories, NY) supplemented with 10% fetal bovine serum (FBS) and 150 ug/ml of L-proline. Penicillin and streptomycin were included at 100 U/ml and 100 ug/ml, respectively. CHO transfectants were grown in Dulbecco's modified Eagle's medium containing the same supplements as those listed above. CHO cells and their derivatives were routinely passaged by trypsinization at a 1:5 splitting ratio.

Methotrexate (Sigma, MO) was prepared at a stock concentration of 10 mg/ml in water. Dilute NaOH (0.2 M) was added in order to solubilize the drug (final pH of 6). The stock was filter-sterilized and stored at -20° C. Stock solutions of methotrexate in media (100 uM) were kept at 4° C for no longer than 1 month.

#### 7.1.2. DNA MANIPULATIONS AND PLASMID CONSTRUCTIONS

Restriction enzymes, T4 DNA ligase, calf intestinal phosphatase, the Klenow fragment of DNA polymerase I and other DNA reagents were purchased from Bethesda Research Laboratories, MD. Standard DNA manipulations were performed as outlined in Maniatis, T., et al., 1982, Molecular Cloning: A Laboratory Manual, Cold Spring Harbor Laboratory, New York.

Plasmid pSV2 (β1-TGF-dhfr), which contains the simian TGF-β1 cDNA and the mouse dhfr gene in tandem as well as intervening SV40 sequences, was constructed as outlined in FIG. 3. The pSV2-β1-TGF plasmid was prepared initially. The 1378 bp PstI-EcoRI fragment of theTGF-β1 cDNA containing the entire coding region for TGF-β1 protein was inserted into the polylinker region of pSP65 in order to place a HindIII restriction site 5′ to the PstI recognition sequence. The resulting plasmid was then digested with EcoRI, repaired to blunt ends with the Klenow fragment of DNA polymerase I, and the HindIII-EcoRI (blunt) fragment containing the TGF-β1 coding sequence was isolated. Plasmid pSV2-β1-TGF was then constructed by inserting the HindIII-EcoRI (blunt) fragment into pSV2-neo in place of the neomycin resistance gene (neo), by ligation into the HindIII-HpaI fragment of the vector.

Construction of pSV2-(β1-TGF-dhfr), at this point, required two steps. The first step required the isolation of the NdeI-EcoRI (blunt) fragment of pSV2-β1-TGF. This was accomplished by digesting the pSV2-β1-TGF plasmid with EcoRI, blunting the ends with the Klenow fragment of DNA polymerase I, cutting the blunted vector with NdeI and PvuI, and isolating the 2.6 kb NdeI- EcoRI (blunt) fragment. PvuI digestion was necessary so that a contaminating plasmid fragment would not copurify upon agarose gel electrophoresis. The final step in the construction was the insertion of the NdeI-EcoRI (blunt) fragment containing the β1-TGF cDNA and SV40 sequences into one NdeI-PvuII fragment of pSV2-dhfr. The resulting pSV2-(β1-TGF-dhfr) plasmid contains a unique NdeI site which can be utilized to linearize the DNA.

## 7.1.3. DNA TRANSFECTIONS

Approximately 24 hours after seeding $10^5$ dhfr-deficient CHO cells onto 100 mm dishes, the cultures were transfected with 20 ug of NdeI linearized pSV2-(β1-TGF-dhfr) plasmid as a calcium phosphate precipitate (Wigler, M., et al., 1979, Proc. Natl. Acad. Sci. U.S.A. 76:1373-1376). Briefly, 20 ug of linearized DNA was added to 1 ml of 250 mM sterile $CaCl_2$. A 1 ml portion of 2X HEPES solution (280 mM NaCl, 50 mM HEPES, 1.5 mM sodium phosphate, pH 7.1) was then added dropwise, and the mixture was allowed to sit on ice for 30 minutes. The precipitate was then dispersed dropwise over the cells containing 10 ml of the F12 media. After incubation at 37°C for 4 hours, the media was removed and replaced with 10 ml of F12 media containing 25% glycerol for 90 seconds at room temperature. Cells were rinsed once with 20 ml of F12 media and incubated in the nonselective F12 media (20 ml) for an additional 48 hours. Selection for dhfr expressing transfectants was accomplished by replacing the media with DMEM supplemented with 10% dialyzed FBS (Gibco, N.Y.) and 150 ug/ml L-proline. Colonies were observed after culturing the cells 10-14 days in the selection media. Ten colonies were aspirated by a pasteur pipet and expanded.

## 7.1.4. SELECTION OF METHOTREXATE RESISTANT CELLS

Dihyrofolate reductase (dhfr) amplified cells were derived from the primary transfectants essentially as described (Gasser, C.S. and Schimke, R.T., 1986, J. Biol. Chem. 261:6938-6946). After expansion, $10^5$ cells were seeded onto 100 mm dishes and adapted to increasing concentrations of methotrexate. The initial concentrations of methotrexate were 50, 100, and 200 nM. The plate containing visible colonies at the highest methotrexate concentration was trypsinized and adapted to that concentration of methotrexate for at least two additional 1:5 cell passages. Cells ($10^5$) were then seeded onto 100 mm dishes in 2,5, and 10-times the concentration of methotrexate. The dish containing visible colonies was again trypsinized and adapted in the methotrexate containing medium. Cells were frozen back at various stages of amplification in media containing 40% FBS, 10% dimethyl sulfoxide and 50% DMEM. Methotrexate was not included in the freezing media.

## 7.1.5. QUANTITATION OF BETA-TGF MESSAGE LEVELS

TGF-β1 mRNA levels were assessed using solution hybridization (Uhler, M.D., et al., 1986, Proc. Natl. Acad. Sci. U.S.A. 83:1300-1304). The 600 bp SmaI-SmaI fragment of the β1-TGF cDNA was cloned into pSP65 and used to make [$^{32}$P]-labeled complementary RNA by SP6 RNA polymerase as detailed by the manufacturer (Promega Biotech, Madison WI). Single stranded M13 DNA containing the entire β1-TGF cDNA, in the same sense as mRNA, was utilized as standards. Total nucleic acid was isolated from transfected cells by proteinase K digestion and phenol/chloroform extraction (McKnight, G.S., et al., 1980, J. Biol. Chem. 255:144-147). DNA content of total nucleic acid samples was measured by the dye binding assay (Labarca, C., and Paigen, K., 1980, Anal. Biochem. 102:344-352). The number of molecules of mRNA per cell was estimated by comparison with the M13 β1-TGF standards assuming 7 picograms of DNA per cell.

## 7.1.6. GROWTH INHIBITION ASSAY

Mink lung epithelial cells, Mv 1 Lu (Accession Number CCL-64, American Type Culture Collection), which are extremely sensitive to TGF-β1 were utilized for the growth inhibition assay. The assay was performed using the thymidine analog 5′-[$^{125}$I]-iodo-2′deoxyuridine ($^{125}$IdU) to assess DNA synthesis. One unit of activity was defined as the amount required to inhibit 50% incorporation of $^{125}$IdU compared to untreated CCL-64 cells. Using isolated TGF-β1 as a standard, one unit of activity generally corresponded to 80-100 pg/ml of TGF-β1.

To assay transfected cells for secretion of active TGF-β1, serum free supernatants were collected from one 24 hour collection on confluent cultures of cells and dialyzed extensively against 0.2 M acetic acid. The acetic acid was removed by lyophilization and the sample was re-dissolved in sterile complete culture medium for assays.

## 7.1.7 STIMULATION OF ANCHORAGE INDEPENDENT GROWTH

Supernatants were tested for their ability to stimulate normal rat kidney fibroblasts (NRK; clone 49) to grow as colonies in soft agar. The soft agar assay was performed as described (Twardzik and Sherwin, 1985, J. Cell. Biochem. 28:289-297; Delarco and Todaro, 1978 Proc. Natl. Acad. Sci. U.S.A. 75:4001-4005) using acid-dialyzed supernatants.

## 7.1.8. PEPTIDE SYNTHESIS AND PRODUCTION OF ANTIBODIES

Peptides were synthesized by solid phase techniques on a Beckman 990 instrument, and cleaved from the resin as previously described (Gentry, L.E., et al., 1983, J. Biol. Chem. 258:11219-11228; Gentry, L.E. and Lawton, A., 1986, Virology 152:421-431). Purification was accomplished by preparative high performance liquid chromatography. The composition of the peptides was confirmed by amino acid analysis.

Synthetic peptides were conjugated to bovine gammaglobulin through the cysteine residue. Coupling reactions were essentially as descirbed (Gentry and Lawton, 1986, supra). The efficiencies of peptide conjugations ranged from 8 to 26 molecules of peptide covalently attached per molecule of gammaglobulin.

New Zealand white rabbits were primed at three to six sites by combined subcutaneous and intradermal inoculations with the peptide conjugates (100 ug equivalents of peptide) emulsified in Freunds complete adjuvant. Booster inoculations were administered at 2-3 week intervals. Bleedings were taken 7-14 days following the boosts.

## 7.1.9. IMMUNOBLOTTING

Proteins were fractionated on 7.5%-17.5% gradient SDS-polyacrylamide gels and transferred to unmodified nitrocellulose (0.45 um; Schleicher and Schuell) for 14-18 hours at 200 mA at 4°C (Burnette, W. N., 1981, Anal. Biochem. 112:195-203). Excess binding capacity of the nitrocellulose was blocked by incubation with 2.5% BLOTTO (Johnson, D.A., et al., 1984, Gene Anal. Techn. 1:3-8) in phosphate-buffered saline (PBS) containing 0.2% NP-40. Rabbit anti-serum diluted 1:75 in 2.5% BLOTTO was incubated with the blocked nitrocellulose sheets for 2 hours at room temperature. After washing away excess antibody by five 5-minute washes in 2.5% BLOTTO, the nitrocellulose sheets were incubated with alkaline phosphatase-conjugated Protein A diluted 1:500 in 2.5% BLOTTO. Following a one hour incubation, the nitrocellulose sheets were washed 5 times in PBS (5 minute washes) containing 0.2% NP-40 and developed (Leary et al., 1983, Proc. Natl. Acad. Sci. U.S.A. 80:4045-4049).

## 7.1.10. NORTHERN BLOT ANALYSIS

Cytoplasmic polyadenylated RNA was prepared from tissue culture cells as described (Purchio and Fareed, 1979, J. Virol. 29:763) and fractionated on a 1% agaroseformaldehyde gel (Lehrach et al., 1977, Biochemistry 16:4743). The RNA was transferred to a Hybond nylon membrane (Amersham) and hybridized to radiolabeled pTGF-β1-2 probe. Hybridization was performed in 0.9 M NaCl, 50 mM sodium phosphate (pH 7.0), 5 mM EDTA, 0.1% SDS, 4x Denhardts, 0.4 mg/ml yeast tRNA, 0.25 mg/ml calf thymus DNA and 50% formamide, for 20 hours at 42°C. Filters were washed four times in 0.25 x SSC at 65°C (30 minutes/wash), dried and exposed for autoradiography.

## 7.1.11. SOUTHERN BLOT ANALYSIS

High molecular weight DNA was isolated from the pelleted nuclei obtained after RNA extraction (Purchio and Fareed, supra). The nuclear pellet was dispersed in TE buffer and then adjusted to 1% sodium dodecyl sulfate/10mM Tris-HCl, pH 7.4/10mM EDTA. Proteinase K was added to 100 ug/ml and incubated at 37° C for 16-18 hours. After the DNA was phenol/chloroform extracted and ethanol precipitated, residual RNA was removed by digestion with DNAse "free" RNAse A for 2 hours in TE buffer, followed by extensive dialysis versus TE.

For the Southern blot analysis, DNA was digested with the appropriate restriction enzyme and blotted to Hybond nylon membranes (Southern, E.M., 1975, J. Mol. Biol. 98:503-517). Hybond nylon membrane filters were hybridized at 65° C for 20 hours in hybridization buffer containing 5 x $10^6$ cpm/ml of denatured nick-translated EcoRI-EcoRI fragment of β1-TGF cDNA obtained from pTGF-β1-2 and washed as detailed by the manufacture (Amersham).

## 7.2. EXPRESSION OF TGF-β1 IN CHO CELLS

In order to express high levels of recombinant bioactive TGF-β1, we utilized the pSV2 vectors originally described (Mulligan, R.C. and Berg, P., 1981, Mol. Cell. Biol. 1:449-459: Subramani, S., et al., 1981, Mol. Cell. Biol. 1:854-864) to prepare an expression plasmid (pSV2-β1-TGF-dhfr) that places the simian TGF-β1 and mouse dihydrofolate reductase cDNAs in tandem within the same plasmid (FIG. 3). The expression plasmid carries a large portion of the TGF-β1 cDNA which encodes the complete TGF-β1 precursor molecule. The physical linkage of these two genes within the same plasmid allows for coamplifiction of TGF-β1 sequences during dhfr amplification. The signals required for transcription initiation, termination, polyadenylation, and splicing for each cDNA are identical and are supplied by simian virus 40 (SV40) DNA sequences.

pSV2-β1-TGF-dhfr was transfected into dhfr-deficient CHO cells as a calcium phosphate precipitate. Ten CHO transfectants expressing the dhfr phenotype were isolated by propagation in selective medium. All ten transfectants displayed TGF-β1 mRNA as assayed by solution hybridization. To increase the level of expression of TGF-β1, cells derived from the ten initial transfectants were selected stepwise in increasing concentrations of methotrexate (MTX). At various stages of amplification, cells were assayed for the level of TGF-β1 mRNA by solution hybridization. Four of the ten initial transfectants showed increased levels of TGF-β1 mRNA. Here, we describe one of these clones (TGF-β1-3), which through MTX amplification, displayed very high levels of TGF-β1 mRNA.

Table I shows the number of TGF-β1 mRNA copies per TGF-β1-3 cell at various stages of amplification. The CHO cells at various stages of amplification will be referred to as TGFβ1-3-0, TGFβ1-3-200 and correspond to CHO cells selected in 0,2 µM and 20 µM MTX respectively.

TABLE I

EXPRESSION OF TGF-β1 mRNA IN CHO TRANSFECTANTS

| Cultured Cells | Number of TGF-β1 mRNA Copies Per Cell[1] MTX Concentration (μM) (0) | (2) | (20) |
|---|---|---|---|
| TGF-β1-3 | 350 | 34,000 | 76,000 |
| CHO#7 | 700 | -- | -- |
| Non transfected CHO | 20 | | |

[1] Number of mRNA copies per cell was determined by solution hybridization. Calculations were based on the approximation that the DNA content of one cell is 7 picograms.

As controls, we included non-transfected CHO cells and a CHO transfectant (CHO #7) initially shown to express recombinant TGF-β1 at low levels. CHO #7 was obtained by cotransfection with pSV2-β1-TGF (FIG. 3) and pSV2-neo, followed by selection in Gentecidin (Gibco, NY). The initial TGF-β1-3 transfectant displayed mRNA levels of β1- TGF similar to those of CHO #7 and approximately 20 times greater than non-transfected CHO cells. However, after MTX selection, the number of β1-TGF mRNA copies observed in TGF-β1-3 cells dramatically increased approaching nearly 80,000 copies per cell at 20 μM MTX. This represented a greater than 200-fold amplification with respect to the initial TGF-β1-3 transfectant and an almost 4000-fold amplification with respect to the non-transfected CHO cells.

A northern blot of poly (A)+ selected mRNA from TGF-β1-3 cells at various stages of amplification is shown in FIG. 4A. Non-transfected CHO cell mRNA was also included to reveal the low levels of endogenous 2.5 Kb TGF-β1 mRNA present in these cells (asterisk, FIG. 4A). This low level expression of TGF-β1 mRNA has also been observed in several other cell lines. The TGF-β1-3 transfectants revealed large amounts of hybridizable RNA migrating at the predicted size of 2 Kb.

The most likely mechanism for the dramatic increase of TGF-β1 mRNA in the TGF-β1 -2 cells after MTX selection is a result of amplification. In order to examine for gene amplification, DNA was isolated from the TGF-β1-3 cells at various stages of MTX selection, digested with restriction enzymes, transferred to Hybond nylon membranes, and hybridized to [$^{32}$P]-labeled TGF-β1 DNA. Both restriction enzymes EcoRI and BamHI cut within the introduced plasmid twice generating linear copies of plasmid DNA lacking flanking sequences. Since one of the BamHI sites resided within the simian TGF-β1 cDNA, digestion with this enzyme should release two fragments which hybridize to the nick-translated probe.

The results of the Southern blot are shown in FIG. 4B. In the MTX selected cells, strong hybridization to the TGF-β1 probe was observed. The size of these fragments corresponded precisely to the predicted size of the TGF-β1 containing fragments of pSV2-TGF-β1-dhfr (a 4.7 Kb fragment derived by EcoRI digestion and 4.4 and 0.51 Kb fragments derived by BamHI digestion). Similar digestion products were also observed at lower levels in the initial non-selected transfectant (TGF-β1-3/0). Densitometric scans comparing the different TGF-β1-3 cells revealed at least a 15-and 35-fold amplification of TGF-β1 sequences in TGF-β1-3/200 and TGF-β1-3/2000 cells, respectively.

### 7.3. DETECTION OF SECRETED BIOACTIVE RECOMBINANT TGF-β1

To determine whether the TGF-β1 protein was made and secreted in the transfected TGF-β1-3 cells, conditioned medium was collected from these cells and tested for bioactive material. A sensitive and specific bioassay based on the ability of β1-TGF to inhibit growth of mink lung epithelial cells (CCL-64) was the initial assay used. A typical standard dose response curve using highly purified authentic human TGF-β1 is shown in FIG. 5A. In this assay, 50% inhibition of growth of mink lung cells is typically observed at 8-12 picograms (80-120 pg/ml) of TGF-β1. Conditioned medium collected from TGF-β1-3 cells at all stages of amplification exhibited the ability to inhibit the growth of CCL-64 cells. The dose response curves of these supernatants are shown in FIG. 5B. These show similar slopes to the inhibition curves observed for authentic natural β1-TGF.

Table II shows the levels of bioactive material present in culture supernatants of TGF-β1-3 cells calculated from the curves shown in FIG. 5B.

**TABLE II**

**CONCENTRATION OF BIOLOGICALLY ACTIVE TGF-β1 HARVESTED FROM THE CHO-TRANSFECTANTS[1]**

| Cultured Cells | Concentration of TGF-β1 in Conditioned Media of CHO Transfectants based upon Bioactivity MTX Concentration (μM) (0) | (2) | (20) |
|---|---|---|---|
| TGF-β1-3 | 5.8 ng/ml | 1000 ng/ml | 5600 ng/ml[2] |
| CHO #7 | 25 ng/ml | --- | --- |
| Non-Transfected CHO | 1 ng/ml | --- | --- |

[1] Concentration of bioactive material in cell-free spernatants is expressed in ng/ml as determined from the data presented in Figure 2B. Calculations were based on 10 picograms of native β-TGF to elicit a 50% inhibition of CCL-64 cells.

[2] Absolute amounts of TGF-β produced by the TGF-β1-3/200 cell line varied from between 1.5 mg/l to 6.5 mg/l. This may be due to several factors including tissue culture conditions of both the indicator cells (CCL-64) as well as the TGF-β1-3/2000 cells.

Low levels of secreted bioactive TGF-β1 were observed in supernatants of nonamplified TGF-β1-3 cells. These levels were similar to the levels observed in the CHO #7 transfectants. MTX selected TGF-β1-3 cells expressed much higher levels of recombinant bioactive TGF-β1. At 20 μM MTX selection, TGF-β1-3 cells (TGF-β1-3/2000) secreted almost 6 ug/ml of active TGF-β1 into the serum free culture supernatants.

Table III shows the levels of bioactive material secreted in culture supernatants of the TGF-β1-3 transfectant at various stages of MTX selection. Bioactivity was assessed using the mink lung cell inhibition assay and the resulting values were normalized per cell per 24 hours. By contrast to the TGF-β1-3 transfectant, low levels of secreted bioactive TGF-β1 were observed in the supernatants of the non-amplified TGF-β1 transfectants. The highest producer of recombinant TGF-β1 material was the TGF-β1-3/2000 cells. The amount of bioactive recombinant material secreted by $10^7$ of these cells into 5 ml of tissue culture medium over a period of 24 hours approached nearly 30 ug of TGF-β1. The amount of bioactivity detected in the conditioned supernatants for each of the transfectants correlated with the relative level of TGF-β1 mRNA observed in these cell lines.

**TABLE III**

**AMOUNT OF BIOACTIVE TGF-β1 SECRETED BY THE CHO TRANSFECTANTS AT VARIOUS STAGES OF MTX SELECTION**

| Transfectant Number | fg of bioactive TGF-β1 secreted/cell/24h[a] 0 nM MTX | 2 μM MTX | 20 μM MTX |
|---|---|---|---|
| TGF-β1-3 | 2.9 | 500 | 2800[b] |

a The amount of bioactive TGF-β1 secreted by a confluent 100 mm round tissue culture dish into 5 ml of serum-free DMEM was determined using the mink lung cell inhibition assay as described in Materials and Methods. Cell number was determined by counting in a hemocytometer.

b One confluent 100 mm round tissue culture dish (c.a. 1 x $10^7$ cells) of these cells will secrete nearly 30 ug of bioactive TGF-β1 into 5 ml of serum free supernatant.

A second bioassay was also employed to further characterize the secreted recombinant TGF-β1. This assay is based on the ability of TGF-β1, in cooperation with EGF-like molecules, to stimulate the growth of NRK cells in soft agar. The results of this bioassay are shown in Table IV. The activity of the recombinant TGF-β1 is indistinguishable from that of the human natural platelet TGF-β1 used as the control.

TABLE IV

SOFT AGAR COLONY ASSAY COMPARING RECOMBINANT TGF-β1 AND NATURAL TGF-β1[1]

| TGF-β1 (ng/ml) | Number of Colonies formed in Soft Agar: Recombinant TGF-β1 Collected from Conditioned Media | Human Platelet TGF-β1 |
|---|---|---|
| 10.0 | 260 | 251 |
| 5.0 | 263 | 247 |
| 1.0 | 225 | 211 |
| 0.5 | 193 | 182 |
| 0.1 | 50 | 85 |

[1] Normal rat kidney cells were plated in 0.3% Difco noble agar with 1 ng/ml epidermal growth factor (EGF). Eight low power fields were counted per well. No colonies formed when TGF-β1 or EGF were absent from the wells. The amount of recombinant TGF-β1 added was estimated based on the values derived in mink lung epithelial cell inhibition assay. Colony forming activity was assessed using supernatants collected from 20 μM MTX selected TGF-β1-3 cells.

## 7.4. ACID ACTIVATION OPTIMIZE BIOACTIVITY OF SECRETED RECOMBINANT TGF-β1

Many cell types have been found to secrete natural TGF-β1 in a latent form requiring acidification for optimal bioactivity. The bioassays presented in previous figures and tables were performed using acid dialyzed material. In order to determine if the transfected CHO cells secrete a latent biologically inactive form of TGF-β1, serum free supernatants collected from TGF-β1-3/2000 cells were dialyzed against 0.2M acetic acid or 50mM $NH_4HCO_3$ (pH 7) and assayed for mink lung cell inhibitory activity. The results are hown in FIG. 6. Supernatants which received acid dialysis were potent in their ability to inhibit CCL-64 mink lung cells. In contrast the $NH_4HCO_3$ dialyzed samples possessed much lower levels (less than 1%) of inhibitory activity. Supernatants which were first dialyzed against $NH_4HCO_3$ and then treated by a second dialysis against 0.2M acetic acid regained their potent inhibitory activity. The dose response curve fo this material was superimposable with the curve generated by samples receiving only the acid dialysis step.

## 7.5. IDENTIFICATION OF MATURE AND PRECURSOR FORMS OF TGF-β1 IN THE CULTURE MEDIUM OF TRANSFECTANT TGF-β1-3 CHO CELLS

Anti-peptide antibodies directed toward peptide sequences within the predicted TGF-β1 molecule were generated in rabbits using synthetic peptides as immunogens. The peptide sequences which were utilized are shown in FIG. 7 which also indicates their relative locations within the TGF-β1 precursor polypeptide:

TGF-$\beta1_{81-94}$, TGF-$\beta1_{225-236}$, and TGF-$\beta1_{369-381}$. One of the antibodies (anti-TGF-$\beta1_{369-381}$) was directed toward epitopes present within the mature form of the TGF-$\beta$ growth factor. The other two antibodies (anti-TGF-$\beta1_{81-94}$ and anti-TGF-$\beta1_{225-236}$) are precursor-specific and are directed toward peptide sequences present only within the precursor molecule of TGF-$\beta$1.

### 7.5.1. IDENTIFICATION OF MATURE TGF-$\beta$1

Supernatants from the TGF-$\beta$-3 transfectants were collected and tested by immunoblotting with anti-TGF-$\beta1_{369-381}$ which readily identified authentic mature TGF-$\beta$1 (FIG. 8). Specificity was demonstrated by pre-absorbing the antibody with synthetic peptide immunogen prior to the immunoblot.

Under reducing conditions (FIG. 8A), authentic TGF-$\beta$1 migrates as a polypeptide of 12-13 kd in size. In the supernatants of MTX selected TGF-$\beta$1-3 cells, a protein comigrating with authentic TGF-$\beta$1 was readily identified (FIG. 8A). Supernatants collected from nonamplified TGF-$\beta$1-3 cells displayed nondetectable levels of recombinant $\beta$1-TGF using immunoblotting. The 20 µM MTX selected TGF-$\beta$1-3 cells appeared to produce the highest levels of mature TGF-$\beta$1, approaching 2-4 times higher than the 2 µM MTX selected cells. These increases are consistent with the results obtained for expression of mRNA levels (Table I) and bioactivity (Table IIA and IIB). Under nonreducing conditions (FIG. 8B), the recombinant TGF-$\beta$1 protein behaves as authentic TGF-$\beta$1, migrating as a dimer at 24 kd.

In addition to mature TGF-$\beta$1, larger forms of immunoreactive material were also observed. On reducing gels, these forms ranged in size from 44 kd to 56 kd (FIG. 8A). The broad nature of these immunoreactive forms may suggest extensive glycosylation. Interestingly, in the absence of reducing agent, these larger forms also appeared to migrate as a dimer 95 kd to 110 kd in size (FIG. 8B). The identification of these larger forms as precursor molecules was confirmed using the precursor-specific antibodies as described in the next subsection.

### 7.5.2. IDENTIFICATION OF PRECURSOR TGF-$\beta$1

Supernatants from the TGF-$\beta$1-3 transfectants were collected and tested by immunoblotting with the precursor- specific antibodies (FIG. 9). After reduction, antibodies directed toward two regions of the precursor sequences (anti-TGF-$\beta1_{81-94}$ and anti-TGF-$\beta1_{225-236}$) identified the 44 kd to 56 kd higher molecular weight forms and did not react with the mature TGF-$\beta$1 present in the supernatants (FIG. 9A). These precursor-specific antibodies in addition to identifying the larger 44 kd to 56 kd forms, also detected precursor polypeptides ranging in molecular weight from 30 kd to 42 kd (FIG. 9A). These smaller precursor molecules did not react with anti-TGF-$\beta1_{369-381}$ and may represent only precursor sequences. Thus, supernatants conditioned by TGF-$\beta$1-3 cells selected in MTX, contain, in addition to mature $\beta$1-TGF, larger precursor forms. These precursor sequences, since they are so highly conserved between species, may display other important biological properties. To illustrate all three TGF-$\beta$1 forms within the transfectant supernatants after reduction, an immunoblot probed with a mixture of precursor-specific antibody (anti-TGF-$\beta1_{225-236}$) and mature TGF-$\beta$1 specific antibody (anti-TGF-$\beta1_{369-381}$) is shown (FIG. 9A).

Supernatants fractionated on non-reducing SDS-polyacrylamide gels and probed with the precursor-specific peptide antibodies or with the mixture of precursor specific antibody and mature TGF-$\beta$1 specific antibody are shown in FIG 9B. A larger molecular weight form ranging in size from 95 kd to 110 Kd was readily identified by each of the antibodies. A mixture of precursor-specific (anti-TGF-$\beta1_{225-236}$) and mature TGF-$\beta$1 (anti-TGF-$\beta1_{369-381}$ antibodies detected the dimeric form of TGF-$\beta$1 (24Kd) in addition to the 95-110 Kd band.

### 7.6. RECOMBINANT TGF-$\beta$1 CONSTITUTES THE MAJORITY OF THE SECRETED PROTEINS FROM TGF-$\beta$1-3-2000 CELLS

TGF-$\beta$1-3/0 and TGF-$\beta$1-3/2000 cells were grown to confluency, labeled in serum-free medium with [$^{35}$S]-cysteine and [$^{35}$S]-methionine for 18 hours, and the radiolabeled secreted proteins were fractionated on reducing SDS-polyacrylamide gels. The results are shown in FIG. 10. Supernatants collected from

radiolabeled TGF-β1-3/0 cells showed no detectable levels of recombinant TGF-β1 proteins. In contrast, supernatants from the TGF-β1-3/2000 cells revealed four major secreted proteins not found in the initial TGF-β1-3/0 transfectant. Three of these proteins migrated identically to the mature and precursor forms of TGF-β1 identified by immunoblotting. The other protein which was heavily labeled with [$^{35}$S]-amino acids and released by the TGF-β1-3/2000 cells migrates at a molecular weight of 22 Kd. Amino-terminal sequence analysis of this protein revealed its identity as dihydrofolate reductase.

## 8. EXAMPLE: CHARACTERIZATION OF THE TGF-β1 GENE PRODUCT

The following examples present data on the purification and extensive characterization of the rTGF-β1 products synthesized by CHO-TGF-β-3-2000 cells. The results indicate that rTGF-β1 is synthesized in CHO cells as pre-pro-TGF-β1 which is processed at the carboxy-terminal side of Gly-29 and Arg 278, that rTGF-β1 precursor is glycosylated and phosphorylated but that the mature protein is not, that rTGF-β1 possesses a specific activity equivalent to that of natural TGF-β1, and that mature rTGF-β1 is a potent inhibitor of tumor cell growth in vitro and in vivo.

### 8.1. GLYCOSYLATION AND PHOSPHORYLATION OF THE rTGF-β1 PRECURSOR

The structural features of the rTGF-β1 precursor relevant to this section are illustrated in the line diagram shown in FIG. 11A. A hydrophobic leader cleaved from the protein at amino acid residue 29 produces a 361 amino acid polypeptide indicated as 'a' in FIG. 11A. Subsequent modification and cleavage would result in mature rTGF-β1 monomer (labeled 'c' in FIG. 11A) and a 249 amino acid protein consisting entirely of amino terminal precursor residues ('b' in FIG. 11A).

The cell line used in this example is TGFβ3-2000 and the TGF-β1 related proteins secreted by these cells, analyzed by immunoblotting, are shown in FIG. 11B. As decribed in Section 7.5., supra, supernatants derived from TGFβ3-2000 cells contain a large 95 Kd-110 Kd form of rTGF-β1 as well as the mature 24 Kd protein dimer when analyzed by SDS-polyacrylamide gels under non-reducing conditions; when analyzed under reducing conditions, these supernatants are found to contain a 44 Kd - 56Kd band ('a' in FIG. 11B, lane 2), a 30 Kd - 42 Kd band ('b' in FIG. 11B, lane 2) and a 12 Kd band ('c' in FIG. 11B, lane 2) which is the mature TGF-β1 monomer. Evidence that bands a, b, and c shown in FIG. 11B contain the regions of the rTGF-β1 precursor shown in FIG. 11A is presented in Section 7.5., supra. These bands can be easily visualized when [$^{35}$S]-methionine and [$^{35}$S]-cysteine labeled supernatants from TGF-β3-2000 cells are analyzed directly by SDS-polyacrylamide gel electrophoresis followed by fluorography (FIG. 11C, lane 1 and FIG. 11D, lane 1); these bands are not detected in supernatants from non-transfected CHO cells.

The diffuse nature of bands 'a' and 'b' shown in FIG. 11 suggested that they may be glycosylated. To investigate this possibility, TGFβ3-2000 cells were labeled with [$^{3}$H]-glucosamine and cell-free supernatants were analyzed by SDS-polyacrylamide gel electrophoresis followed by fluorography. FIG. 11C (lane 2) shows, that the 95-110 Kd form was labeled; no label was detected in the mature 24 Kd protein dimer. When analyzed under reducing conditions (FIG. 11D, lane 2), bands 'a' and 'b' were labeled. No label was found in band'c'. Thus, the rTGF-β1 precursor is glycosylated while the mature of 12 Kd monomer is not.

The nature of this glycosylation was further investigated by treating supernatants from TGFβ3-2000 cells with various glycolytic enzymes followed by fractionation of the digestion products on SDS-polyacrylamide gels. FIG. 12A shows an immunoblot analysis of these digests. Neuraminidase treatment caused bands 'a' and 'b' to migrate as faster but still diffuse bands (FIG. 12A, lane 3) indicating the presence of sialic acid residues. Endoglycosidase H, which predominantly cleaves high-mannose oligosaccharide chains, had no noticeable effect (FIG. 12A, lane 4). Digestion with N-glycanase, which removes N-linked carbohydrate, caused bands 'a' and 'b' to migrate as two sharp bands, the largest of which had a molecular weight of approximately 39 Kd (FIG. 12A, lane 2). As expected, no change in the migration of the mature 12 Kd monomer was noted. The same results were obtained using [$^{35}$S]-cysteine labeled supernatants from TGFβ3-2000 cells (FIG. 12B).

In order to determine the size of the unmodified TGF-β1 precursor, a 1350 base pair Pst I-Eco RI fragment containing the entire coding region of TGF-β1 (Sharples et al., 1987, DNA 6:239-244) was subcloned into pSP64 and transcribed with SP6 polymerase (Kreig et al., 1984, Nucleic Acids Res. 18:7057-70). Analysis of these transcripts on agarose-urea gels indicated that a single RNA species was produced (FIG. 13A) which programmed the synthesis of a 42 Kd polypeptide in a message-dependent reticulocyte cell-free translation system (FIG. 13B). Longer exposure of this gel revealed the presence of a minor 40 Kd product. The size (42 Kd) of the major cell-free translation production is in good agreement with that expected for a 390 amino acid protein and most likely corresponds to the unmodified TGF-β1 polypeptide backbone. The 39 Kd band shown in FIG. 12A (lane 2) and FIG. 12B (lane 4) would then represent the deglycosylated protein core of rTGF-β1 minus the hydrophobic leader sequence ('a' in FIG. 11A). The band below this corresponds to the deglycosylated band 'b' in FIG. 11A.

Incubation of TGFβ3-2000 cells in the presence of [$^{32}$P]-orthophosphate and subsequent fractionation of cell-free supernatants on SDS-polyacrylamide gels indicated that the rTGF-β1 precursor, but not the mature 12 Kd monomer, was phosphorylated (FIG. 11E). This layer electrophoresis of acid hydrolysates showed that most of the phosphate was not attached to serine, threonine or tyrosine (spots X, Y, Z, FIG. 11F). Data obtained by example Section 9, infra, demonstrates that phosphate is incorporated into asparagine-linked complex carbohydrate moieties as mannose-6-phosphate. The implications of this finding are also discussed.

## 8.2. PURIFICATION OF BIOLOGICALLY ACTIVE TGF-β1

CHO cell transfectants expressing rTGF-β1 (TGF-β3-2000 cells) were propagated and passaged as described in Section 7, supra. Roller bottles (850 cm$^2$) containing 50 ml of Dulbecco's modified Eagles' medium (DMEM) supplemented with fetal bovine serum (10% v/v), penicillin (100 U/ml), streptomycin (100 µg/ml), L-proline (15)0 µg/ml) and methotrexate (20 µM) were seeded with one confluent 150 cm$^2$ round tissue culture dish of TGF-β3-2000 cells and grown at 37° C. After cells attached and reached confluency, they were rinsed twice with 50 ml of serum-free medium supplemented as above and then incubated for 24h in 50 ml of serum-free medium supplemented additionally with ascorbate and reduced glutathione at 100 µg/ml and 20 µg/ml, respectively.

Serum-free supernatants were collected from roller bottles, centrifuged at 200 x g to remove cellular debris, and immediately adjusted to 10 mg/liter phenylmethylsulfonyl fluoride, 50 trypsin inhibitory units/liter of aprotinin (Sigma), and 0.2M acetic acid. Supernatants were concentrated 40-fold by ultrafiltration (YM10 membrane, 10,000 molecular weight cut-off; Amicon) and the resulting concentrate dialyzed extensively against 0.2M acetic acid. The dialyzed material was lyophilized and stored at -20° C prior to purification.

Conditioned medium was first fractionated by gel permeation chromatography. A representative elution profile is shown in FIG. 14. Greater than 95% of the biological activity eluted, based on marker proteins, at a molecular weight of approximately 15,000. The same elution pattern was observed with nTGF-β1 using the same column conditions. The low apparent molecular weight of TGF-β1, as determined by gel permeation chromatography, may be due to the tightly folded structure of the dimeric growth factor molecule or to nonspecific adsorption. High molecular weight activity was observed in void volume of the column and accounted for less than 5% of the total applied activity. SDS-PAGE under non-reducing conditions of pool A and B revealed that the majority of the biological activity eluted as a 24 KDal polypeptide species, whereas the minor activity eluted as a large 95-110 KDal molecular weight component (data not shown).

To confirm that the 24 KDal component represented the properly processed rTGF-β1, we purified this species to homogeneity using reversed-phase HPLC for subsequent characterization. Pool B was fractionated on a C18 µBondapak support (FIG. 15). The biologically active component eluted on a shallow acetonitrile gradient as a homogeneous peak with the same retention time as nTGF-β1. Analysis on SDS-polyacrylamide gels under non-reducing and reducing conditions demonstrated that this active component was homogeneous, comigrating with nTGF-β1 isolated from bovine spleen (FIG. 16). rTGF-β1 was further characterized by protein sequency analysis (Table V).

## TABLE V

AMINO-TERMINAL SEQUENCE ANALYSIS OF rTGF-β1 POLYPEPTIDES

| | Polypeptide[a] a | Polypeptide[a] b | | HPLC Purified Mature rTGF-β1[b] | | | |
|---|---|---|---|---|---|---|---|
| Cycle | Yield (pmol) | Yield (pmol) | Position (Residue) | Yield (pmol) | Position (Residue) | Yield (pmol) | Position (Residue) |
| 1 | 42.9 | 93.8 | 30 (Leu) | 40.1 | 279 (Ala) | 27.4 | 383 (Ile) |
| 2 | 17.0 | 29.1 | 31 (Ser) | 36.1 | 380 (Leu) | 27.3 | 384 (Val) |
| 3 | 13.2 | 27.0 | 32 (Thr) | 26.8 | 281 (Asp) | 13.5 | 385 (Arg) |
| 4 | N.D. | N.D. | 33 (Cys)[c] | 10.9 | 282 (Thr) | 7.3 | 386 (Ser) |
| 5 | 34.9 | 49.4 | 34 (Lys) | 15.2 | 283 (Asn) | N.D. | 387 (Cys)[c] |
| 6 | 12.5 | 20.0 | 35 (Thr) | 14.8 | 284 (Tyr) | 8.1 | 388 (Lys) |
| 7 | 21.1 | 32.5 | 36 (Ile) | N.D. | 285 (Cys)[c] | N.D. | 389 (Cys)[c] |
| 8 | 17.9 | 38.8 | 37 (Asp) | 14.6 | 286 (Phe) | 4.7 | 390 (Ser) |

[a] Polypeptides a and b were electroeluted from a coomassie blue stained gel similar to that shown in FIG. 1b prior to amino-terminal sequence analysis.

[b] Sequence of purified mature rTGF-β1. The rTGF-β1 was cleaved with CNBr and results indicate simultaneous sequences of amino- and carboxy-ends of the growth factor.

[c] N.D.: Not determined.

The purified polypeptide was chemically cleaved with cyanogen bromide prior to sequencing. Since mature TGF-β1 contains only one methionine at residue 382, two sequences were obtained simultaneously; one corresponding to the amino-terminal sequence of the growth factor (beginning at Ala-279) and one representing the carboxyterminal 8 amino acids (beginning at ILe-383). Our results (Table V) demonstrate that biologically active rTGF-β1 is properly processed.

A summary of the purification steps of rTGF-β1 is shown in Table VI. The growth factor was purified greater than 30-fold in two purification steps. In this particular experiment, the overall yield was 54% and resulted in 0.65 mg of rTGF-β1 per liter of conditioned culture medium. Processing of other preparations resulted in greater than 85% recovery of biologically active recombinant protein, a yield of more than 1 mg per liter.

TABLE VI

PURIFICATION OF MATURE rTGF-β1 FROM CONDITIONED MEDIUM

| Fraction | Protein[b] (mg) | Units[a] | Specific Activity Units/mg | Percent Yield |
|---|---|---|---|---|
| Conditioned Medium[c] | 39.0 | $14 \times 10^7$ | $3.6 \times 10^6$ | 100 |
| TSK (Fraction B) | 1.37 | $8.3 \times 10^7$ | $61 \times 10^6$ | 59 |
| HPLC-C18 | 0.65[d] | $7.5 \times 10^7$ | $119 \times 10^6$ | 54 |

[a] One unit of activity is defined in Experimental Procedures.

[b] Protein determined assuming 1 absorbance unit at 280 nm = 1 mg/ml protein.

[c] Started with one liter of conditioned medium.

[d] Calculated from amino acid analysis.

## 8.3 PURIFICATION AND NATUURE OF rTGF-β1 PRECURSOR

The rTGF-β1 precursor was purified by taking advantage of its glycosylated nature. Precursor eluting in the void volume of a TSK-250 colum (Pool A) was dialyzed against neutral buffer (50mM $NH_4HCO_3$, pH 7.8), and centrifuged at 15,000 x g prior to fractionation on a conconavalin A lectin column. A column containing one ml of conconavalin A covalently bound to Sepharose 4B (Pharmacia) was extensively washed with phosphate buffered saline (PBS) and equilibrated with 50 mM $NH_4HCO_3$, pH 7.0. Samples to be absorbed were loaded and recirculated four times through the column before washing with ten volumes of PBS. Specifically bound material was eluted with 100mM methyl-α-D-mannopyranoside in PBS.

Conconvalin A-bound pecursor was eluted specifically with α-methyl mannoside. An SDS-polyacrylamide gel profile of the purified precursor stained with Coomassie blue is shown in FIG. 16. The eluted protein migrated at a molecular weight of between 95-120 KDal. This large form was reactive with antibodies directed toward the precursor sequences and mature growth factor. No contaminating mature rTGF-β1 was detected in this preparation, even on overloaded SDS-polyacrylamide gels.

Although the mature, dimeric growth factor was absent from the preparation, the purified precursor when analyzed by reducing SDS-PAGE revealed a 14 KDal species which comigrated with monomeric rTGF-β1 (FIG. 16). Also apparent on the gels where the two precursor species, pro-TGF-β1 (30-390) and

the 30-42 KDal pro region of the precursor (30-278; see also FIG. 17). Further attempts to fractionate this larger complex into separate components were unsuccessful. Amino terminal sequence analysis of the conconavalin A purified material revealed two amino terminal sequences, one beginning at Leu-30 and the other at Ala-279. These results strongly suggest that the 95-120 KDal rTGF-β1 precursor purified from the conditioned medium of the CHO cells represents a mixture of pro-TGF-β1 (30-390), the pro region of the precursor (30-278), and the mature chain of rTGF-β1 (279-390) all interlinked by disulfide-bonds. To confirm this observation, we digested the precursor with CNBr and purified the CNBr peptides to establish the chemical nature of this complex. TGF-β1-precursor (800 pmol) was dissolved in 30 µl of 70% formic acid and 16 µl of a solution containing 15mg CNBr in 100 11 70% formic acid was added (Gross and Witkop, 1962, J. Biol. Chem. 237:1856-1860). The reaction proceeded under nitrogen for 4 hours at 30°C in the dark. The digest was chromatographed on a Bio-Sil TSK-250 gel permeation chromatography column equilibrated in 0.1% trifluoracetic acid (TFA) containing 40% acetonitrile as described (Ikeda et al., 1987, Biochemistry 26:2406-2410). Reversed-phase HPLC was performed on a µ Bondpak C-18 column (3.9 x 300 mm, 10 µm particle size; Waters) with a linear gradient composed of 0.05% TFA in water as starting buffer and 0.045% TFA acetonitrile as limiting buffer. Fractions were collected in polypropylene tubes to minimize losses from non-specific adsorption. A TSK-250 elution profile of CNBr-cleaved rTGF-β1 precursor is shown in FIG. 18. The various peaks were identified by amino acid sequencing. A major CNBr peptide fragment containing a disulfide bridge between a precursor cysteine residue and the Cysteine residue of the mature growth factor is M(30-38/279-382/383-390) and its amino-terminal sequence analysis is shown in Table VII. This particular peptide fragment involves Cys-33 of the precursor and one of the cysteine residues of the mature growth factor. The amino-terminal CNBr peptide M(30-38/262-382/383-390) represents a disulfide-linked peptide involving pro-TGF-β1.

TABLE VII

AMINO-TERMINAL SEQUENCE OF DISULFIDE CROSS-LINKED FRAGMENT CB PEPTIDE I DERIVED FROM rTGF-β1 PRECURSOR

| Cycle | Yield (pmol) | Position (Residue) | Yield (pmol) | Position (Residue) | Yield (pmol) | Position (Residue) |
|---|---|---|---|---|---|---|
| 1 | 22.6 | 30 (Leu) | 25.5 | 279 (Ala) | 16.0 | 383 (Ile) |
| 2 | 10.6 | 31 (Ser) | 28.3 | 280 (Leu) | 18.5 | 384 (Val) |
| 3 | 11.3 | 32 (Thr) | 21.2 | 281 (Asp) | 8.5 | 385 (Arg) |
| 4 | N.D. | 33 (Cys)[a] | 14.9 | 282 (Thr) | 9.3 | 386 (Ser) |
| 5 | 14.8 | 34 (Lys) | 18.9 | 282 (Asn) | N.D. | 387 (Cys)[a] |
| 6 | 10.9 | 35 (Thr) | 21.3 | 284 (Tyr) | 14.7 | 388 (Lys) |
| 7 | 13.3 | 36 (Ile) | N.D. | 285 (Cys)[a] | N.D. | 389 (Cys)[a] |
| 8 | 12.3 | 37 (Asp) | 22.1 | 286 (Phe) | 5.6 | 390 (Ser) |

[a] N.D.: Not determined.

## 8.4. AMINO TERMINAL SEQUENCE OF rTGF-β1 POLYPEPTIDES

Automated sequence analysis was performed on a model 475A amino acid sequencer (Applied Biosystems). Phyenylthiohydantoin-amino acid derivatives were separated by reversed phase HPLC, on-line, on a model 120A PTH analyzer (Applied Biosystems) as described (Marquardt et al., 1987, J. Biol. Chem. 262:12127-12136).

Serum-free conditioned media from CHO-TGF-β1-3/2000 cells expressing high levels of simian rTGF-β1 was electrophoresed on SDS-PAGE under reducing conditions. Coomassie blue staining reveals the three molecular forms of rTGF-β1 secreted by these cells (FIG. 17). The largest form, a broadly migrating species ranging in size from 44-56 KDal ("a" in FIG. 17) and possessing immunological epitopes derived from TGF-β1 precursor and mautre TGF-β1 (Section 7.5, supra), most likely represents unprocessed TGF-β1 precursor. The 30-42 KDal polypeptide ("b" in FIG. 17) only contains precursor-derived epitopes (Section 7.5, supra) indicating that this species has undergone proteolytic cleavage separating it and the mature TGF-β1 form. The 14 KDal species ("c" in FIG. 17) represents the mature, fully processed TGF-β1 monomer.

Recombinant precursor proteins ("a" and "b" in FIG. 17) were electroeluted from acrylamide slices and characterized by amino-terminal sequence analysis. The results are shown in Table V supra. Sequence analysis revealed that the two larger precursor forms have identical amino-terminal sequences. Comparison of this sequence with that predicted from the simian TGF-β1 cDNA (Sharples, et al., 1987, DNA 6:239-244) indicates that both larger proteins have undergone specific proteolytic cleavage at Gly-29/Leu-30 removing the first 29-amino acids of the intact pre-pro-TGF-β1 molecule. Cleavage of this hydrophobic 29-amino acid leader sequence is most likely the result of a signal peptidase. The Gly-29/Leu-30 peptide bond is the predicted signal peptide cleavage site (Von Heijne, 1986, Nucleic Acids Res. 14:4683-4690). Based on these results, the 44-56 KDal TGF-β1 polypeptide ("a" in FIG. 17) represents pro-TGF-β1 (30-390) whereas the 30-42 KDal species ("b" in FIG.17) corresponds to the pro region of the precursor (30-278) lacking the signal peptide and mature TGF-β1 sequences. Sequence analysis of the 14 KDal polypeptide (data not shown) revealed an intact amino-terminus beginning at Ala-279 of the mature growth factor indicating that CHO-TGF-β1-3/2000 cells properly process the simian rTGF-β1 at the dibasic cleavage site. A summary of a proposed processing scheme for TGF-β1 is presented in FIG. 20.

## 8.5. BIOLOGICAL ACTIVITY IN VITRO

Purified mature and precursor forms of rTGF-β1 were tested for biological activity on mink lung epithelial cells as described in Section 7.1.6., supra. The biological activity profiles of mature and precursor rTGF-β1 and natural TGF-β1 from bovine spleen are presented in FIG. 19. Molarity calculations were based on predicted amino acid compositions (for precursor rTGF-β1, the amino acid compositions of pro-TGF-β1 was used). The results indicate that mature rTGF-β1 is a potent inhibitor of mink lung cell proliferation (1-2 pM for half-maximal inhibition) and has an activity curve which is superimposable with that obtained for natural TGF-β1. In other words, the recombinant TGF-β1 of the invention and natural TGF-β1 possess identical specific activities. In contrast, the precursor preparation was 50-fold less active than the mature growth factor (50-60 pM for half-maximal inhibition) and a comparison of the inhibition profiles in FIG. 19 revealed a slight difference in the dose-response curves, suggesting receptor affinity differences between the mature and precursor forms.

## 8.6. BIOLOGICAL ACTIVITY IN VIVO

The effects of TGF-β1 in vivo are largely unknown although its role in inhibiting mammary gland growth (Silberstein and Daniel, 1987, Science 237:291), in wound healing (Sporn et al., 1983, Science 219:1329), and in embryonic development (Weeks et al., 1987, Cell 51:861) have been suggested. Both naturally derived TGF-β1 and TGF-β2 from bone (Seyedin et al, 1986, J. Biol. Chem. 261:5693; Seyedin et al., 1987, J. Biol, Chem. 262:1946) and recombinant Simian TGF-$\beta$1 cloned (Sharples et al., 1987, DNA 6:239-244) and expressed (Gentry et al., 1987, Mol. Cell. Biol. 7:3418) in CHO-TGF-$\beta$1-3-2000 cells are potent inhibitors of DNA synthesis in a variety of established tumor cell lines of human epithelial origin (Ranchalis et al., 1987, Biochem. Biophys. Res. Comm. 148:783). In this study, we present in vivo evidence that TGF-$\beta$ is also tumorstatic for a human lung tumor grown in athymic nude mice. Particularly striking is the induction by TGF-$\beta$ of a differentiated-like cellular phenotype in the inhibited tumor cells. The human lung carcinoma cell line designated A549, established from a male Caucasian with an adenocarcinoma of the lung, is a responsive target (in vitro) for inhibition by picomolar concentrations of natural TGF-$\beta$1 or TGF-$\beta$2 and the recombinant TGF-$\beta$1 of the invention. Nude mice were subcutaneously inoculated with A549 cells; palpable tumors developed in approximately 80% of the animals in three weeks. FIG. 21 shows the effect of TGF-$\beta$1 and TGF-$\beta$2 treatment on the further growth of A549 tumors in these mice. Each experimental group contained five animals and values on the ordinate represents the average tumor measurements in three dimensions (volume). Day 1 corresponds to the first day the groups received treatment; test compounds were subcutaneously injected in the vicinity of the tumor but not into the tumor itself. Animals in control groups received injections of bovine serum albumin in a carrier solution identical to TGF-$\beta$ treated tumor-bearing animal groups. Tumor volume in control groups doubled approximately every 7-8 days. Similar doubling times were also observed in separate experiments in tumor-bearing animals which received a biologically inactive synthetic peptide. In contrast, successive injections, corresponding to those days as indicated on the abscissa, of 200 ng of TGF-$\beta$1 and TGF-$\beta$2 (1.4 $\mu$g total per treatment regimen for each) were tumorstatic and retarded the further growth of tumors. As shown in FIG. 21, TGF-$\beta$1 appeared slightly more effective than TGF-$\beta$2. In a separate experiment, the dose response of TGF-$\beta$1 on A549 tumor inhibition was examined (FIG. 21). Values represent average tumor volumes in TGF-$\beta$ treated animals relative to tumor volumes from mock-treated animals. About 25% inhibition was observed at the lowest dose tested (12.5 ng per injection) of TGF-$\beta$1. At higher doses, 50 and 200 ng per injection, a correspondingly greater inhibition in tumor growth was observed (37% and 60% respectively). In some experiments, the larger the tumor volume at day 1, the lower the percent reduction in inhibited tumor volume relative to tumors derived from control groups. Animals receiving even the highest doses of TGF-$\beta$ (1.4 $\mu$g total after 20 days) exhibited no gross manifestations of TGF-$\beta$ toxicity. As shown in Fig. 23, TGF-$\beta$1 treated animals displayed normal characteristics; no apparent abnormalities were found on gross examination of major organs during biopsy. The inset is representative of the size of tumors removed from each group at the end of the experiment (day 20) prior to submission for pathology. Tumors removed from experimental animals (day 21, FIG. 21) had a greater than 90% reduction in average net weights.

Recombinant Simian TGF-$\beta$1 purified to homogeneity from serum-free culture supernatants exhibits an in vitro dose response inhibition curve similar to the natural (bovine bone) molecule when tested on a variety of tumor cells including the A549 tumor cells used in these studies. A comparison of the effect of recombinant and natural TGF-$\beta$1 on the growth of A549 human lung carcinoma in athymic mice is shown in Table VIII. The recombinant product was more effective in inhibiting tumor growth than bone-derived TGF-$\beta$1, 60% inhibition compared to 46% inhiibtion.

TABLE VIII

COMPARISON OF THE EFFECTS OF RECOMBINANT AND NATURAL TGF-β1 ON TUMOR GROWTH

| TGF-β1 | Tumor size* Control | Tumor size* Treated | % Inhibition |
|---|---|---|---|
| Recombinant | 105.4 | 42.6 | 60.0 |
| Bone-derived | 83.4 | 45.4 | 46.0 |

* Protocol for these experiments is as described in legend to FIG. 21 except that tumors were measured only in two dimensions. Values represent average tumor area in animals from each group. Purified rTGF-β1, and natural TGF-β1 from bovine bone (Seyedin et al., 1986, J. Biol. Chem. 261:5693) were injected peritumorally (200 ng/injection, 1 μg total). Measurements represent average tumor size at day 17 post treatment.

Tumors were excised from control and treated animal groups, fixed and submitted for histological pathological examination. As shown in FIG. 24, panel A, under low power, trichrome stained control tumor sections demonstrate predominantly large areas of necrosis dispersed through a rather heterogeneous field of different cell types, mostly columnar epithelial. A large number of blood vessels are also observed. In contrast, similarly prepared sections of TGF-β1 inhibited tumor specimens "b" in FIG. 24 demonstrated little necrosis, more apparent organization, and a different distribution of cell types. Particularly apparent was the increase in connective tissue banding (blue staining) relative to control tumor specimens. In addition, general vascularity appeared diminished relative to control tumors. When examined under higher magnification, non necrotic areas of control tumors ("c" in FIG. 24) exhibited a combination of epithelial-like and poorly differentiated cell types, also apparent is the high density of stained nuclei indicating a high rate of tumor cell proliferation. In contrast, TGF-β-treated tumor specimens ("d" in FIG. 25) presented a significantly different picture. The predominant cell type seen is large and round; nuclei are more dispersed in the field and display the crescent morphology characteristic of goblet cells (a normal lung cell type that secretes mucins). Although some mucous secreting cells were observed in control tumor specimens they represented only aminor subpopulation of cells. Also more prevalent in sections of TGF-β inhibited tumors ("d", inset, in FIG. 25) were scattered foci of columnar epithelial cells organized around blood vessels.

The mucous secreting nature of the goblet-like cell type from treated tumors was confirmed by periodic acid Schniff base stain (PAS). Control tumor sections ("a" in FIG. 25) revealed a few scattered areas of high glycoprotein density. Dramatically amplified PAS staining intensity was seen throughout TGF-β-treated tumor sections ("b" in FIG. 25). A more differentiated and organized phenotype is also evident. Both control and TGF-β-treated tumor sections were also examined for glycosaminóglycans (GAGs) as measured by staining with Alcian blue at pH 2.5. Control specimens ("c" in FIG. 25) exhibited very light staining whereas TGF-β-treated specimens ("d" in FIG. 25) showed significantly more, suggesting a higher level of hyaluronic acid synthesis and deposition.

## 9. EXAMPLE: IDENTIFICATION OF MANNOSE-6-PHOSPHATE IN TWO ASPARAGINE-LINKED SUGAR CHAINS OF rTGF-β1 PRECURSOR

The following example demonstrates that all three potential glycosylation sites (Asn-82, -136 and -176) in simian rTGF-β1 are used for carbohydrate addition and that phosphorylation occurs within the oligosaccharide side chains. The results suggest an independent functional role of the rTGF-β1 precursor.

### 9.1. MATERIALS AND METHODS

#### 9.1.1. MATERIALS

Sequencer reagents were obtained from Applied Biosystems. Solvents for HPLC were from Burdick and Jackson. CNBr was from Kodak; 4-vinylpyridine was from Aldrich Chemical Co.; all other chemicals were reagent grade. Staphylococcus aureus V8 protease was from Miles Laboratories; L-(tosylamido-2-phenyl) ethyl chloromethyl ketone-treated trypsin was obtained from Worthington.

#### 9.1.2. CELL CULTURE

The TGF-β-3-2000 cell line was propagated as described in Section 7.1.1., supra. Individual clones were isolated by limiting dilution in 96 well plates. One clone, TGF-β-3-2000-17 (hereafter referred to as clone 17) was found to produce approximately 2 µg/ml of active TGF-β1 and was used for further analysis. These cells were routinely passaged in Dulbecco's modified Eagle's medium containing 10% fetal bovine serum, 150 µg/ml L-proline, 100 U/ml penicillin, 100 µg/ml streptomycin, and 20 µM methotrexate.

#### 9.1.3. RADIOLABELING

Clone 17 cells were grown to confluence, washed three times in phosphate-free medium minus serum and incubated in the same medium for 30 minutes at 37° C. The medium was then replaced with fresh methionine, cysteine and serum-free medium containing 200 µCi/ml [$^{35}$S]-methionine and [$^{35}$S]-cysteine (NEN, Boston, MA) and cells were incubated for 4 hours at 37° C. For labeling with [$^{3}$H]-sugars, cells were grown to confluence, washed with serum-free medium and labeled for 20 hours in serum-free medium containing 100 µCi/ml of [$^{3}$H]-glucosamine or [$^{3}$H]-mannose (NEN, Boston, MA). Radiolabeled serum-free supernatants were collected, centrifuged at 4000 X g for 10 minutes, dialyzed extensively against 0.2 M acetic acid and lyophylized.

#### 9.1.4. POLYACRYLAMIDE GEL ELECTROPHORESIS

Dried pellets were analyzed under reducing conditions on a 15% SDS-polyacrylamide or on 7.5-15% gradient SDS-polyacrylamide gels as described (Laemmli, 1970, Nature 227:680-685). Gels were stained with Coomassie blue, fluorographed (for [$^{35}$S]- and [$^{3}$H]-labeled proteins (Chamberlain, 1979, Anal. Biochem. 98:132-136) and exposed to Cronex-4 X-ray film. Alternatively, lyophylized samples were digested with N-glycanase (Genzyme, Boston, MA) for 16 hours at 37° C, using conditions recommended by the manfacturer, prior to polyacrylamide gel electrophoresis.

### 9.1.5. ACID HYDROLYSIS

Acid hydrolysis - [$^{32}$P]-labeled rTGF-β1 precursor, and [$^{32}$P]-labeled glycopeptides were hydrolyzed in 6N HCl for 2 hours at 95°C. The products were separated by electorphoresis at pH 1.9 and 3.5 and detected by autoradiography as described (Cooper et al., 1983, Meth. Enzymol. 99:387-402). Alternatively, electrophoresis at pH 8.9 (1% ammonium carbonate) was followed by chromatography (65% isobutyric acid, 5% pyridine, 3% acetic acid, 2% butanol, 25% water). Internal standards (phosphoserine, phosphothreonine, phosphotyrosine) were detected by ninhydrin staining. Mannose-6-phosphate was detected by spraying with 70% perchloric acid: 1M HCl: 4% ammonium molybdate: acetone (5:10:25:60), drying, and exposing to ultraviolet light.

### 9.1.6. S-PYRIDYLETHYLATION

For reduction, 50 μg of TGF-β1 precursor and 225,000 cpm of [$^{32}$P]-labeled rTGF-β1 precursor, derived from serum-free supernatant of clone 17 cells was treated with dithiothreitol (20 mM) in 100 μl of 0.4 M Tris-HCl buffer, pH 8.5, containing 6 M guanidine HCl, 0.1% $Na_2$EDTA, for 2 hours at 50°C and subsequently S-pyridylethylated with 4-vinylpyridine (100 mM) for 4 hours at 22°C. The reaction mixture was acidified to pH 2.0 with 20% TFA and desalted on an RP-300 column (2.1 X 30 mm; Applied Biosystems) using a TFA/acetonitrile gradient for elution. The concentration of acetonitrile was increased linearly from 0.1% TFA in water to 60% acetonitrile containing 0.08% TFA ovr 1.5 hours, at a flow rate of 100 μl/min at 35°C.

### 9.1.7. CHEMICAL AND ENZYMATIC CLEAVAGE

For CNBr cleavage at methionyl residues, 650 pmol of S-pyridylethylated TGF-β1 precursor and 160,000 cpm of [$^{32}$P]-labeled S-pyrindylethylated TGF-β1 precursor were dissolved in 30 μl of 70% formic acid and 4 l of a solution containing 60 mg CNBr in 100 μl of 70% formic acid was added (Gross and Witkop, 1962, J. Biol, Chem. 237:1856-1860). The reaction preceeded under a nitrogen cushion for 4 hours at 30°C and continued for an additional 18 hours at 22°C in the dark.

Cleavage with S. aureaus V8 protease was done in 40 μ of 0.1 M Tris-acetic acid buffer, pH 8.0, containing 3 M urea at 37°C for 10 hours. The enzyme/substrate ratio was 1 to 10 (wt/wt). Trypsin digestion of pool A and pool B (FIG. 31-A) was done in 40 μl of 0.1 M Tris-acetic acid buffer, pH 8.0, containing 30% acetonitrile, at an enzyme substrate ratio of 1 to 20 at 37°C. for 15 hours. The enzymatic digests wre acidified with 20% TFA to pH 2.0 and separated by rpHPLC.

### 9.1.8. PEPTIDE PURIFICATION

HPLC was performed on a Waters HPLC system consisting of two M 6000 A pumps, a system controller, a U6K injector, a model 441 fixed wavelength detector (214 nm), or on a model 130A separation system (Applied Biosystems), and a chart recorder. Gel permeation chromatography on a Bio-Sil TSK-250 column (7.5 x 600 mm; Bio-Rad Laboratories) was carried out in 0.1% TFA containing 40% acetonitrile at a flow rate of 0.25 ml/min. Peptide purification by rpHPLC was carried out at 35°C on a RP-300 column (2.1 x 30 mm; Applied Biosystems). Linear acetonitrile gradients composed of 0.1% TFA in water as starting buffer and acetonitrile containing 0.08% TFA as limiting buffer were employed for elution. Peptides were collected manually. V8 protease peptides indicated by E, and trypsin peptides indicated by T could be used for sequence analysis without further purification.

## 9.1.9. AMINO ACID SEQUENCE ANALYSIS

Automated sequence analysis was performed on a model 475A amino acid sequencer (Applied biosystems) using the RUN470-1 program. A total of 1.5 mg BioBrene Plus (Applied Biosystems) was applied and subjected to two precycles of Edman Degradation prior to sample application. Conversion of the thiazolinone derivatives to phenylthiohydantoin amino acids was carried out with 25% TFA. Phenylthiohydantoin amino acid derivatives were separated by rpHPLC, on-line, on a model 120A PTH analyzer (Applied Biosystems), as described (Marquardt et al., 1987, J. Biol. Chem. 262:12127-12131).

## 9.2. RESULTS

The three structural forms of rTGF-β1 secreted by clone 17 cells are illustrated by the line diagram in FIG. 26A. Also indicated in FIG. 26A are the three potential asparagine-linked glyclosylation sites predicted from the DNA sequence of simian TGF-β1 precursor: Asn-82, -136 and -176 (FIG. 1 and Sharples et al., 1987, DNA 6:239-244).

FIG. 26B shows an autoradiogram of [$^{35}$S]-labeled proteins secreted by clone 17 cells analyzed by SDS-polyacrylamide gel electrophoresis. Proteins a, b and c can be easily visualized. Precursor proteins a and b can be labeled with [$^{3}$H]-glucosamine, [$^{3}$H]-mannose, and [$^{32}$P]-phosphate (FIG. 26C, 26D, and 26E) indicating the rTGF-β1 precursor proteins a and b, but not mature rTGF-β1 (protein c), are both phosphorylated and glycosylated. Digestion of [$^{35}$S]-labeled precursor proteins with N-glycanase resulted in a shift in migration of bands a and b to sharper and faster migrating bands, the largest of which had a molecular weight of approximately 39,000 (FIG. 27A, lane 2), consistent with the calculated molecular weight of 41,200 for the TGF-β1 precursor protein a. Digestion of [.$^{32}$P]-labeled proteins with N-glycanase and subsequent fractionation of the digest by SDS-polyacrylamide gel electrophoresis indicated that the enzyme has removed all the label from the rTGF-β1 precursor proteins (FIG. 27B, lane 2), suggesting that [$^{32}$P]-label was incorporated into asparagine-linked oligosaccharides.

The glycosylated and phosphorylated rTGF-β1 precursor proteins a and b were subjected to cyanogen bromide cleavage and subsequent enzymatic digestion for further characteization of the phosphorylation sites. The labeled glycopeptides were purified by gel permeation chromatography and rpHPLC. Sequence analysis of the three fragments listed in FIG. 28 indicated that Asn-82, Asn-136, and Asn-176 are glycosylated. Over 95% of the label was found in peptides E(77-91) and E(134-139). Peptide T(174-180) contained less than 5% of the total incorporated [$^{32}$P]-label.

S-pyridylethylated rTGF-β1 precursor was cleaved with CNBr at methionine residues. Gel permeation chromatography of the CNBr fragments on a Bio-Sil TSK-250 column resolved [$^{32}$P]-M(134-253), and [$^{32}$P]-M(39-113). A representative chromatogram is shown in FIG. 29.

M(39-113) was further subfragmented with S. aureus V8 protease. The enzyme digest was acidified and the peptides separated by rpHPLC. A representative chromatogram is shown in FIG. 30. The complete sequences of [$^{32}$P]-E(76-91) and [$^{32}$P]-E(76-94) were determined. Both peptides contain a carboxyl-terminal glutamic acid, consistent with the specificity of the protease. Both peptides contained on unidentified residue at position 82 (Table IX). The DNA sequence predicts an asparagine residue at position 82 of the TGF-β1 precursor translation product, a potential site for N-linked glycosylation. The expected yield of PTH-Asn at cycle 7 would have allowed identification of an unmodified asparagine. The yield of PTH-Asn at position 82 was approximately 0.5% of that expected based on the yields of the adjacent residues. The lower yield of PTH-Asn-82 may be due to the decreased solubility of the modified thiazolinone derivative in the extracting solvent, butyl chloride, relative to other thiazolinone amino acids. Acid hydrolysis and subsequent 2-dimensional electrophoresis of [$^{32}$P]-E(76-91) detected mannose-6-[$^{32}$P]-phosphate FIG. 31.

M(134-253) was further subfragmented with S. aureus V8 protease, yielding two major [$^{32}$P]-labeled peptides, as shown in FIG 31A. The sequences of the listed peptides were determined, both containing a carboxyl-terminal glutamic acid, consistent with the specificity of the protease. Peptide [$^{32}$P]-E(134-139) contained one unidentified residue at position 136 (Table IX). The yield of the predicted PTH-Asn was approximately 2% of that expected, based on the yields of the adjacent residues, and was thus assumed to be glycosylated Asn. Acid hydrolysis and subsequent 2-dimensional electrophoresis of [$^{32}$P]-E(134-139) detected mannose-6-[$^{32}$P]-phosphate (FIG. 32).

Peak A (FIG. 31A), containing E(170-194), was pooled, dried, and subfragmented with trypsin. The digest was acidified and the peptides separated by rpHPLC. A representative chromatogram is shown in

FIG. 31B. The sequence of T(174-180) was determined (Table IX). Peptide T(174-180) contained one unidentifed residue at position 176. The yield of the predicted PTH-Asn was approximately 1% of the expected, based on the yield of the adjacent residue Asn-177 suggesting glycosylation of Asn-176. The chromatographic heterogeneity of T(174-180) is evident. This peptide contained less than 5% of the total [$^{32}$P]-incorporated into the precursor protein. Acid hydrolysis and subsequent 2-dimensional electrophoresis detected mannose-6-[$^{32}$P]-phosphate (not shown).

Peak B (FIG. 31A) was dried and redigested with S. aureus V8 protease and subsequently with trypsin and the peptides were separated by rpHPLC. Similar peptide patterns were obtained as shown in FIG. 31A AND FIG. 31B, suggesting incomplete cleavage of M(134-253) with V8 protease.

Thin layer electrophoretic analysis of acid hydrolysates of total precursor proteins (a and b) as well as purified glycopeptides showed that [$^{32}$P]-phosphate was incorporated into mannose-6-phosphate; no [$^{32}$P]-phosphate was incorporated into Ser, Thr, Tyr (FIG. 32A-C). Comigration of peptide-incorporated [$^{32}$P]-label and standards of mannose-6-phosphate was observed upon electrophoresis in buffers at pH 1.9, pH 3.5 and pH 8.9, and in two different chromatography buffers (FIG. 32D and data not shown). Acid hydrolysis may also generate mannose-6-phosphate from proteins modified with glycosylphosphatidylinositol (Lon and Saltiel, 1988, Science 239:268-295), but this has only been found at the carboxyterminus of proteins and is therefore unlikely to account for mannose-6-phosphate in the rTGF-β1 precursor.

**TABLE IX**

**AMINO ACID SEQUENCE DATA FOR GLYCOPEPTIDES FROM S-PYRIDYLETHYLATED rTGF-β1 PRECURSOR**

| Position | Residue | Peptide (cycle) yield (pmol) | Position | Residue | Peptide (cycle) yield(pmol) |
|---|---|---|---|---|---|
| | | E(76-91) | | | E(134-139) |
| 76 | Ala | (1) 87.0 | 134 | Phe | (1) 99.4 |
| 77 | Val | (2) 91.3 | 135 | Phe | (2) 98.3 |
| 78 | Leu | (3) 111.2 | 136 | Asn | (3) 2.1 |
| 79 | Ala | (4) 100.5 | 137 | Thr | (4) 55.5 |
| 80 | Leu | (5) 75.7 | 138 | Ser | (5) 28.1 |
| 81 | Tyr | (6) 71.4 | 139 | Glu | (6) 52.8 |
| 82 | Asn | (7) 0.2 | | | |
| 83 | Ser | (8) 28.5 | | | T(174-180) |
| 84 | Thr | (9) 42.5 | | | |
| 85 | Arg | (10) 43.6 | 174 | Tyr | (1) 10.0 |
| 86 | Asp | (11) 45.8 | 175 | Ser | (2) 3.8 |
| 87 | Arg | (12) 47.6 | 176 | Asn | (3) 0.1 |
| 88 | Val | (13) 39.0 | 177 | Asn | (4) 8.5 |
| 89 | Ala | (14) 37.9 | 178 | Ser | (5) 3.3 |
| 90 | Gly | (15) 23.3 | 179 | Trp | (6) 5.1 |
| 91 | Glu | (16) 10.7 | 180 | Arg | (7) 1.7 |

## 10. DEPOSIT OF MICROORGANISMS

The following transformants have been deposited with the American Type Culture Collection, Rockville, Md, and have been assigned the listed accession numbers:

| Transfectant No. | Plasmid | Accession |
|---|---|---|
| CHO-TGF-β-3/2000 | pSV2-β-TGF | CRL 9434. |

The present invention is not to be limited in scope by the cell line deposited since the deposited embodiment is intended as a single illustration of one aspect of the invention and any which are functionally equivalent are within the scope of this invention. Indeed, various modifications of the invention in addition to those shown and described herein will become apparent to those skilled in the art from the foregoing description and accompanying drawings. Such modification are intended to fall within the scope of the appended claims.

It is also to be understood that all base pair and amino acid residue numbers and sizes given for nucleotides and peptides are approximate and are used for purposes of description.

## Claims

1. A method for producing transforming growth factor-β1, comprising:

(a) culturing a eucaryotic cell containing a nucleotide sequence encoding simian transforming growth factor-β1 under the control of a second nucleotide sequence that regulates gene expression so that a peptide or protein having transforming growth factor-β1 activity is produced by the eucaryotic cell; and

(b) recovering the transforming growth factor-β1 from the culture.

2. The method according to claim 1 in which the nucleotide sequence encoding the simian transforming growth factor-β1 comprises the nucleotide sequence substantially as depicted in FIG. 1 from nucleotide number 1 to 1173.

3. The method according to claim 1 in which the eucaryotic cell comprises a Chinese Hamster Ovary cell.

4. The method according to claim 1 in which the second nucleotide sequence which controls gene expression comprises an SV40 promoter.

5. The method accoding to claim 1 in which the second nucleotide sequence comprises a promoter and a sequence encoding a selectable marker for which the eucaryotic cell is deficient, so that the eucaryotic cell containing the simian transforming growth factor-β1 coding sequence can be identified.

6. The method according to claim 5 in which the selectable marker comprises dihydrofolate reductase.

7. The method according to claim 6 further comprising exposing the eucaryotic cell to methotrexate, so that resistant colonies are selected which contain amplified levels of the coding sequences for dihydrofolate reductase and simian transforming growth factor-β1.

8. The method according to claim 7 in which the eucaryotic cell comprises a dihydrofolate reductase-deficient Chinese Hamster Ovary cell.

9. A method for producing transforming growth factor-β1, comprising

(a) culturing transfectant CHO-TGF-β1-3/2000 as deposited with the ATCC and assigned accession number CRL 9434; and

(b) recovering the transforming growth factor-β1 from the culture.

10. The method according to claim 9 in which the transfectant is cultured in the presence of methotrexate.

11. A eucaryotic cell containing a nucleotide sequence encoding simian transforming growth factor-β1 under the control of a second nucleotide sequence that regulates gene expression so that the eucaryotic cell produces active transforming growth factor-β1.

12. The eucaryotic cell according to claim 11 as used and defined in anyone of claims 2 to 8.

13. A cell line comprising CHO-TGF-β1-3/2000 as deposited with the ATCC and assigned accession number CRL 9434.

14. A substantially pure polypeptide comprising the amino acid sequence substantially as depicted in FIG. 1, preferably comprising amino acid residue numbers 1 to 390;
amino acid residue numbers 30 to 390; or
amino acid residue numbers 30 to 278.

15. The substantially pure polypeptide of claim 14 which is glycosylated, or
phosphorylated, preferably containing
at least one mannose-6-phosphate.

16. The substantially pure polypeptide of claim 15 in which the mannose-6-phosphate is linked to an asparagine linked sugar chain of the rTGF-$\beta$1 precursor.

17. The substantially pure polypeptide of claim 16 in which the asparagine site is amino acid residue number 82, 136 or 176.

18. The use of transforming growth factor-$\beta$1 for preparing a pharmaceutical composition for the treatment of tumors.

```
Simian            -261 AGGGGATCTGTGGCAGGTCGGAGA---AAGATC---CGTCTCCTGGTACCAGATCTCGCCCATCTAGGTT  -198
Human                  CTCC..C...CCA...A..CCCT.TTC....C.ACC.AC..T............G.............

Simian  ATTTCCGTGGGATACTGAGACACCCCCGGTCCAAGCCTCCCCTCCACCACTGCGCCCTTCTCCCGTAGGA-CCTCAACTTTCCCTCGAGGCCCTCCTAC  -100
Human   ......................................................................G.....G.....................

Simian  CTTTTCCCGGGGGACCCCCAGCCCCTGCAGGGGCGGGGCCTCCCCACCAAACTAGCCCTGTTCGCGCTCTCGGCAGTGCCGGGGGGCGCCGCCTCCCC  -1
Human   .....G.....A..............................C..C..................................................

                                           10                                      20                 Gly Pro
        Met Pro Pro Ser Gly Leu Arg Leu Leu Pro Leu Leu Leu Pro Leu Leu Trp Leu Leu Val Leu Thr Pro Ser Arg
Simian  ATG CCG CCC TCC GGG CTG CGG CTG CTG CCG CTG CTG CTA CCG CTG CTG TGG CTA CTG GTG CTG ACG CCT AGC CGG  75
Human   ... ... ... ... ... ... ... ... ... ... ... ... ... ... ... ... ... ... ... ... ... ... ... G.. .C.

                                           35                                      45          Ala
        Pro Ala Ala Gly Leu Ser Thr Cys Lys Thr Ile Asp Met Glu Leu Val Lys Arg Lys Arg Ile Glu Thr Lle Arg
Simian  CCG GCC GCA GGA CTA TCC ACC TGC AAG ACT ATC GAC ATC GAG CTG GTG AAG CGG AAG CGC ATC GAG ACC ATC CGC  150
Human   ... ... ... ... ... ... ... ... ... ... ... ... ... ... ... ... ... ... ... ... ... ... G.. ... ...

                                           60                                      70
        Gly Gln Ile Leu Ser Lys Leu Arg Leu Ala Ser Pro Pro Ser Gln Gly Glu Val Pro Pro Gly Pro Leu Pro Glu
Simian  GGC CAG ATC CTG TCC AAG CTG CGG CTC GCC AGC CCC CCG AGC CAG GGG GAG GTG CCG CCC GGC CCG CTG CCC GAG  225
Human   ... ... ... ... ... ... ... ... ... ... ... ... ... ... ... ... ... ... ... ... ... ... ... ... ...

                                           85                                      95
        Ala Val Leu Ala Leu Tyr Asn Ser Thr Arg Asp Arg Val Ala Gly Glu Ser Ala Glu Pro Glu Pro Glu Pro Glu
Simian  GCC GTG CTC GCC CTG TAC AAC AGC ACC CGC GAC CGG GTG GCC GGG GAG AGT GCG GAG CCG GAG CCC GAA CCG GAG  300
Human   ... ... ... ... ... ... ... ... ... ... ... ... ... ... ... ... ... ..A ..A ... ... ... ..G ..T ...

                                          110                                     120
        Ala Asp Tyr Tyr Ala Lys Glu Val Thr Arg Val Leu Met Val Glu Thr His Asn Glu Ile Tyr Asp Lys Phe Lys
Simian  GCC GAC TAC TAC GCC AAG GAG GTC ACC CGC GTG CTA ATG GTG GAA ACC CAC AAC GAA ATC TAT GAC AAG TTC AAG  375
Human   ... ... ... ... ... ... ... ... ... ... ... ... ... ... ... ... ... ... ... ... ... ... ... ... ...

                                          135                                     145
        Gln Ser Thr His Ser Ile Tyr Met Phe Phe Asn Thr Ser Glu Leu Arg Glu Ala Val Pro Glu Pro Val Leu Leu
Simian  CAG AGC ACA CAC AGC ATA TAT ATG TTC TTC AAC ACA TCA GAG CTC CGA GAA GCA GTA CCT GAA CCT GTG TTG CTC  450
Human   ... ..T ... ... ... ... ... ... ... ... ... ... ... ... ... ... ... ..G ... ... ... ..C ... ... ...

                                    Arg      160                                  170
        Ser Arg Ala Glu Leu Arg Leu Leu --- Arg Leu Lys Leu Lys Val Glu Gln His Val Glu Leu Tyr Gln Lys Tyr
Simian  TCC CGG GCA GAG CTG CGT CTG CTG --- AGG CTC AAG TTA AAA GTC GAG CAG CAT GTG GAG CTG TAC CAG AAA TAC  522
Human   ... ... ... ... ... ... ... ... AGG ... ... ... ... ... ... ... ... ..C ... ... ... ... ... ... ...
```

FIG. 1

```
                                                185                          Asp                 195
         Ser Asn Asn Ser Trp Arg Tyr Leu Ser Asn Arg Leu Leu Ala Pro Ser Asn Ser Pro Glu Trp Leu Ser Phe Asp
Simian   AGC AAC AAT TCC TGG CGA TAC CTC AGC AAC CGG CTG CTG GCG CCC AGC AAC TCG CCG GAG TGG TTG TCT TTT GAT    597
Human    ... ... ... ... ... ... ... ... ... ... ... ... ... ..A ... ... G.. ... ..A ... ... ..A ... ... ...

                                                210                                                  220
         Val Thr Gly Val Val Arg Gln Trp Leu Ser Arg Gly Gly Glu Ile Glu Gly Phe Arg Leu Ser Ala His Cys Ser
Simian   GTC ACC GGA GTT GTG CGG CAG TGG TTG AGC CGC GGA GGG GAA ATT GAG GGC TTT CGC CTT AGC GCC CAC TGC TCC    672
Human    ... ... ... ... ... ... ... ... ... ... ..T ... ... ... ... ... ... ... ... ... ... ... ... ... ...

                     Arg                        235                                                  245
         Cys Asp Ser Lys Asp Asn Thr Leu Gln Val Asp Ile Asn Gly Phe Thr Thr Gly Arg Arg Gly Asp Leu Ala Thr
Simian   TGT GAC AGC AAA GAT AAC ACA CTG CAA GTG GAC ATC AAC GGG TTC ACT ACC GGC CGC CGA GGT GAC CTG GCC ACA    747
Human    ... ... ... .GG ... ... ... ... ... ... ... ... ... ... ... ... ... ... ... ... ... ... ... ... ..G

                                                260                                                  270
         Ile His Gly Met Asn Arg Pro Phe Leu Leu Leu Met Ala Thr Pro Leu Glu Arg Ala Gln His Leu Gln Ser Ser
Simian   ATT CAT GGC ATG AAC CGG CCT TTC CTG CTT CTC ATG GCC ACC CCG CTG GAG AGG GCC CAA CAT CTG CAA AGC TCC    823
Human    ... ... ... ... ... ... ... ... ... ... ... ... ... ... ... ... ... ... ... ..G ... ... ... ... ...

                                                285                                                  295
         Arg His Arg Arg Ala Leu Asp Thr Asn Tyr Cys Phe Ser Ser Thr Glu Lys Asn Gys Cys Val Arg Gln Leu Tyr
Simian   CGG CAC CGC CGA GCC CTG GAC ACC AAC TAC TGC TTC AGC TCC ACG GAG AAG AAC TGC TGC GTG CGG CAG CTG TAT    897
Human    ... ... ... ... ... ... ... ... ... ..T ... ... ... ... ... ... ... ... ... ... ... ... ... ... ..C

                                                310                                                  320
         Ile Asp Phe Arg Lys Asp Leu Gly Trp Lys Trp Ile His Glu Pro Lys Gly Tyr His Ala Asn Phe Cys Leu Gly
Simian   ATT GAC TTC CGC AAG GAC CTC GGC TGG AAG TGG ATC CAC GAG CCC AAG GGC TAC CAT GCC AAC TTC TGC CTG GGG    972
Human    ... ... ... ... ... ... ... ... ... ... ... ... ... ... ... ... ... ... ... ... ... ... ... ..C ...

                                                335                                                  345
         Pro Cys Pro Tyr Ile Trp Ser Leu Asp Thr Gln Tyr Ser Lys Val Leu Ala Leu Tyr Asn Gln His Asn Pro Gly
Simian   CCC TGT CCC TAC ATT TGG AGC CTG GAC ACG CAG TAC AGC AAG GTC CTG GCC CTG TAC AAC CAG CAT AAC CCG GGC   1047
Human    ... ..C ... ... ... ... ... ... ... ... ... ... ... ... ... ... ... ... ... ... ... ... ... ... ...

                                                360                                                  370
         Ala Ser Ala Ala Pro Cys Cys Val Pro Gln Ala Leu Glu Pro Leu Pro Ile Val Tyr Tyr Val Gly Arg Lys Pro
Simian   GCC TCG GCG GCG CCG TGC TGC GTG CCG CAG GCG CTG GAG CCA CTG CCC ATC GTG TAC TAC GTG GGC CGC AAG CCC   1122
Human    ... ... ... ... ... ... ... ... ... ... ... ... ... ..G ... ... ... ... ... ... ... ... ... ... ...

                                                385
         Lys Val Glu Gln Leu Ser Asn Met Ile Val Arg Ser Cys Lys Cys Ser
Simian   AAG GTG GAG CAG CTG TCC AAC ATG ATC GTG CGC TCC TGA AAA TGC AGC TGA GGCCCCGCCCCGCCCCGCCCCACCCCGGCAG   1204
Human    ... ... ... ... ... ... ... ... ... ... ... ... ... ..G ... ... ... ..T.................G.........

Simian   GCCCGGCCCCGCCCCACCCCACCCCCGCTGTCTTGCCCTTGGGGGCTGTATTTAAGGACACCCGTGCCCCAAGCCCACCTGGGGCCCCATTAAAGA   1300
Human    ..........A....G....G.........C.......A.........................................................
```

FIG. 1 (CONT.)

FIG. 2

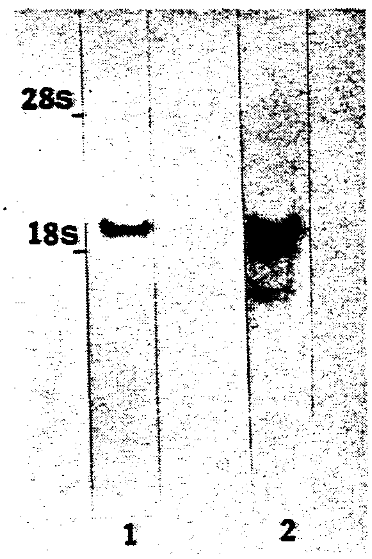

FIG. 3

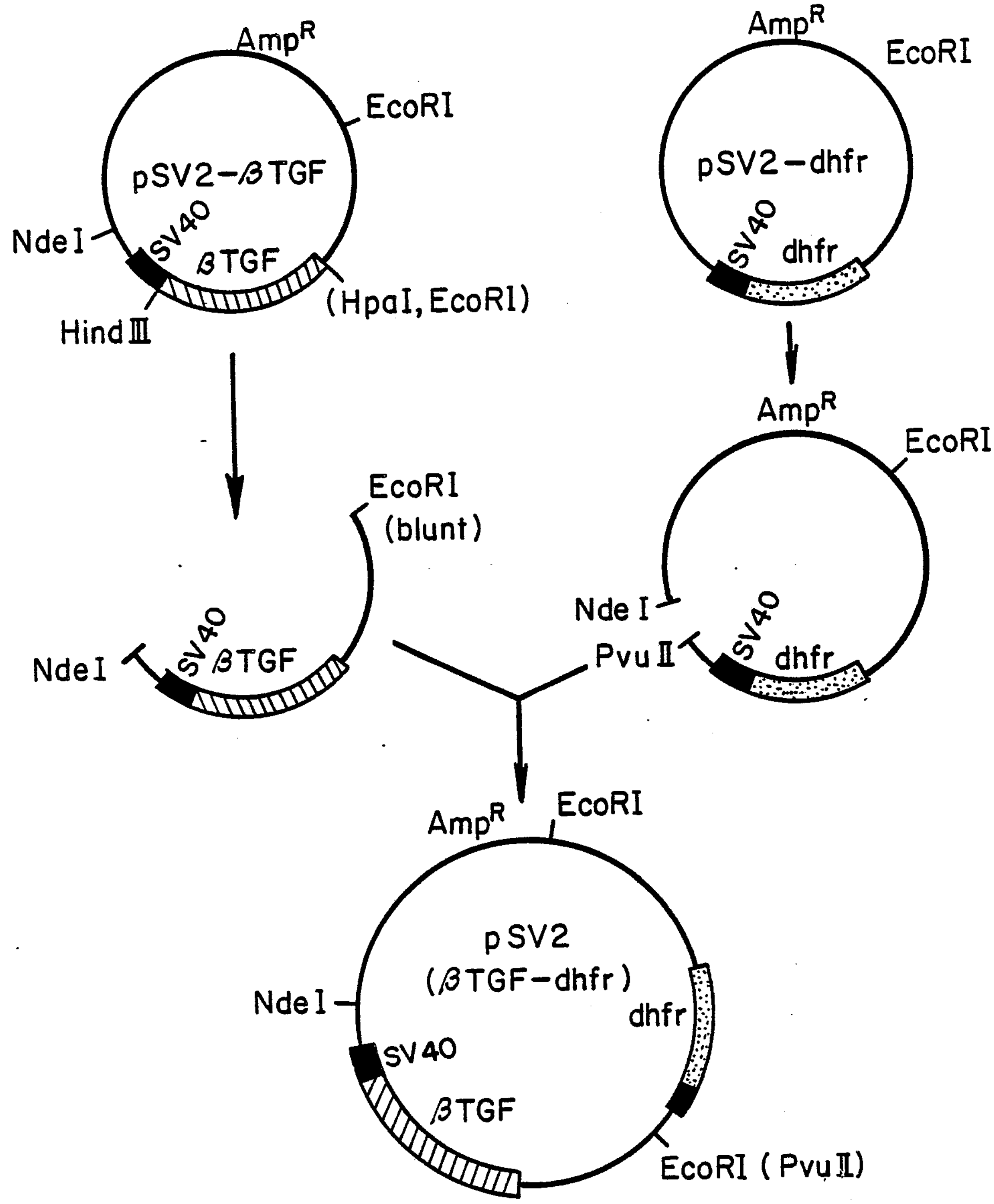

AmpR
EcoRI
pSV2-βTGF
NdeI
SV40
βTGF
(HpaI, EcoRI)
HindIII
AmpR
EcoRI
pSV2-dhfr
SV40
dhfr
AmpR
EcoRI
EcoRI
(blunt)
NdeI
SV40
βTGF
NdeI
PvuII
SV40
dhfr
AmpR
EcoRI
pSV2
(βTGF-dhfr)
NdeI
dhfr
SV40
βTGF
EcoRI (PvuII)

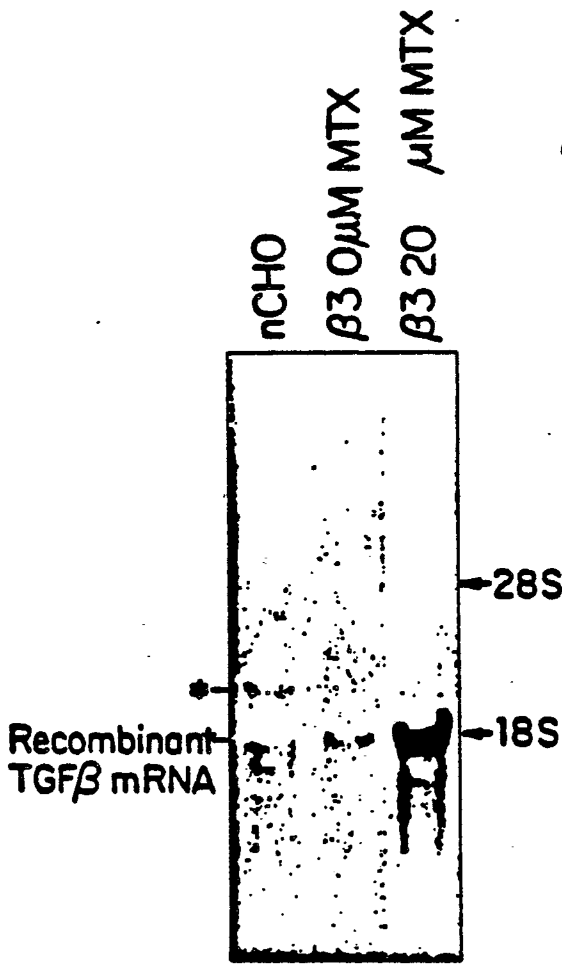
nCHO
β3 0μM MTX
β3 20 μM MTX
28S
18S
Recombinant TGFβ mRNA

FIG. 4A

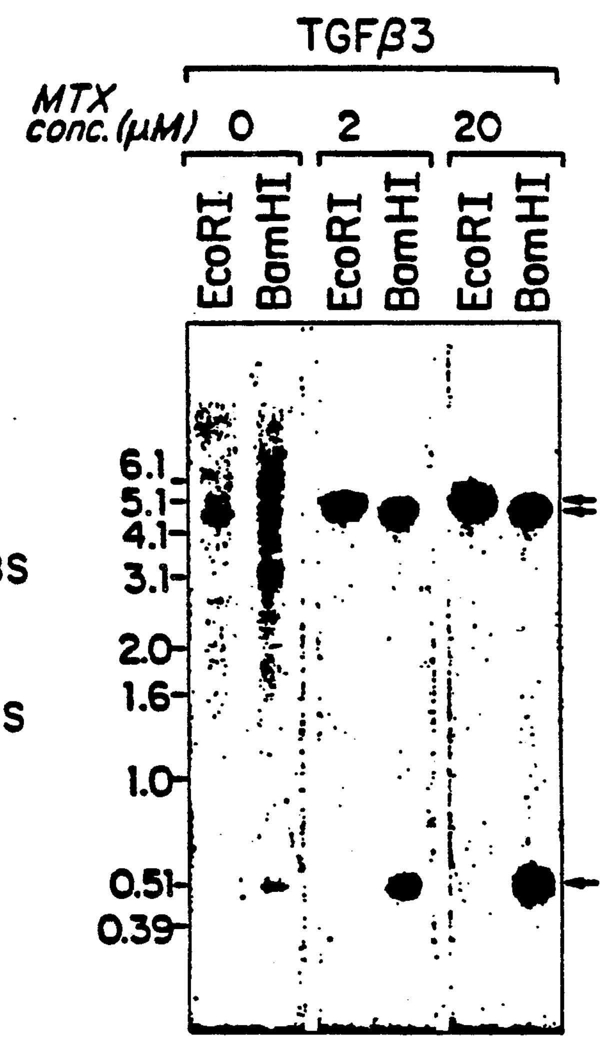
TGFβ3
MTX conc. (μM)
0
2
20
EcoRI
BamHI
EcoRI
BamHI
EcoRI
BamHI
6.1
5.1
4.1
3.1
2.0
1.6
1.0
0.51
0.39

FIG. 4B

FIG. 5

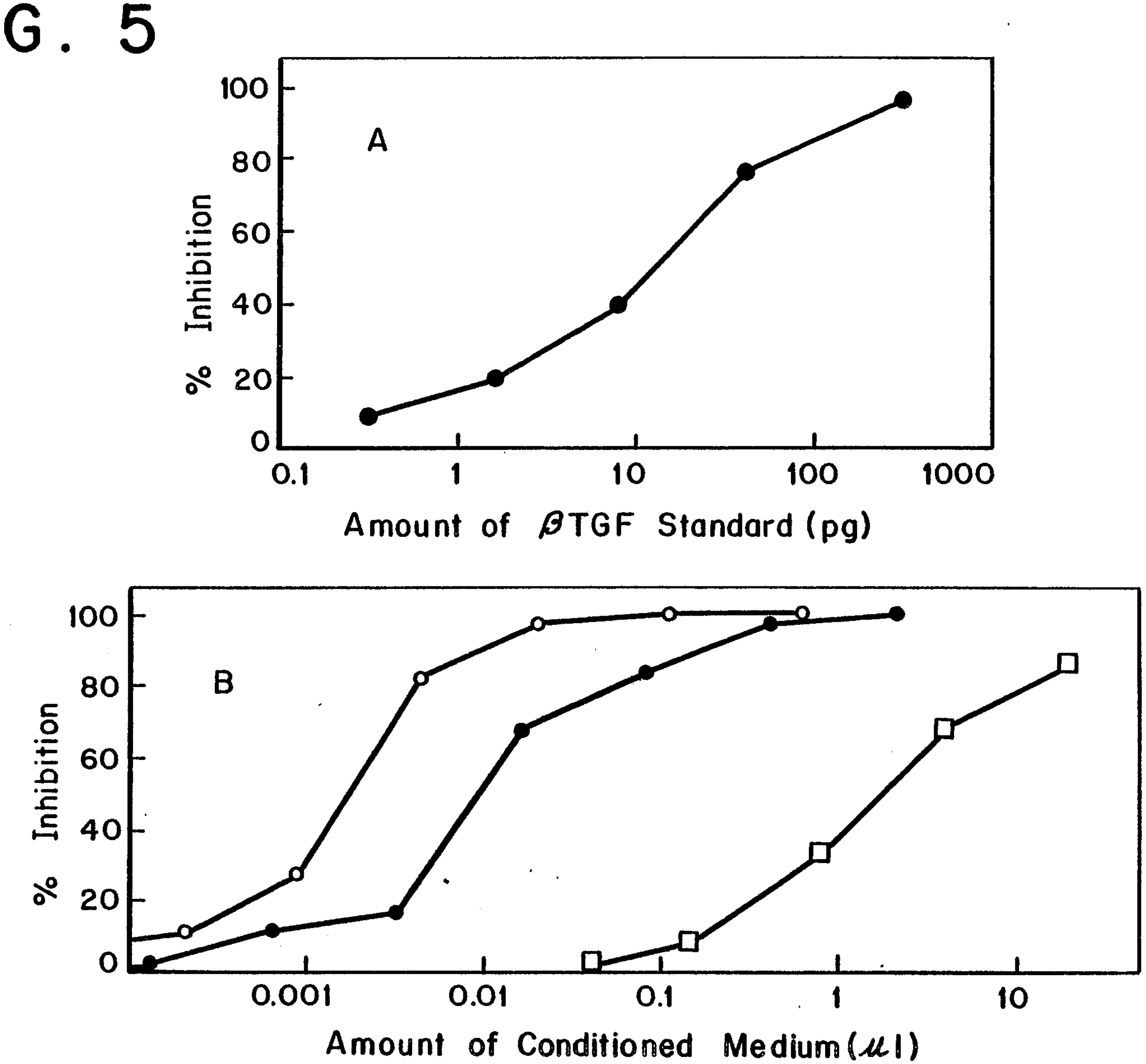
A
% Inhibition
100
80
60
40
20
0
0.1
1
10
100
1000
Amount of βTGF Standard (pg)
B
% Inhibition
100
80
60
40
20
0
0.001
0.01
0.1
1
10
Amount of Conditioned Medium (μl)

FIG. 6

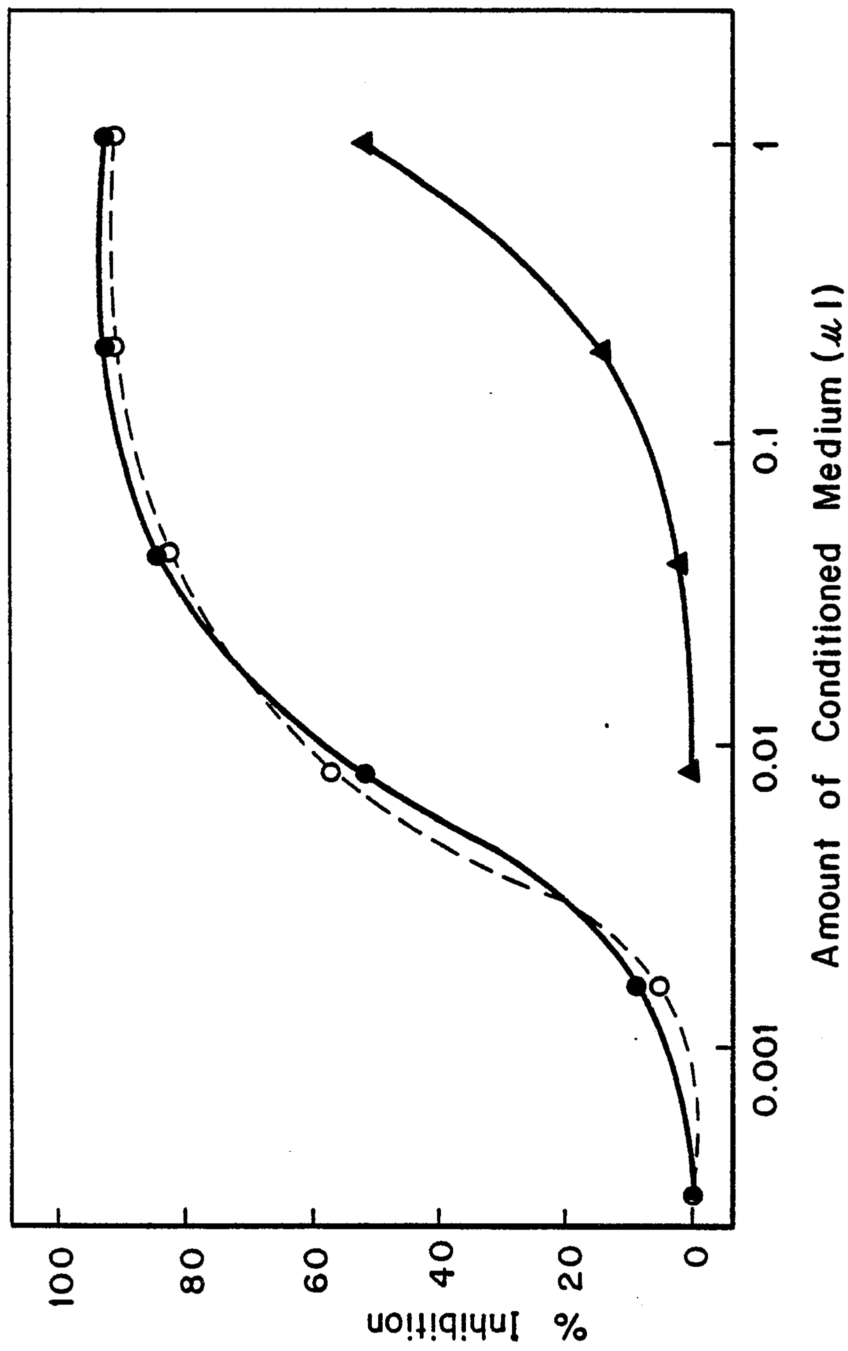
% Inhibition
100
80
60
40
20
0
0.001
0.01
0.1
1
Amount of Conditioned Medium (μl)

# FIG. 7

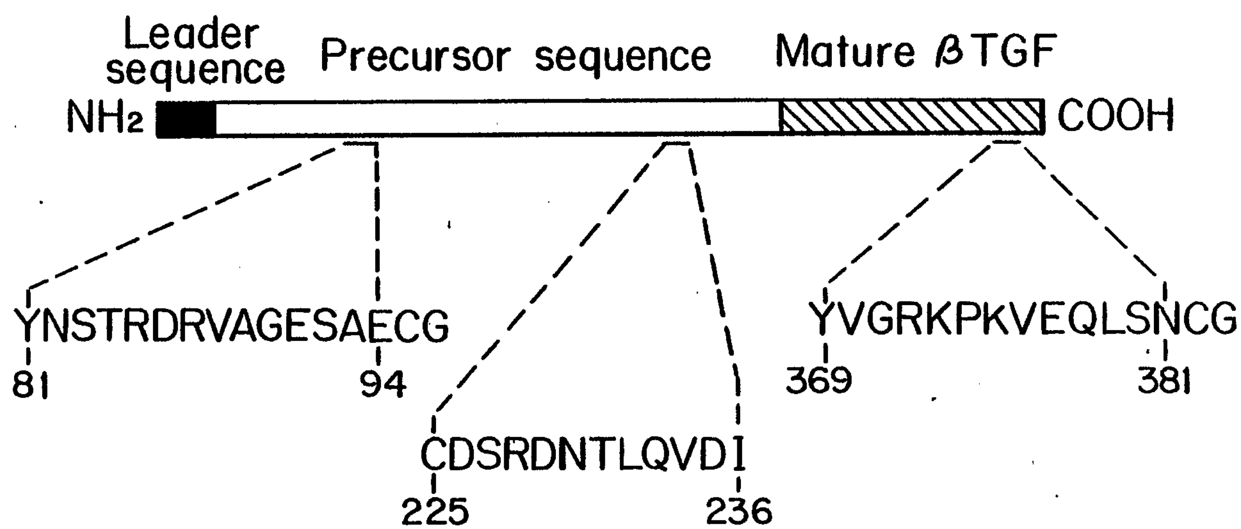
Leader sequence
Precursor sequence
Mature βTGF
NH2
COOH
YNSTRDRVAGESAECG
81
94
CDSRDNTLQVDI
225
236
YVGRKPKVEQLSNCG
369
381

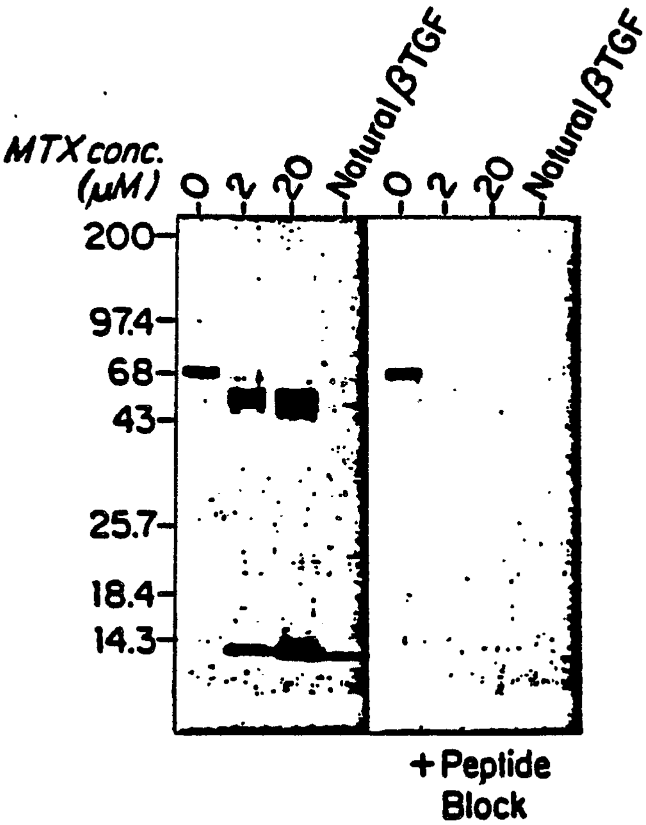
MTX conc. (μM)
0
2
20
Natural βTGF
0
2
20
Natural βTGF
200
97.4
68
43
25.7
18.4
14.3
+ Peptide Block

FIG. 8A

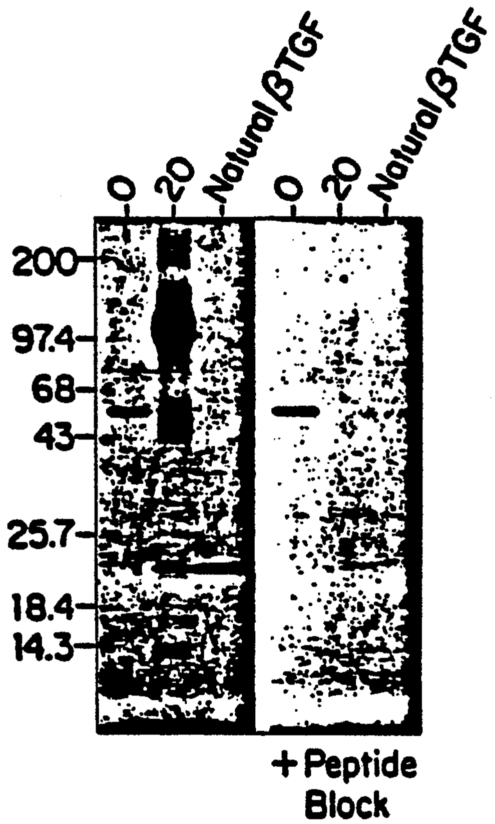
0
20
Natural βTGF
0
20
Natural βTGF
200
97.4
68
43
25.7
18.4
14.3
+ Peptide Block

FIG. 8B

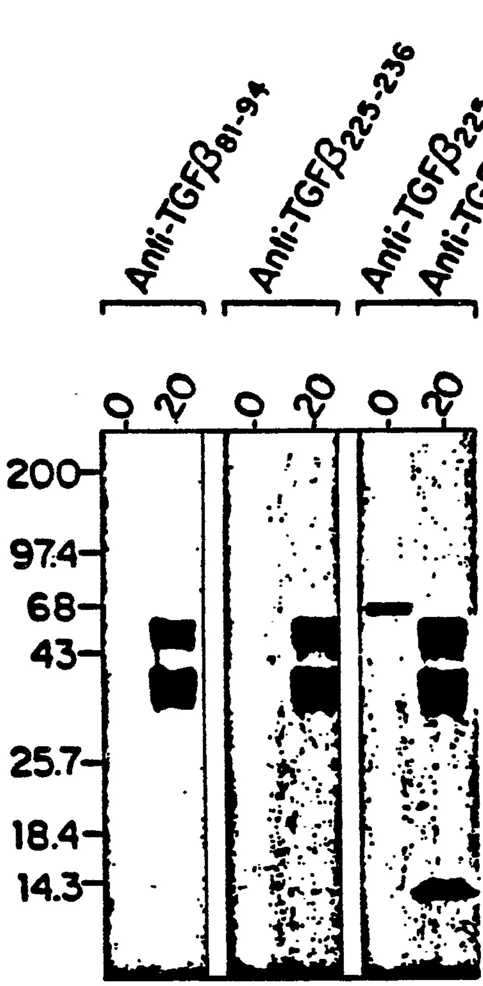
Anti-TGFβ81-94
Anti-TGFβ225-236
Anti-TGFβ225-236 +
Anti-TGFβ369-381
0
20
0
20
0
20
200
97.4
68
43
25.7
18.4
14.3

FIG. 9A

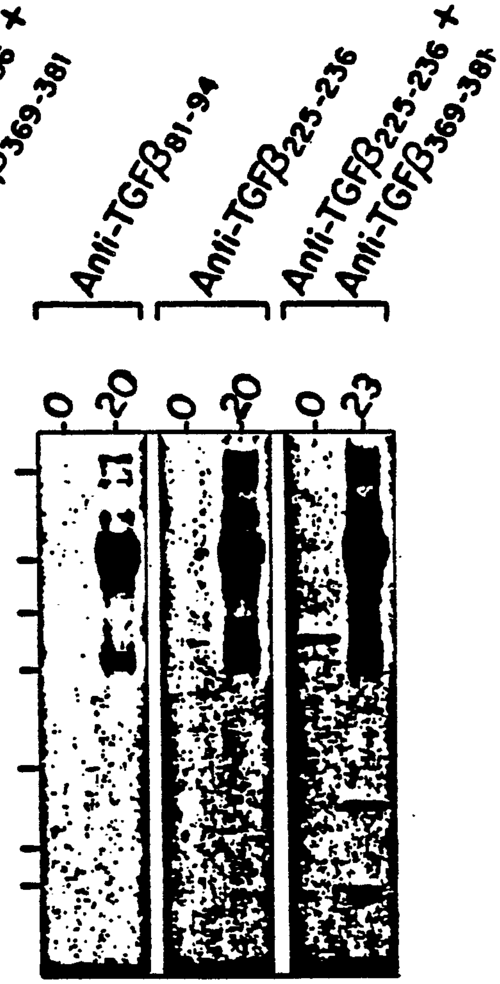
Anti-TGFβ81-94
Anti-TGFβ225-236
Anti-TGFβ225-236 +
Anti-TGFβ369-381
0
20
0
20
0
23

FIG. 9B

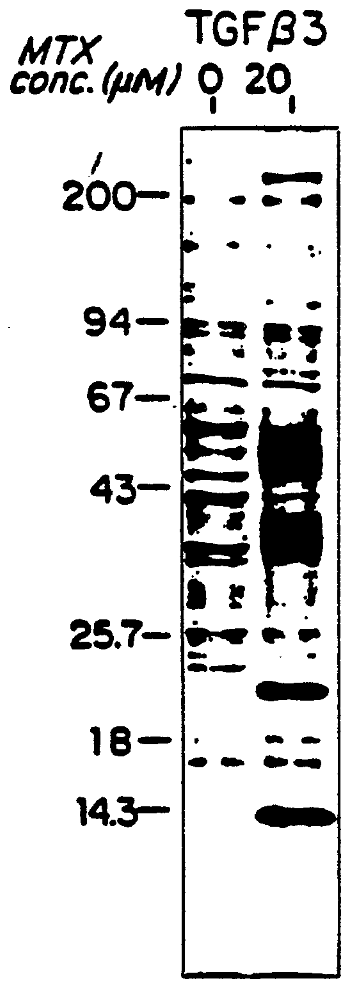


FIG. 10

FIG.11

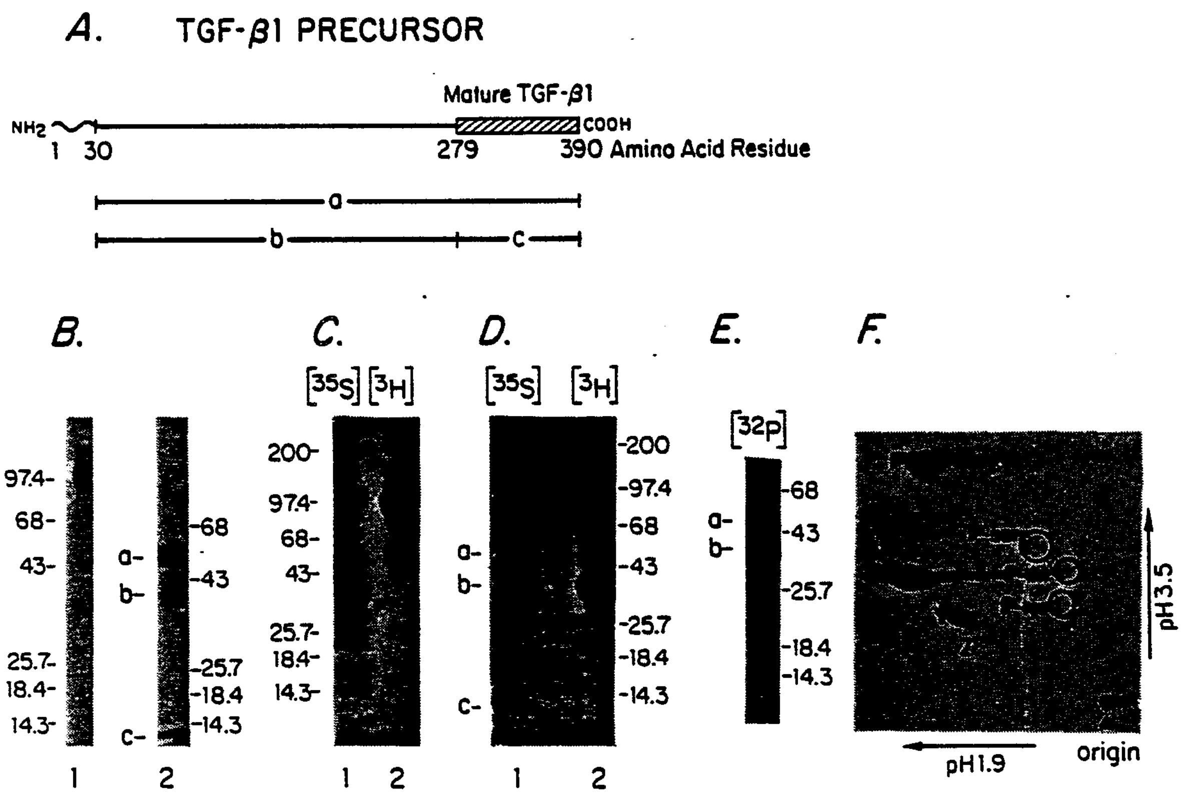
A.
TGF-β1 PRECURSOR
Mature TGF-β1
NH2
COOH
1
30
279
390 Amino Acid Residue
a
b
c
B.
C.
D.
E.
F.
[35S] [3H]
[35S] [3H]
[32P]
200-
97.4-
68-
43-
25.7-
18.4-
14.3-
-200
-97.4
-68
-43
-25.7
-18.4
-14.3
a-
b-
c-
1
2
pH1.9
origin
pH3.5

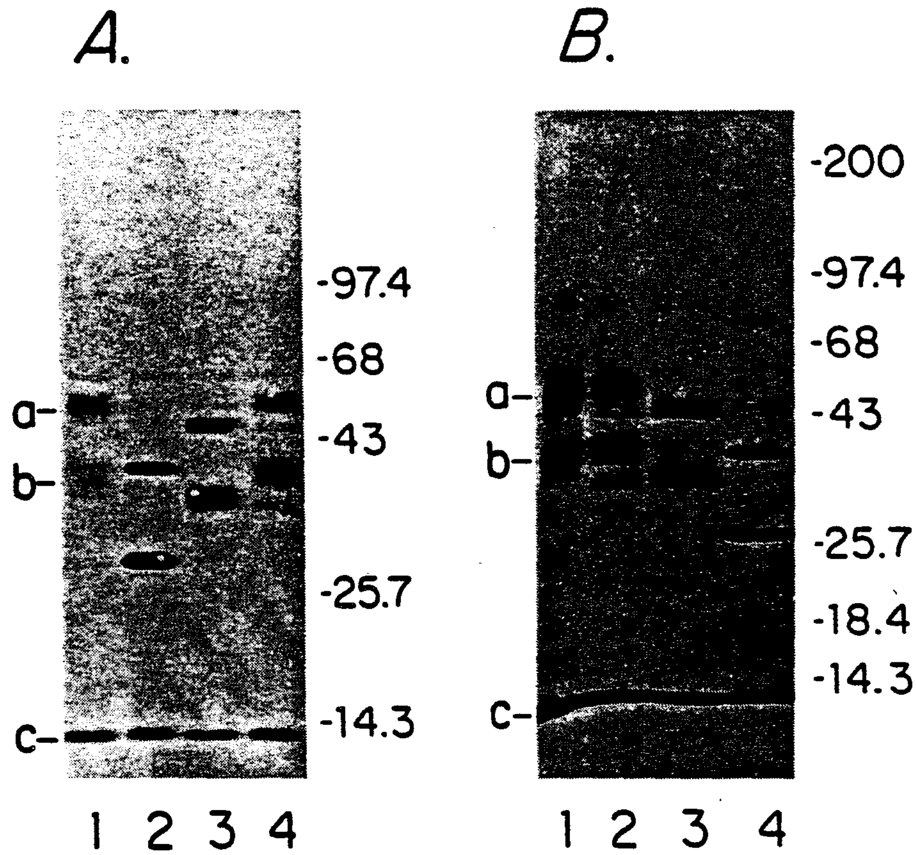


FIG.12

FIG. 13

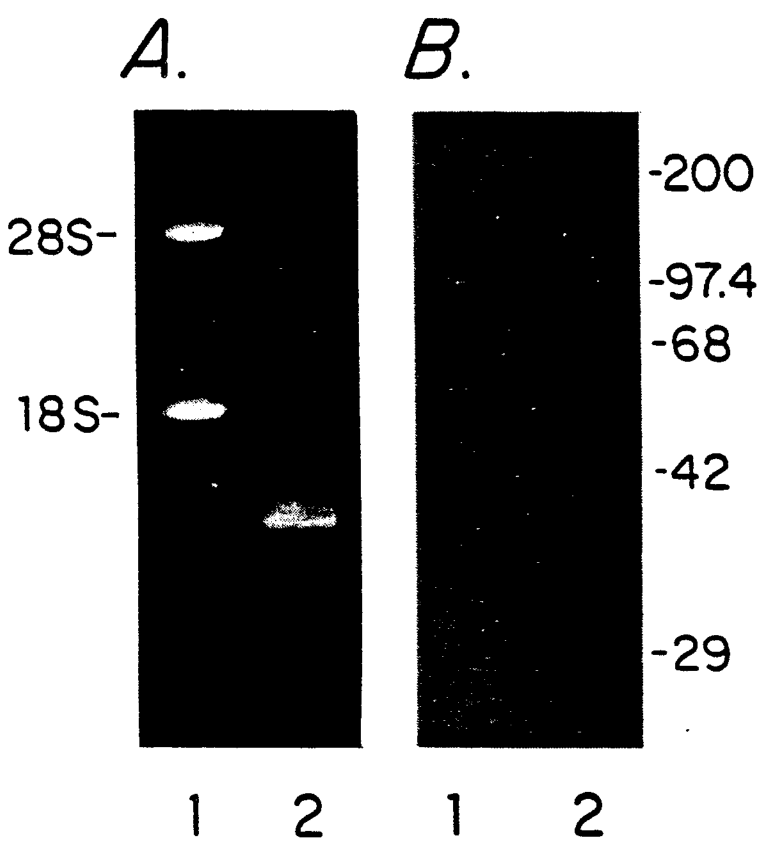

FIG. 14

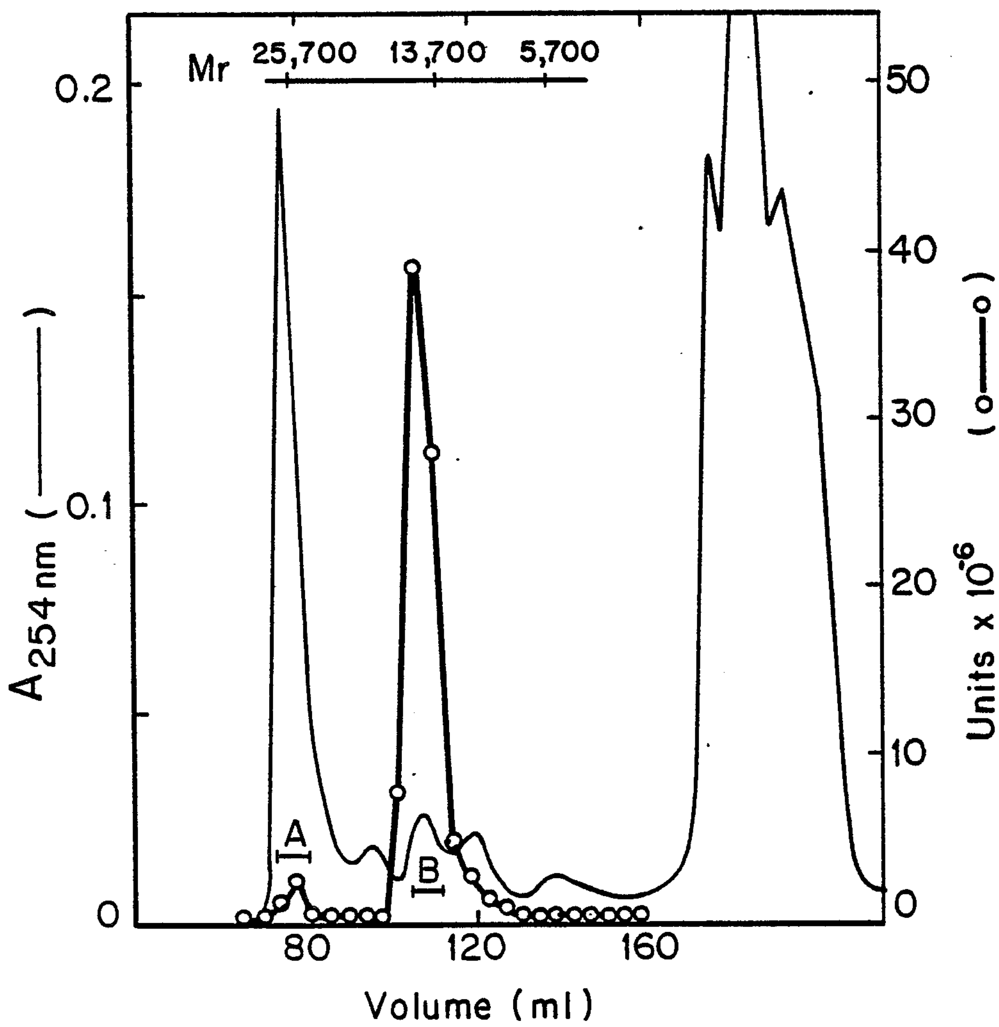
Mr
25,700
13,700
5,700
0.2
0.1
0
$A_{254\,nm}$ ( ——— )
50
40
30
20
10
0
Units x $10^{-6}$ (o—o)
A
B
80
120
160
Volume (ml)

FIG. 15

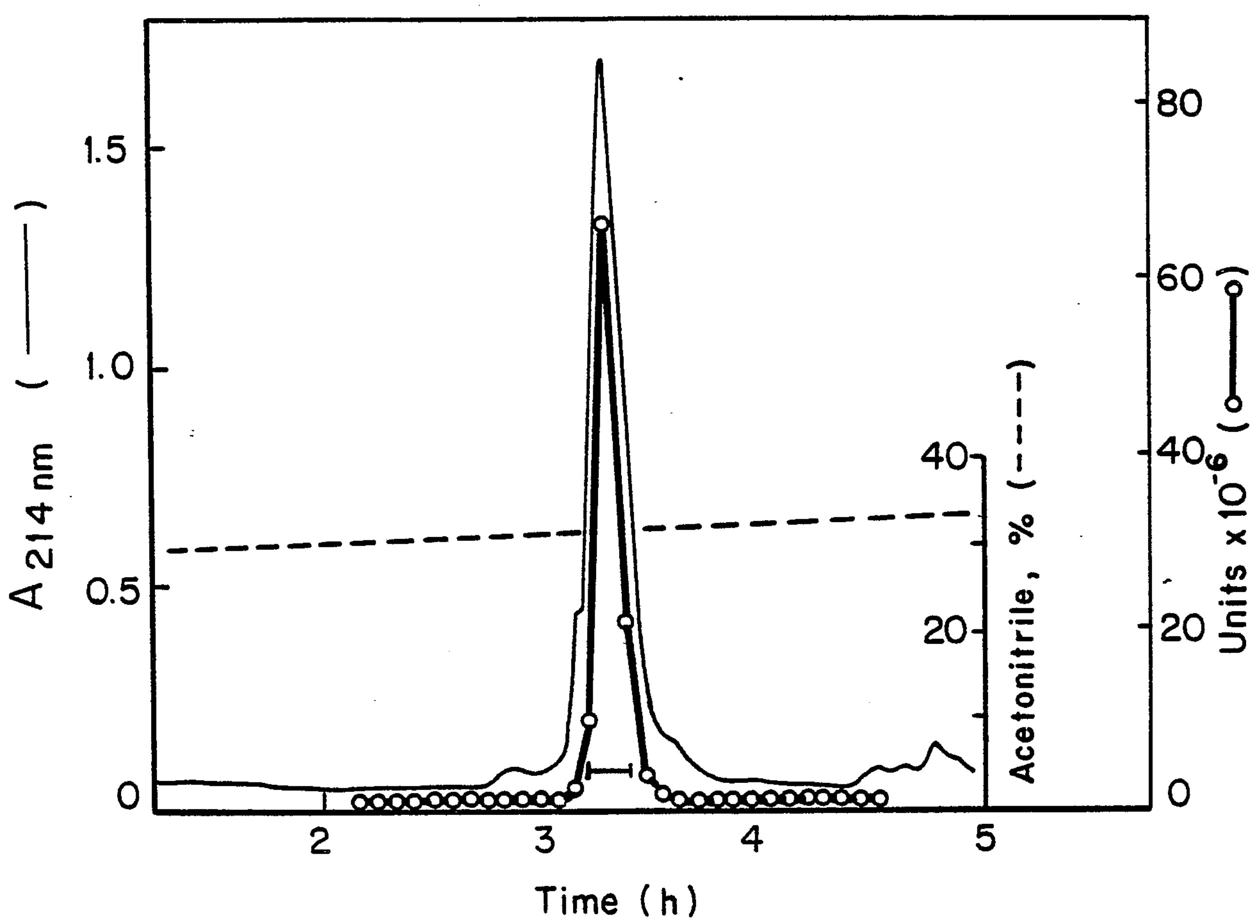
1.5
1.0
0.5
0
A 214 nm (——)
80
60
40
20
0
Units x $10^{-6}$ (o—o)
40
20
Acetonitrile, % (----)
2
3
4
5
Time (h)

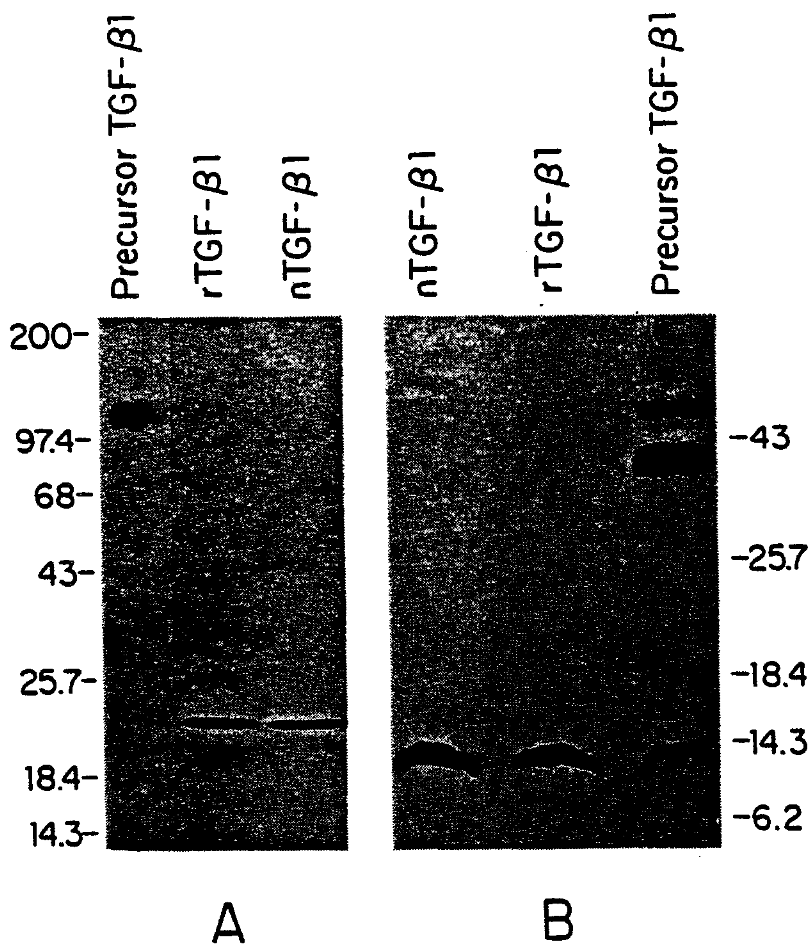
Precursor TGF-β1
rTGF-β1
nTGF-β1
nTGF-β1
rTGF-β1
Precursor TGF-β1
200-
97.4-
68-
43-
25.7-
18.4-
14.3-
-43
-25.7
-18.4
-14.3
-6.2
A
B

FIG.16

FIG. 17

A

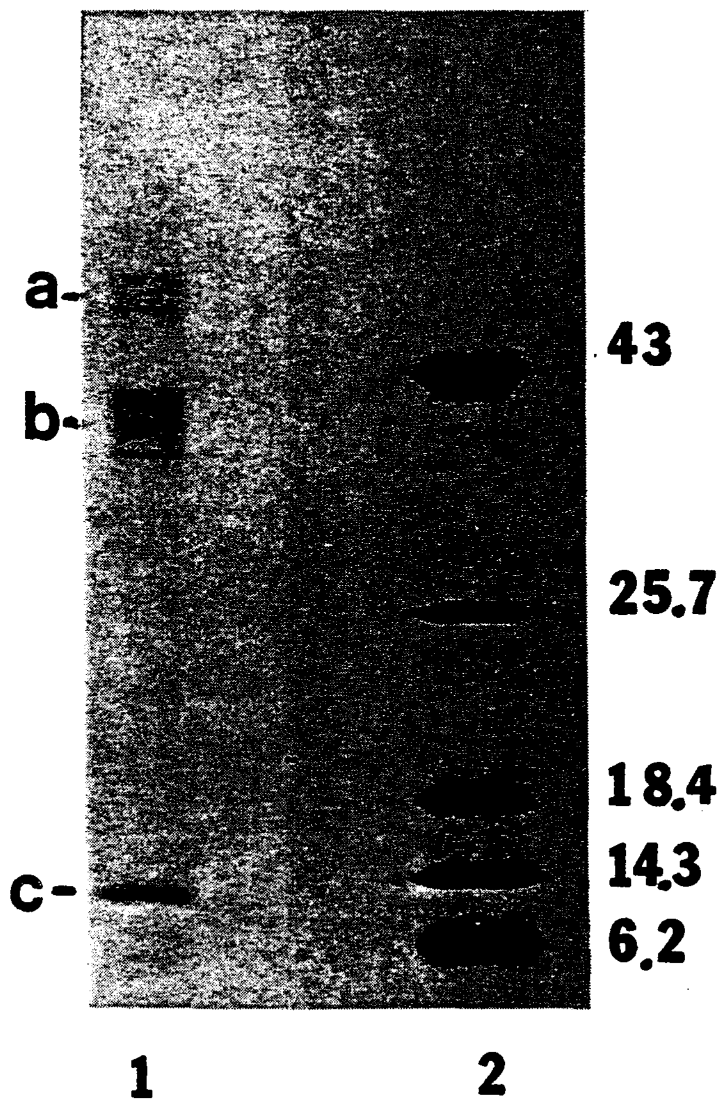

FIG. 18

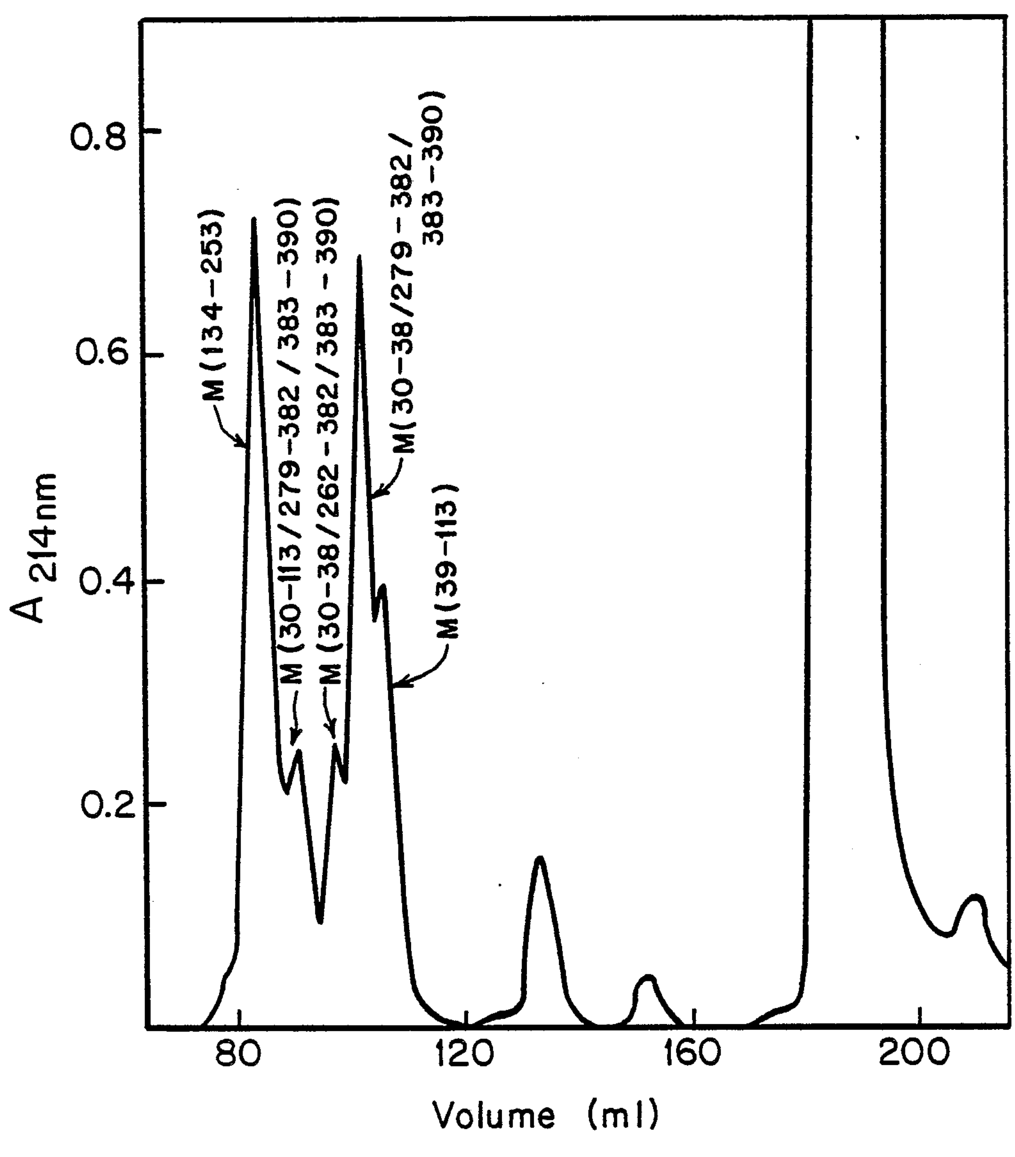
0.8
0.6
0.4
0.2
A 214nm
M(134-253)
M(30-113/279-382 / 383-390)
M(30-38/262-382/383-390)
M(30-38/279-382/
383-390)
M(39-113)
80
120
160
200
Volume (ml)

FIG. 19

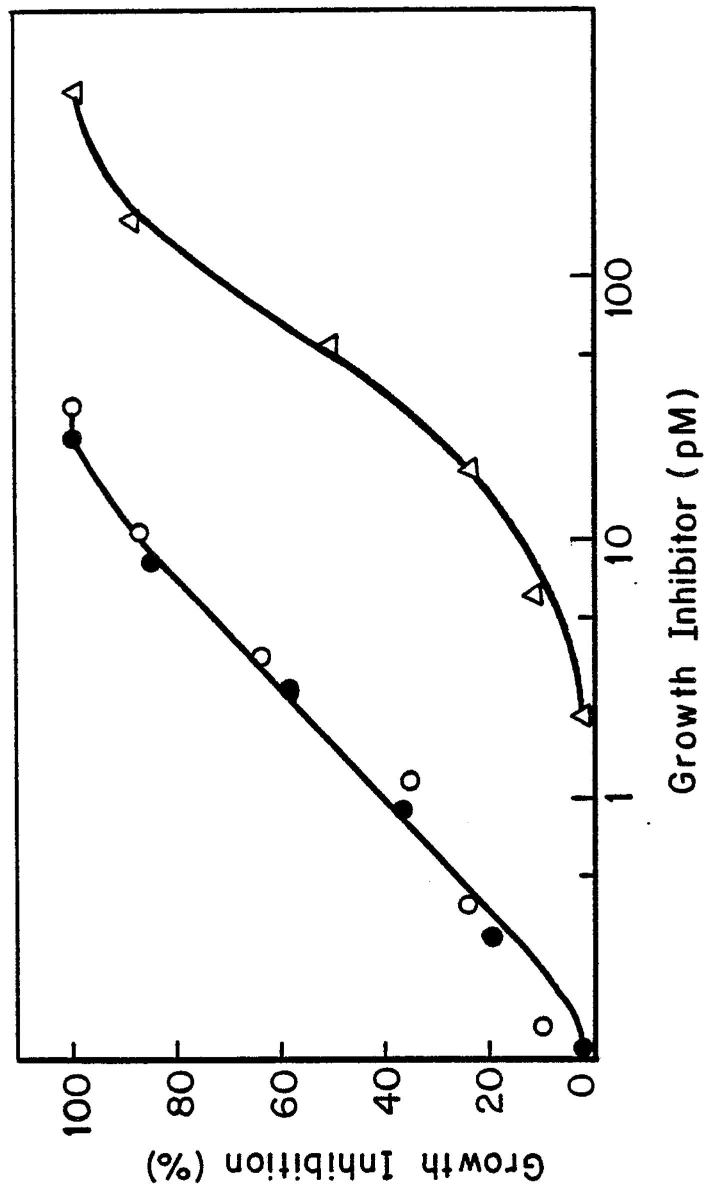
Growth Inhibition (%)
0
20
40
60
80
100
Growth Inhibitor (pM)
1
10
100

FIG. 20

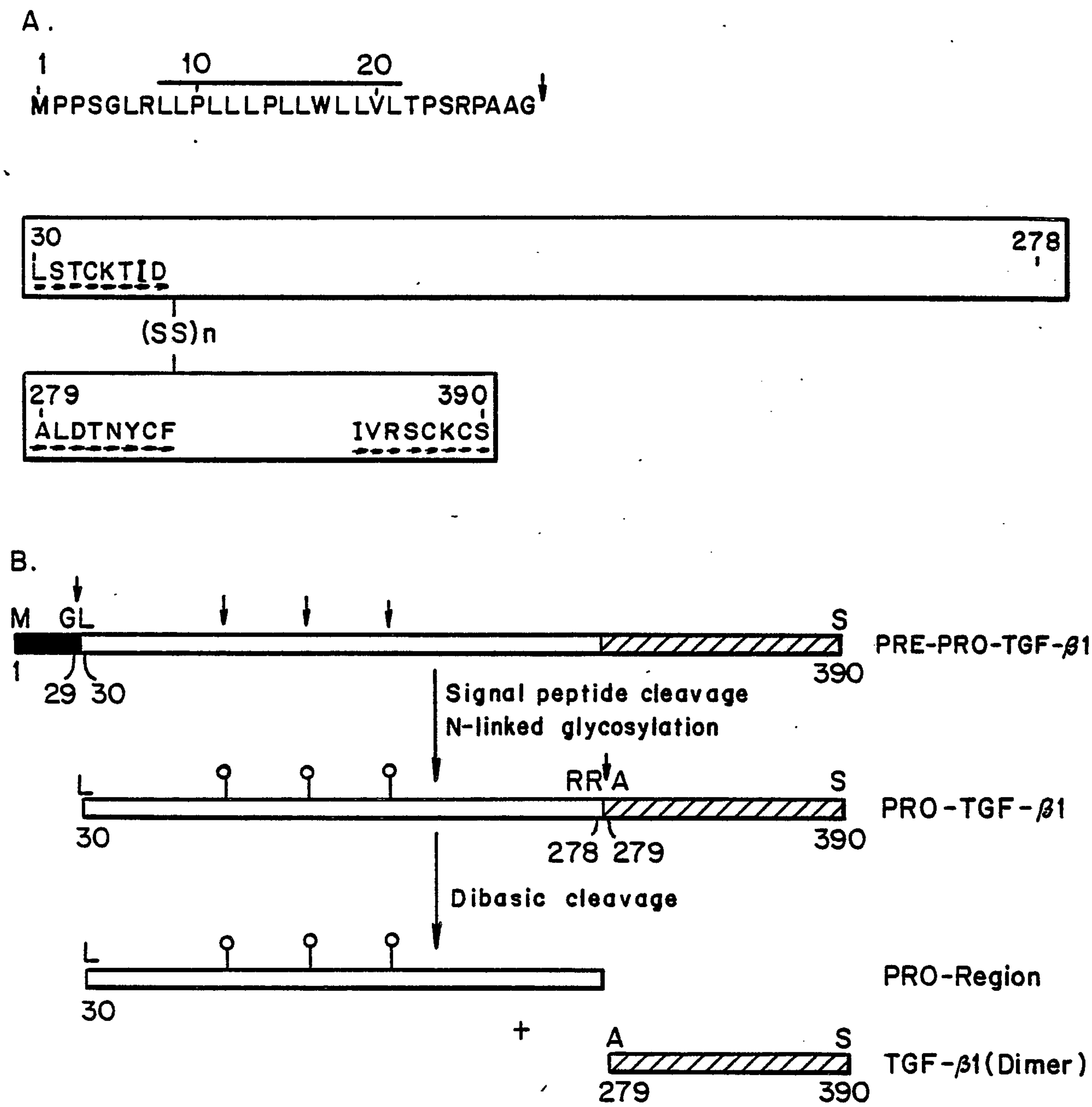
A.
1
10
20
MPPSGLRLLPLLLPLLWLLVLTPSRPAAG
30
278
LSTCKTID
(SS)n
279
390
ALDTNYCF
IVRSCKCS
B.
M
GL
S
PRE-PRO-TGF-β1
1
29
30
390
Signal peptide cleavage
N-linked glycosylation
L
RR A
S
PRO-TGF-β1
30
278
279
390
Dibasic cleavage
L
PRO-Region
30
+
A
S
TGF-β1(Dimer)
279
390

FIG. 21

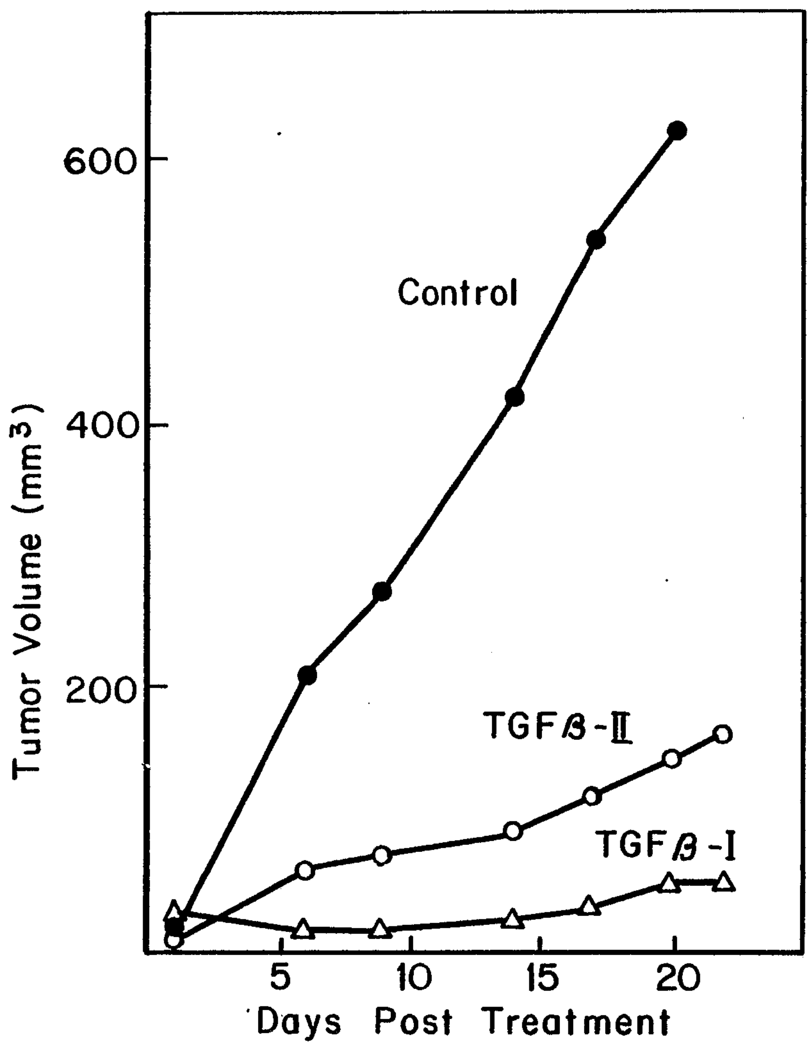
Tumor Volume (mm³)
600
400
200
Control
TGFβ-II
TGFβ-I
5
10
15
20
Days Post Treatment

FIG. 22

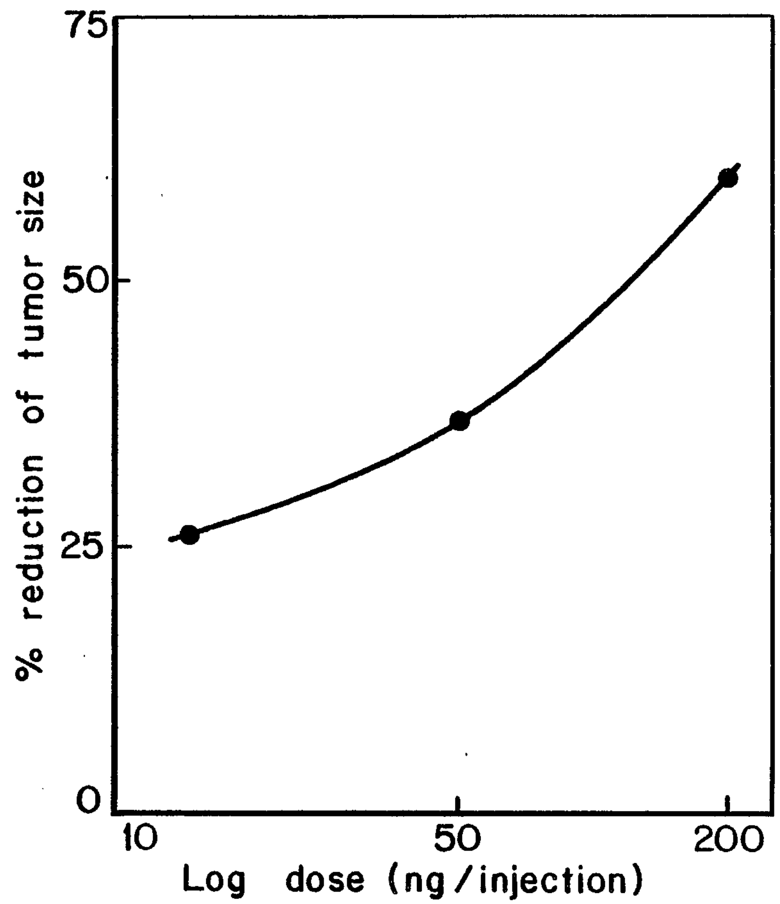
% reduction of tumor size
75
50
25
0
10
50
200
Log dose (ng/injection)

FIG. 23

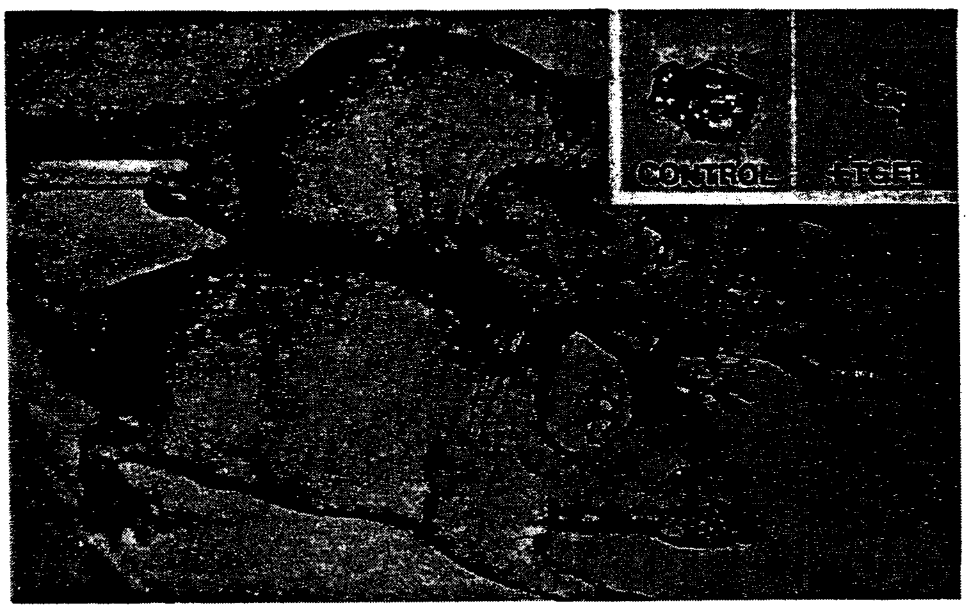

FIG. 24

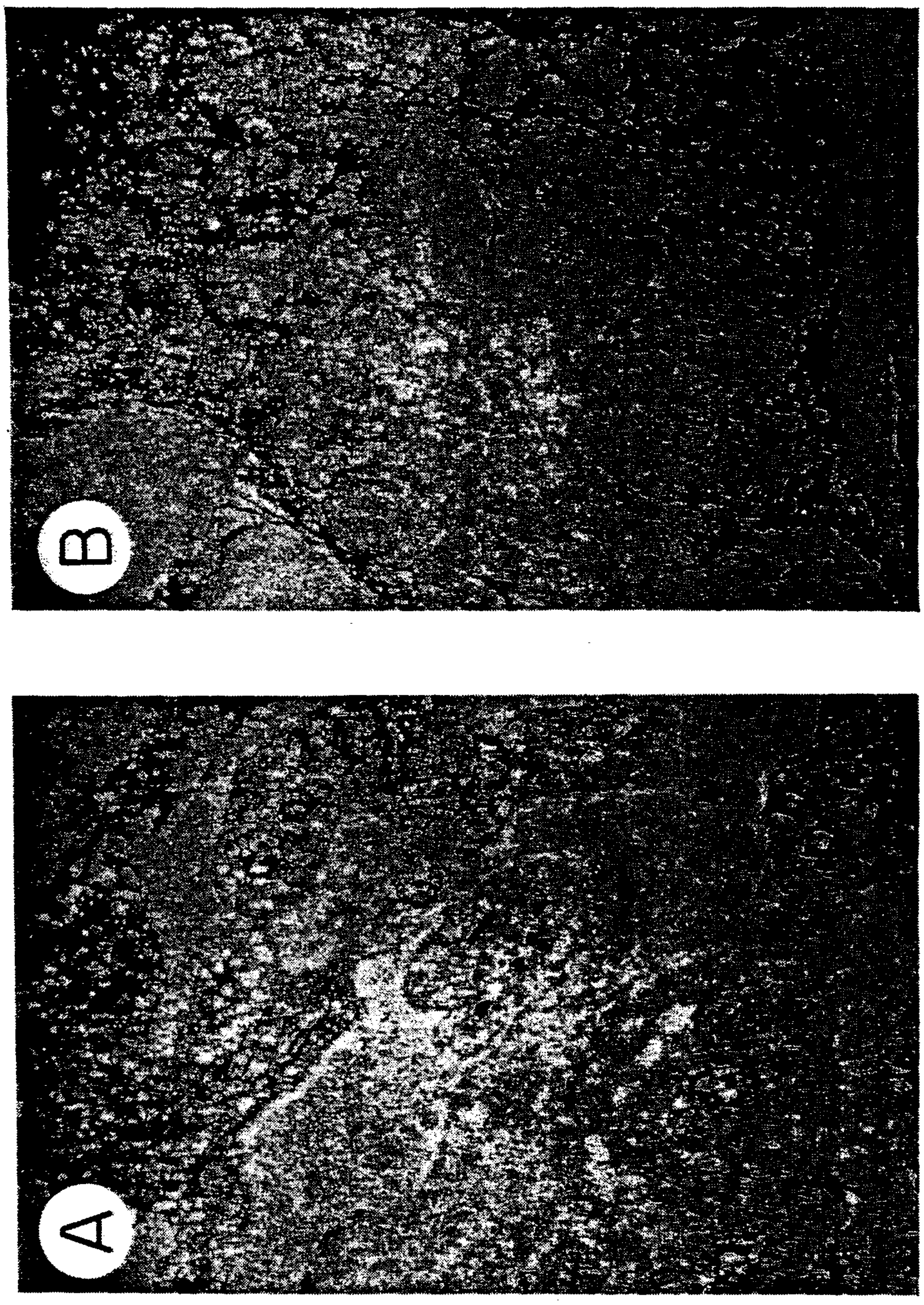

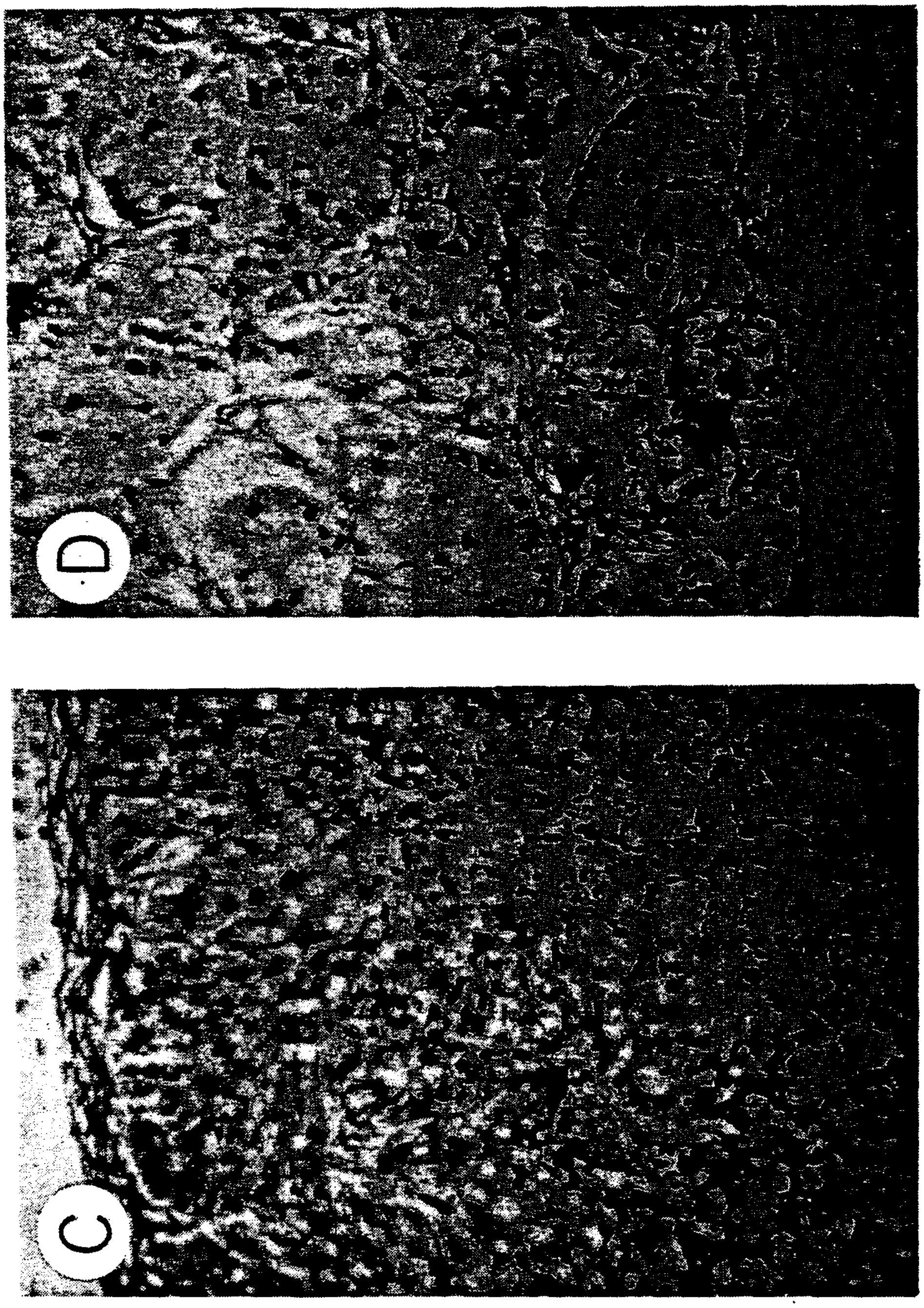


FIG. 24 (CONT.)

FIG. 25

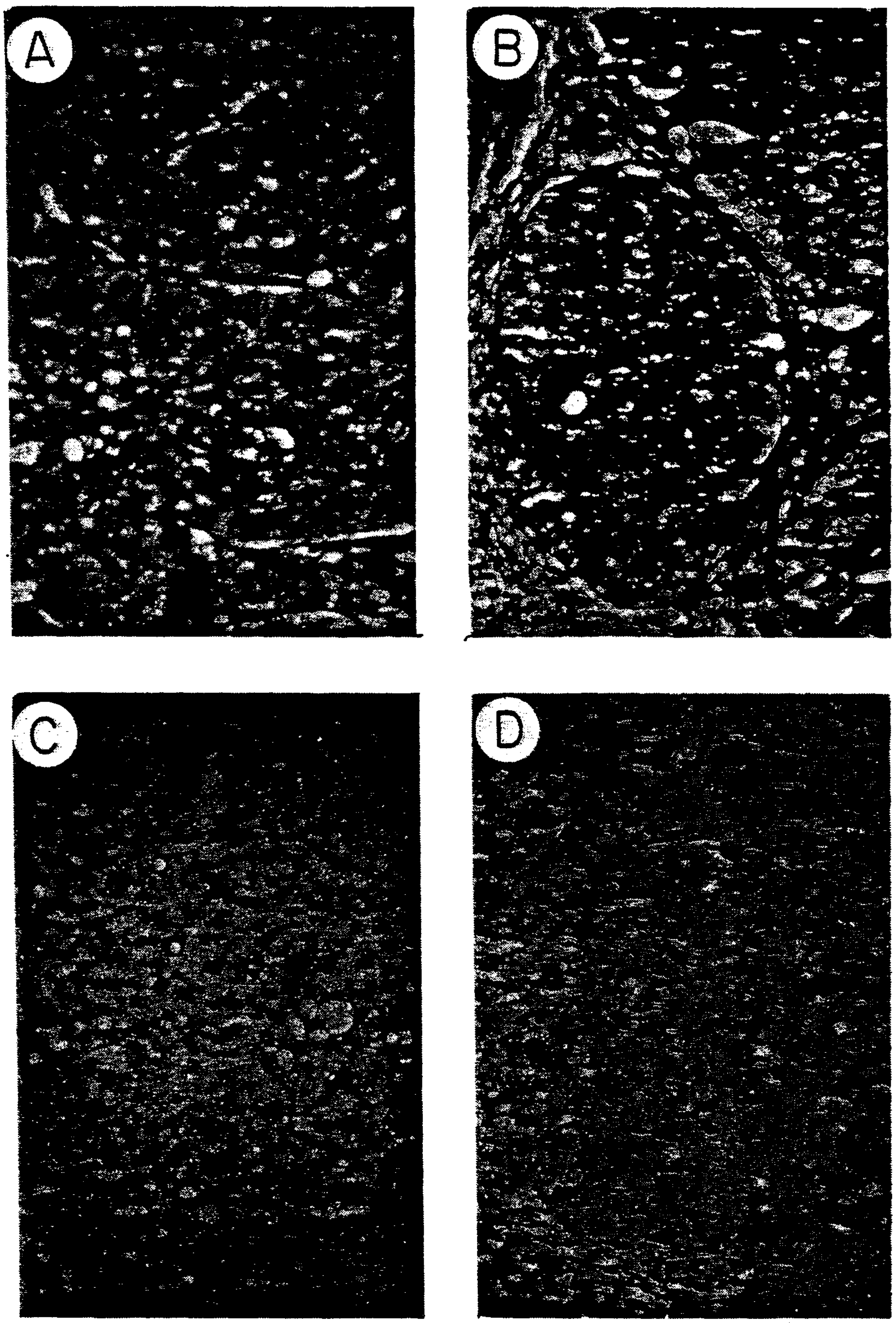

# FIG. 26

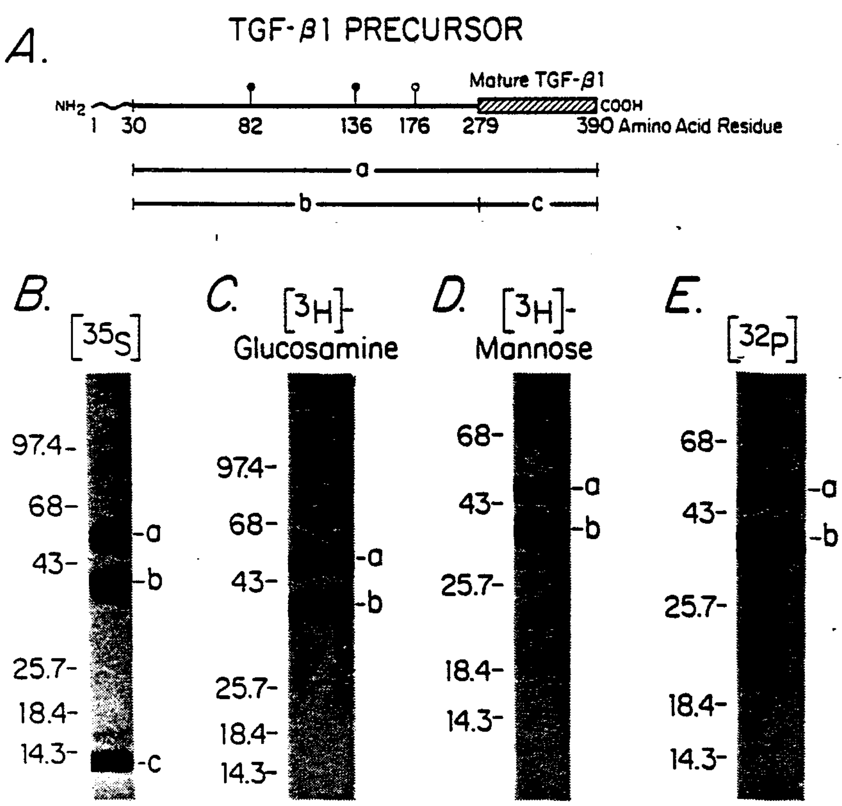
A.
TGF-β1 PRECURSOR
Mature TGF-β1
NH2
COOH
1 30 82 136 176 279 390 Amino Acid Residue
a
b
c
B.
[35S]
97.4-
68-
43-
25.7-
18.4-
14.3-
-a
-b
-c
C.
[3H]-
Glucosamine
97.4-
68-
43-
25.7-
18.4-
14.3-
-a
-b
D.
[3H]-
Mannose
68-
43-
25.7-
18.4-
14.3-
-a
-b
E.
[32P]
68-
43-
25.7-
18.4-
14.3-
-a
-b

FIG. 27

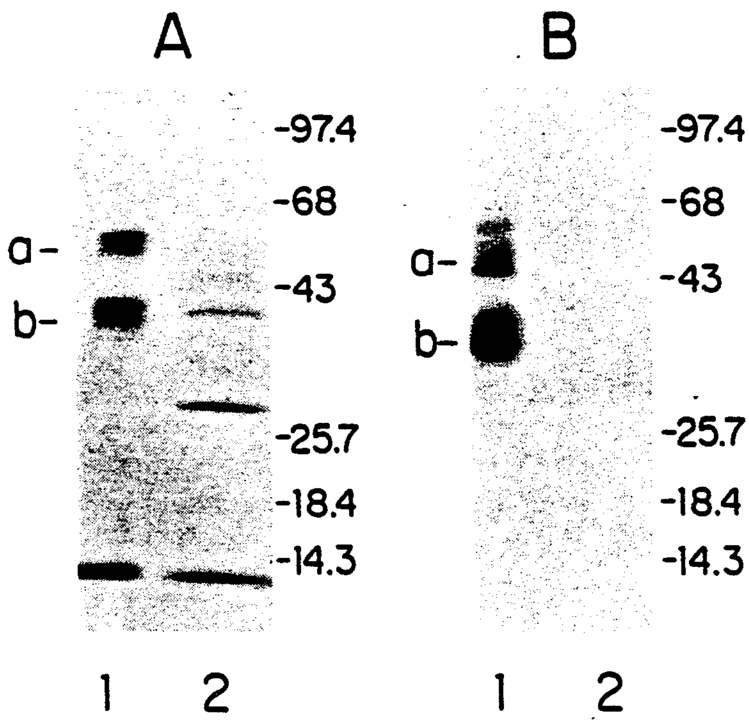

# FIG. 28

E(76-91) A V L A L Y X S T R D R V A G E

E(134-139) F F X T S E

T(174-180) Y S X N S W R

# FIG. 29

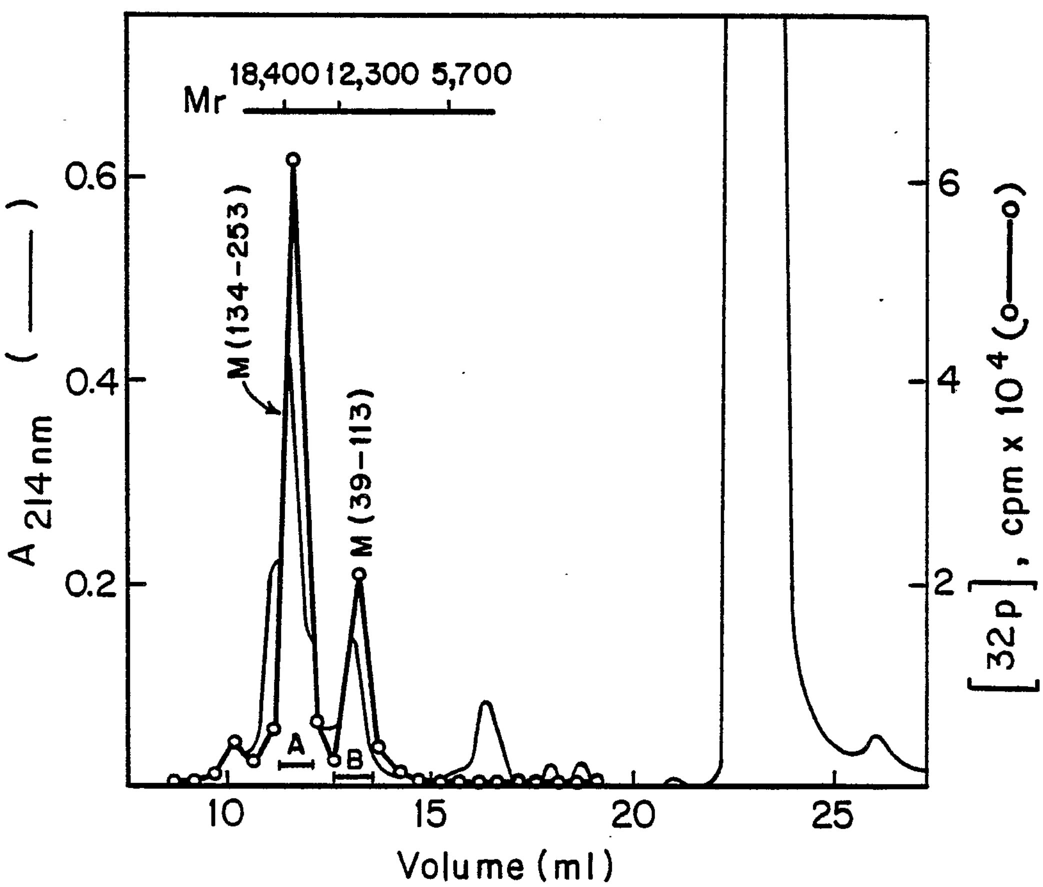

FIG. 30

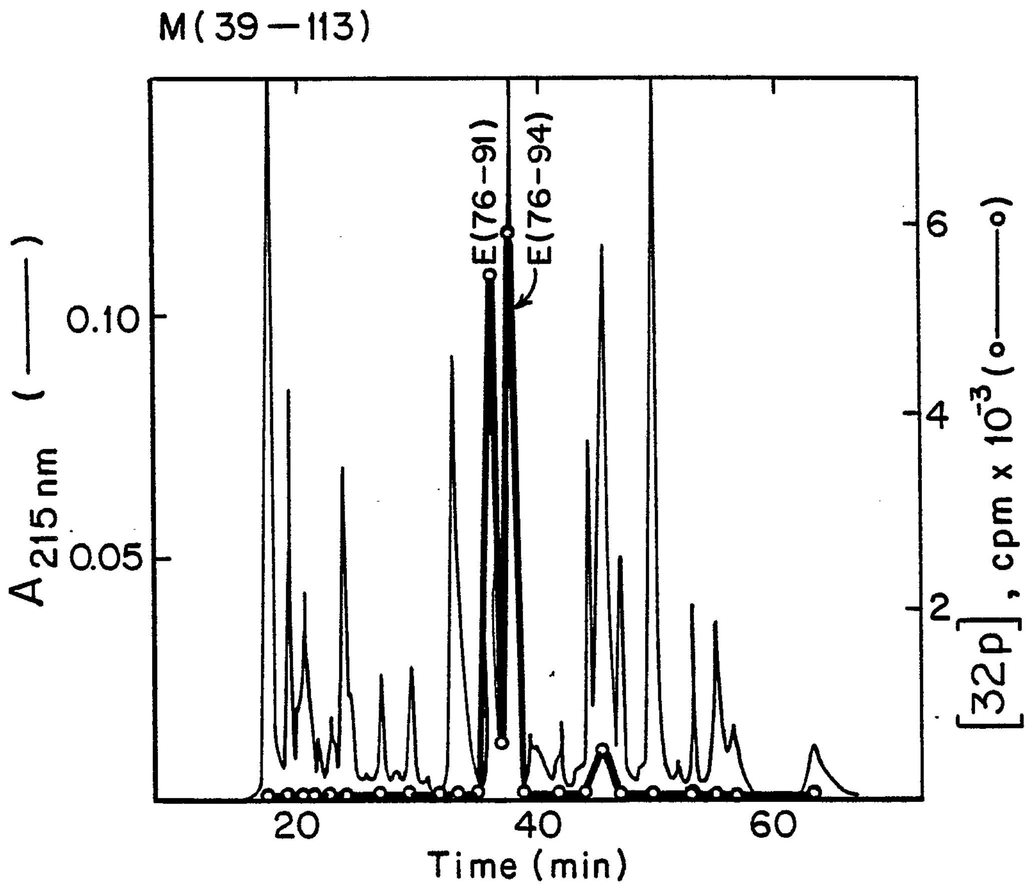
M(39—113)
E(76-91)
E(76-94)
0.10
0.05
$A_{215\,nm}$ (——)
6
4
2
[32p], cpm x $10^{-3}$ (o——o)
20
40
60
Time (min)

# FIG. 31

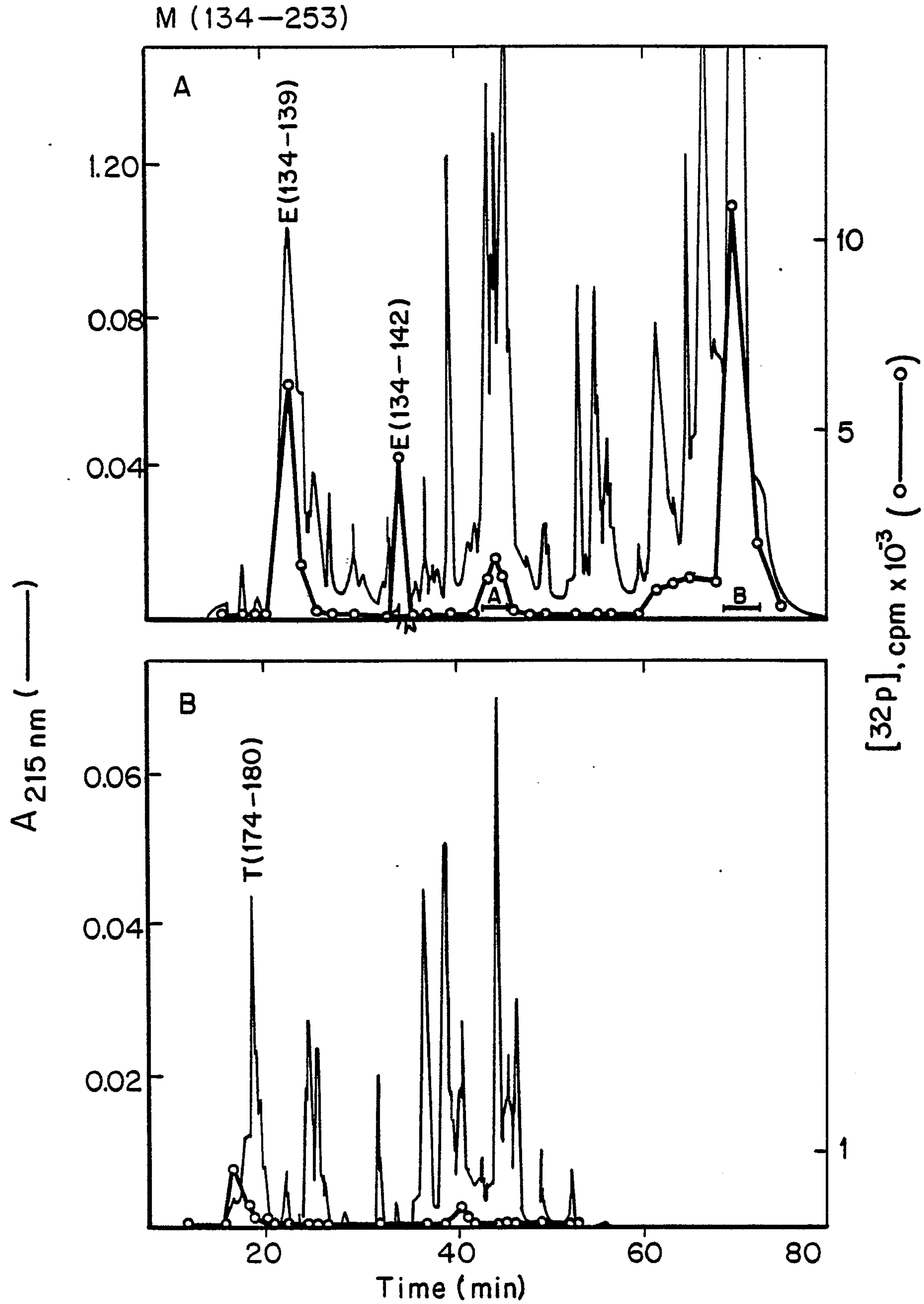
M (134—253)
A
E(134-139)
E(134-142)
A
B
1.20
0.08
0.04
10
5
B
T(174-180)
0.06
0.04
0.02
1
20
40
60
80
Time (min)
A 215 nm (——)
[32p], cpm x 10-3 (o——o)

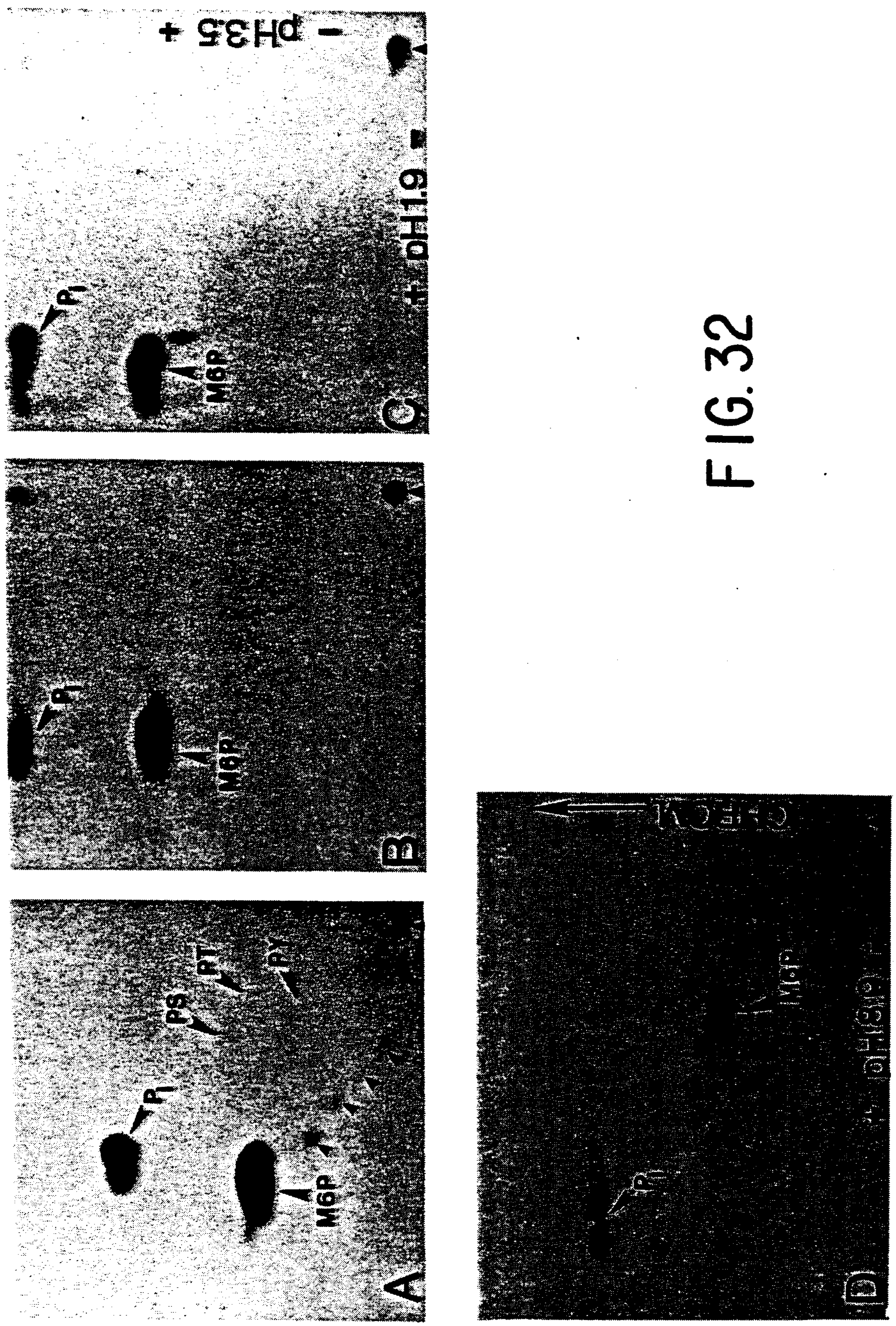


FIG. 32

# CLONING AND EXPRESSION OF SIMIAN TRANSFORMING GROWTH FACTOR-β1

## TABLE OF CONTENTS


| | Page |
|---|---|
| 1. Introduction | 4 |
| 2. Background of the Invention | 4 |
| 3. Summary of the Invention | 6 |
| 4. Description of the Figures | 7 |
| 5. Description of the Invention | 19 |
| 5.1. Isolation or Generation of The Simian TGF-β1 Coding Region | 21 |
| 5.2. Construction of Expression Vectors Containing the TGF-β1 Coding Sequence | 24 |
| 5.3. Identification of Transfectants or Transformants Expressing the TGF-β1 Gene Product | 28 |
| 5.4. Initial Characterization of the TGF-β1 Gene Product | 30 |
| 5.5. The TGF-β1 Precursor: Cellular Processing, Structural Nature and Possible Function | 31 |
| 5.6. Biological Activity of TGF-β1 | 35 |
| 6. Example: cDNA Cloning of TGF-β1 Precursor | 35 |
| 6.1. Materials and Methods | 36 |
| 6.1.1. Growth of Cells and RNA Extraction | 36 |
| 6.1.2. cDNA Library Construction and Screening | 36 |
| 6.1.3. Northern Blot Analysis | 37 |
| 6.2. Results | 37 |
| 7. Example: Expression of TGF-β1 | 40 |
| 7.1. Materials and Methods | 41 |
| 7.1.1. Cell Culture | 41 |

7.1.2. DNA Manipulations and Plasmid Constructions.................... 41
7.1.3. DNA Transfections.................. 42
7.1.4. Selection of Methotrexate Resistant Cells.................. 43
7.1.5. Quantitation of Beta1-TGF Message Levels.................. 44
7.1.6. Growth Inhibition Assay........... 44
7.1.7. Stimulation of Anchorage Independent Growth................ 45
7.1.8. Peptide Synthesis and Production of Antibodies.................... 45
7.1.9. Immunoblotting.................... 45
7.1.10. Northern Blot Analysis............ 46
7.1.11. Southern Blot Analysis............ 46
7.2. Expression of rTGF-β1 in CHO Cells.......... 47
7.3. Detection of Secreted Bioactive Recombinant TGF-β1......................... 50
7.4. Acid Activation Optimizes Bioactivity of Secreted Recombinant TGF-β1............. 53
7.5. Identification of Mature and Precursor Forms of rTGF-β1 in the Culture Medium of Transfectant TGF-β1-3 CHO Cells......................... 54
7.5.1. Identification of Mature rTGF-β1........................... 55
7.5.2. Identification of Precursor rTGF-β1........................... 55
7.6. Recombinant TGF-β1 Constitutes the Majority of the Secreted Proteins from TGF-β1-3/2000 Cells......................... 56
8. Example: Characterization of the TGF-β1 Gene Product...................................... 57
8.1. Glycosylation and Phosphorylation of the rTGF-β1 Precursor......................... 57

8.2. Purification of Biologically Active rTGF-β1. 60
8.3. Purification and Nature of rTGF-β1 Precursor.................................. 64
8.4. Amino Terminal Sequence of rTGF-β1 Polypeptides.............................. 67
8.5. Biological Activity In Vitro............... 68
8.6. Biological Activity In Vivo................ 69
9. Example: Identification of Mannose-6-Phosphate In Two Asparagine-Linked Sugar Chains of rTGF-β1 Precursor.................................. 73
9.1. Materials and Methods...................... 73
9.1.1. Materials......................... 73
9.1.2. Cell Culture...................... 73
9.1.3. Radiolabeling..................... 73
9.1.4. Polyacrylamide Gel Electrophoresis................... 74
9.1.5. Acid Hydrolysis................... 74
9.1.6. 5-Pyridylethylation.............. 75
9.1.7. Chemical and Enzymatic Cleavage.......................... 75
9.1.8. Peptide Purification.............. 76
9.1.9. Amino Acid Sequence Analysis...... 76
9.2. Results.................................... 77
10. Deposit of Microorganisms........................ 80